(19) Europäisches Patentamt
European Patent Office
Office européen des brevets

(11) EP 4 381 096 B1

(12) **EUROPEAN PATENT SPECIFICATION**

(45) Date of publication and mention of the grant of the patent:
**28.05.2025 Bulletin 2025/22**

(21) Application number: **22761989.7**

(22) Date of filing: **04.08.2022**

(51) International Patent Classification (IPC):
***C12Q 1/6841*** *(2018.01)*

(52) Cooperative Patent Classification (CPC):
(C-Sets available)
**C12Q 1/6841** (Cont.)

(86) International application number:
**PCT/EP2022/071935**

(87) International publication number:
**WO 2023/012272 (09.02.2023 Gazette 2023/06)**

# (54) METHOD FOR DETECTING AN ANALYTE IN A PATHOGEN-COMPRISING SAMPLE

VERFAHREN ZUM NACHWEIS EINES ANALYTEN IN EINER PATHOGENHALTIGEN PROBE

PROCÉDÉ DE DÉTECTION D'UN ANALYTE DANS UN ÉCHANTILLON COMPRENANT UN PATHOGÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.08.2021 US 202163230247 P**

(43) Date of publication of application:
**12.06.2024 Bulletin 2024/24**

(73) Proprietor: **Resolve BioSciences GmbH
40789 Monheim am Rhein (DE)**

(72) Inventors:
- **KORFHAGE, Christian
40764 Langenfeld (DE)**
- **REINECKE, Frank
45134 Essen (DE)**
- **GEIPEL, Andreas
40822 Mettmann (DE)**

(74) Representative: **Roth, Andy Stefan
Dr. Roth Patentanwaltskanzlei
Plange Mühle 5
40221 Düsseldorf (DE)**

(56) References cited:
**WO-A1-2017/019481 WO-A1-2020/254519**

- **FAY WANG ET AL: "RNAscope. A novel in situ RNA analysis platform for formalin-fixed, paraffin-embedded tissues", THE JOURNAL OF MOLECULAR DIAGNOSTICS, vol. 14, no. 1, 1 January 2012 (2012-01-01), pages 22 - 29, XP055191966, ISSN: 1525-1578, DOI: 10.1016/j.jmoldx.2011.08.002**
- **KULASINGHE ARUTHA ET AL: "Spatial Profiling of Lung SARS-CoV-2 and Influenza Virus Infection Dissects Virus-Specific Host Responses and Gene Signatures", MEDRXIV, 6 November 2020 (2020-11-06), XP055982022, Retrieved from the Internet <URL:https://www.medrxiv.org/content/10.1101/2020.11.04.20225557v1.full.pdf> [retrieved on 20221116], DOI: 10.1101/2020.11.04.20225557**
- **LEE JEFFREY Y ET AL: "Absolute quantitation of individual SARS-CoV-2 RNA molecules: a new paradigm for infection dynamics and variant differences", BIORXIV, 9 July 2021 (2021-07-09), XP055982462, Retrieved from the Internet <URL:https://www.biorxiv.org/content/10.1101/2021.06.29.450133v2.full.pdf> [retrieved on 20221117], DOI: 10.1101/2021.06.29.450133**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

- **ZITO MARINO FEDERICA ET AL: "Multiplex HPV RNA in situ hybridization/p16 immunohistochemistry: a novel approach to detect papillomavirus in HPV-related cancers. A novel multiplex ISH/IHC assay to detect HPV", INFECTIOUS AGENTS AND CANCER, vol. 15, no. 1, 1 December 2020 (2020-12-01), XP055981913, Retrieved from the Internet <URL:https://infectagentscancer.biomedcentral.com/counter/pdf/10.1186/s13027-020-00310-x.pdf> DOI: 10.1186/s13027-020-00310-x**


Remarks:

The complete document including Reference Table(s) and the Sequence Listing(s) can be downloaded from the EPO website

(52) Cooperative Patent Classification (CPC): (Cont.)

C-Sets

**C12Q 1/6841, C12Q 2537/143, C12Q 2537/149, C12Q 2537/162, C12Q 2563/131, C12Q 2563/179, C12Q 2565/601**

**Description**

## FIELD OF THE DISCLOSURE

The technology provided herein relates to methods for detecting analytes in a pathogen-comprising sample, in particular by detecting the analytes in the pathogen-inactivated sample by a a multiplex spatial transcriptomics method, as well as *in vitro* methods for the diagnosis of a disease.

## BACKGROUND

Methods for detecting specific molecular, cellular and pathogen targets (such as viruses, bacteria , or other pathogens) are fundamental tools for medical and veterinary diagnostics, environmental testing, and industrial quality control. Examples of methods for detecting specific targets in clinical medicine include over-the-counter rapid pregnancy tests, microbiological culture tests for determining the resistance of infectious agents to specific antibiotics, and highly automated tests for cancer markers in blood samples. Detecting pathogen contaminants in food, high throughput screening of candidate compounds for drug discovery, and quantifying active ingredients in pharmaceuticals exemplify industrial manufacturing applications that depend on methods for determining the presence of specific targets. Environmental applications requiring testing for specific targets include detecting water supply contamination, airborne biothreat agents, and household fungal contaminants.

For example, the COVID-19 pandemic caused by SARS-CoV-2 highlights clinical needs to detect infection, track strain evolution, and identify biomarkers of disease course. Analysis of gene expression depends on instruments that may not be placed with in labs of higher safety level used for detecting pathogens. Therefore, these analyses of samples with a higher safety level are not possible if the instruments are located in the same laboratory. An alternative method is to extract the biomolecules of interest which in turn reduce the safety level so that the analysis can be performed outside of higher saftety level lab. Typically, spatial transcriptomic analysis of biosafety samples are done after RNA or DNA is isolated. This is typical for a scenario using the Visium technology (10x genomics) or the GeoMx system (Nanostring; https://www.nature.com/articles/s41467-021-21361-7).

The analysis and detection of small quantities of analytes in biological and non-biological samples has become a routine practice in the clinical and analytical environment. Numerous analytical methods have been established for this purpose. Some of them use encoding techniques assigning a particular readable code to a specific first analyte which differs from a code assigned to a specific second analyte.

One of the prior art techniques in this field is the so-called 'single molecule fluorescence *in-situ* hybridization' (smFISH) essentially developed to detect mRNA molecules in a sample. In Lubeck et al. (2014), Single-cell in situ RNA profiling by sequential hybridization, Nat. Methods 11(4), p. 360-361, the mRNAs of interest are detected via specific directly labeled probe sets. After one round of hybridization and detection, the set of mRNA specific probes is eluted from the mRNAs and the same set of probes with other (or the same) fluorescent labels is used in the next round of hybridization and imaging to generate gene specific color-code schemes over several rounds. The technology needs several differently tagged probe sets per transcript and needs to denature these probe sets after every detection round.

A further development of this technology does not use directly labeled probe sets. Instead, the oligonucleotides of the probe sets provide nucleic acid sequences that serve as initiator for hybridization chain reactions (HCR), a technology that enables signal amplification; see Shah et al. (2016), In situ transcription profiling of single cells reveals spatial organization of cells in the mouse hippocampus, Neuron 92(2), p. 342-357.

Another technique referred to as 'multiplexed error robust fluorescence in situ hybridization' (merFISH) is described by Chen et al. (2015), RNA imaging. Spatially resolved, highly multiplexed RNA profiling in single cells, Science 348(6233):aaa6090. There, the mRNAs of interest are detected via specific probe sets that provide additional sequence elements for the subsequent specific hybridization of fluorescently labeled oligonucleotides. Each probe set provides four different sequence elements out of a total of 16 sequence elements. After hybridization of the specific probe sets to the mRNAs of interest, the so-called readout hybridizations are performed. In each readout hybridization, one out of the 16 fluorescently labeled oligonucleotides complementary to one of the sequence elements is hybridized. All readout oligonucleotides use the same fluorescent color. After imaging, the fluorescent signals are destroyed via illumination and the next round of readout hybridization takes place without a denaturing step. As a result, a binary code is generated for each mRNA species. A unique signal signature of 4 signals in 16 rounds is created using only a single hybridization round for binding of specific probe sets to the mRNAs of interest, followed by 16 rounds of hybridization of readout oligonucleotides labeled by a single fluorescence color.

A further development of this technology improves the throughput by using two different fluorescent colors, eliminating the signals via disulfide cleavage between the readout-oligonucleotides and the fluorescent label and an alternative hybridization buffer; see Moffitt et al. (2016), High-throughput single-cell gene-expression profiling with multiplexed error-robust fluorescence in situ hybridization, Proc. Natl. Acad. Sci. U S A. 113(39), p. 11046-11051.

A technology referred to as 'intron seqFISH' is described in Shah et al. (2018), Dynamics and spatial genomics of the nascent transcriptome by intron seqFISH, Cell 117(2), p. 363-376. There, the mRNAs of interest are detected via specific probe sets that provide additional sequence elements for the subsequent specific hybridization of fluorescently labeled oligonucleotides. Each probe set provides one out of 12 possible sequence elements (representing the 12 'pseudo colors' used) per color-coding round. Each color-coding round consists of four serial hybridizations. In each of these serial hybridizations, three readout probes, each labeled with a different fluorophore, are hybridized to the corresponding elements of the mRNA-specific probe sets. After imaging, the readout probes are stripped off by a 55% formamide buffer and the next hybridization follows. After 5 color-coding rounds with 4 serial hybridizations each, the color-codes are completed.

EP 0 611 828 discloses the use of a bridging element to recruit a signal generating element to probes that specifically bind to an analyte. A more specific statement describes the detection of nucleic acids via specific probes that recruit a bridging nucleic acid molecule. This bridging nucleic acids eventually recruit signal-generating nucleic acids. This document also describes the use of a bridging element with more than one binding site for the signal generating element for signal amplification like branched DNA.

Player et al. (2001), Single-copy gene detection using branched DNA (bDNA) in situ hybridization, J. Histochem. Cytochem. 49(5), p. 603-611, describe a method where the nucleic acids of interest are detected via specific probe sets providing an additional sequence element. In a second step, a preamplifier oligonucleotide is hybridized to this sequence element. This preamplifier oligonucleotide comprises multiple binding sites for amplifier oligonucleotides that are hybridized in a subsequent step. These amplifier oligonucleotides provide multiple sequence elements for the labeled oligonucleotides. This way a branched oligonucleotide tree is build up that leads to an amplification of the signal.

A further development of this method referred to as is described by Wang et al. (2012), RNAscope: a novel in situ RNA analysis platform for formalin-fixed, paraffin-embedded tissues, J. Mol. Diagn. 14(1), p.22-29, which uses another design of the mRNA-specific probes. Here two of the mRNA-specific oligonucleotides have to hybridize in close proximity to provide a sequence that can recruit the preamplifier oligonucleotide. This way the specificity of the method is increased by reducing the number of false positive signals.

WO 2017/019481 relates to the use of conventional "ligation in-situ hybridization" (LISH) methods for the detection of pathogens.

Choi et al. (2010), Programmable in situ amplification for multiplexed imaging of mRNA expression, Nat. Biotechnol. 28(11), p. 1208-1212, disclose a method known as 'HCR-hybridization chain reaction'. The mRNAs of interest are detected via specific probe sets that provide an additional sequence element. The additional sequence element is an initiator sequence to start the hybridization chain reaction. Basically, the hybridization chain reaction is based on metastable oligonucleotide hairpins that self-assemble into polymers after a first hairpin is opened via the initiator sequence.

A further development of the technology uses so called split initiator probes that have to hybridize in close proximity to form the initiator sequence for HCR, similarly to the RNAscope technology, this reduces the number of false positive signals; see Choi et al. (2018), Third-generation in situ hybridization chain reaction: multiplexed, quantitative, sensitive, versatile, robust. Development 145(12).

Mateo et al. (2019), Visualizing DNA folding and RNA in embryos at single-cell resolution, Nature Vol, 568, p. 49ff., disclose a method called 'optical reconstruction of chromatin structure (ORCA). This method is intended to make the chromosome line visible.

EP 2 992 115 B1 describes a method of sequential single molecule hybridization and provides technologies for detecting and/or quantifying nucleic acids in cells, tissues, organs or organisms through sequential barcoding.

WO 2020/254519 A1 discloses a method of sequential signal-encoding of analytes in a sample, a use of a set of decoding oligonucleotides to sequentially signal-encode analytes in a sample, and to a kit for sequentially signal-encoding of analytes in a sample.

Against this background, it is an object underlying the present disclosure to provide a method for detecting an analyte in a pathogen-comprising sample by means of which the disadvantages of the prior art methods can be reduced or even avoided.

## SUMMARY OF THE DISCLOSURE

The present disclosure pertains to novel multiplex methods and kits for detecting different analytes in a sample in parallel by sequential signal-encoding of said analytes. In particular, the present disclosure pertains to a method that results in inactivation of pathogens for *in-situ* spatial transcriptomics (e.g. Molecular Cartography) without the step DNA or RNA preparation prior to analysis. One important advantage is that the analysis can be done outside of a BSL3 or BSL4 lab.

The technical information set out below may in some respects go beyond the scope of the invention, which is defined exclusively by the appended claims. The additional technical information is provided to place the actual invention in a broader technical context and to illustrate possible related technical developments. Any additional technical information, which does not fall within the scope of the appended claims, is not as such claimed or to be construed as being part of the invention.

In an advantageous embodiment of the present disclosure is that the method for detecting an analyte in a pathogen-comprising sample comprises the steps of:

1) Taking a sample containing pathogens with a risk level > 2.

2) Inactivation of the pathogen within the sample without isolation RNA or DNA from the pathogen or sample

3) Performing spatial *omics analysis.

In a first aspect, embodiments of the disclosure in particular pertains to a method for detecting an analyte in a pathogen-comprising sample comprising:

i) Inactivation of the pathogen within the sample without isolation of RNA and/or DNA from the pathogen or the sample;
ii) Detecting the analyte by spatial transcriptomics.

In particular, the spatial transcriptomics detecting comprises a multiplex method for detecting different analytes in a sample by sequential signal-encoding of said analytes, comprising:

**(A)** contacting the sample with at least twenty (20) different sets of analyte-specific probes for encoding of at least 20 different analytes, each set of analyte-specific probes interacting with a different analyte, wherein if the analyte is a nucleic acid each set of analyte-specific probes comprises at least five (5) analyte-specific probes which specifically interact with different sub-structures of the same analyte, each analyte-specific probe comprising

(aa) a binding element (S) that specifically interacts with one of the different analytes to be encoded, and
(bb) an identifier element (T) comprising a nucleotide sequence which is unique to the analyte to be encoded (unique identifier sequence),

wherein the analyte-specific probes of a particular set of analyte-specific probes differ from the analyte-specific probes of another set of analyte-specific probes in the nucleotide sequence of the identifier element (T), wherein the analyte-specific probes in each set of analyte-specific probes binds to the same analyte and comprises the same nucleotide sequence of the identifier element (T) which is unique to said analyte; and

**(B)** contacting the sample with at least one set of decoding oligonucleotides per analyte, wherein in each set of decoding oligonucleotides for an individual analyte each decoding oligonucleotide comprises:

(aa) an identifier connector element (t) comprising a nucleotide sequence which is essentially complementary to at least a section of the unique identifier sequence of the identifier element (T) of the corresponding analyte-specific probe set, and
(bb) a translator element (c) comprising a nucleotide sequence allowing a specific hybridization of a signal oligonucleotide;

wherein the decoding oligonucleotides of a set for an individual analyte differ from the decoding oligonucleotides of another set for a different analyte in the first connect element (t); and
**(C)** contacting the sample with at least a set of signal oligonucleotides, each signal oligonucleotide comprising:

(aa) a translator connector element (C) comprising a nucleotide sequence which is essentially complementary to at least a section of the nucleotide sequence of a translator element (c) comprised in a decoding oligonucleotide, and
(bb) a signal element.

**(D)** Detecting the signal caused by the signal element;
**(E)** selectively removing the decoding oligonucleotides and signal oligonucleotides from the sample, thereby essentially maintaining the specific binding of the analyte-specific probes to the analytes to be encoded;
**(F)** Performing at least three (3) further cycles comprising steps B) to E) to generate an encoding scheme with a code word per analyte, wherein in particular the last cycle may stop with step (D).

In a second aspect, embodiments of this disclosure relate to *in vitro* methods for diagnosis of a disease due to a pathogen infection like a viral or bacterial infection comprising the use of a method according to the present disclosure.

In a third aspect, embodiments of this disclosure provide *in vitro* methods for diagnosis of a disease in plants caused by infectious and/or parasitic origin, said method comprising the use of a method according to the present disclosure.

Before the disclosure is described in detail, it is to be understood that this disclosure is not limited to the particular component parts of the steps of the methods described. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is moreover to be understood that, in case parameter ranges are given which are delimited by numeric values, the ranges are deemed to include these limitation values.

## BRIEF DESCRIPTION OF THE DRAWINGS

Fig. 1: Embodiment where the analyte is a nucleic acid and the probe set comprises oligonucleotides specifically binding to the analyte. The probes comprise a unique identifier sequence allowing hybridization of decoding oligonucleotides.

Fig. 2: Embodiment where the analyte is a protein and the probe set comprises proteins (here: antibodies) specifically binding to the analyte. The probes comprise a unique identifier sequence allowing hybridization of decoding oligonucleotides.

Fig. 3: Flowchart of the method according to the disclosure.

Fig. 4: Alternative options for the application of decoding and signal oligonucleotides.

Fig. 5: Example for signal encoding of three different nucleic acid sequences by two different signal types and three detection rounds; in this example, the encoding scheme includes error detection.

Fig. 6: Number of generated code words (logarithmic scale) against number of detection cycles.

Fig. 7: Calculated total efficiency of a 5-round encoding scheme based on single step efficiencies.

Fig. 8: Comparison of relative transcript abundances between different experiments.

Fig. 9: Correlation of relative transcript abundances between different experiments.

Fig. 10: Comparison of intercellular distribution of signals.

Fig. 11: Comparison of intracellular distribution of signals.

Fig. 12: Distribution pattern of different cell cycle dependent transcripts.

Fig. 13: Detection of multiple targets using a 8 round code with 2 labels (A and B) and no label (-). The targets 1, 2, 3, 4, 5, 20, and n are represented. The rounds 1, 2, 3, and 8 of the coding scheme are represented. Herein, the blank is part of the code.

Fig. 14: Detection of multiple targets can be performed by an encoding scheme using a detectable marker. The ending scheme may comprise also the "0" as a marker. That means that at a specific position the transcript is not detected. Consequently, the encoding scheme may be represented by the following constructs using only two gene specific probes:

1) With detectable label F: detectable during imaging
2) With detectable label F and quencher Q: not detectable during imaging
3) With quencher Q: not detectable during imaging
4) Without label F: not detectable during imaging

5) Without signaling oligonucleotide: not detectable during imaging
6) With a decoder oligonucleotide that cannot recruit a signaling oligonucleotide
7) Without decoder oligonucleotide: not detectable during imaging

Fig. 15: Possible structures of a multi-decoder. The numbers depict the examples. (A) is the unique identifier sequence, (a) is the corresponding sequence of the decoding oligonucleotide or multi-decoder and (c1) to (c3) are different sequence elements, that specifically bind to different signal oligonucleotides. Examples 2 to 5 show different versions of multi decoders. The order of the different sequence elements as well as the number of signal oligonucleotide binding elements is not fixed. Example 1 shows a normal decoding oligonucleotide since there is only one signal oligonucleotide binding element (c1).

Fig. 16: Example for signal encoding of three different nucleic acid sequences by using multi-decoders and two different signal oligonucleotides creating three different signal types and three detection rounds. In this example, the encoding scheme includes error detection and correction.

Fig. 17: Number of generated code words (logarithmic scale) against number of detection cycles. The number of code words for merFISH does not exponentially increase with the number of detection cycles but gets less effective with each added round. In contrast, the number of code words for intronSeqFISH, the method of the present disclosure without using multi-decoders, the method with multi-decoders increases exponentially. The slope of the curve for the method using multi-decoders is much higher than that of the prior invention, leading to more than 20 000 000 times more code words usable after 20 rounds of detection.

## DETAILED DESCRIPTION OF THE DISCLOSURE

Disclosed herein are novel multiplex methods and kits for detecting different analytes in a sample by sequential signal-encoding of said analytes.

The present disclosure describes the usage of a set of labeled and unlabeled nucleic acid sequences for specific quantitative and/or spatial detection of different analytes in parallel via specific hybridization. The technology allows the discrimination of more different analytes than different detection signals are available. The discrimination may be realized via sequential signal-coding of the analytes achieved by several cycles of specific hybridization, detection of signals and selective elution of the hybridized nucleic acid sequences.

In contrast to other state-of-the-art methods, the oligonucleotides providing the detectable signal are not directly interacting with sample-specific nucleic acid sequences but are mediated by so called "decoding-oligonucleotides". This mechanism decouples the dependency between the analyte-specific oligonucleotides and the signal oligonucleotides. The use of decoding-oligonucleotides allows a much higher flexibility while dramatically decreasing the number of different signal oligonucleotides needed which in turn increases the coding capacity achieved with a certain number of detection rounds.

The utilization of decoding-oligonucleotides leads to a sequential signal-coding technology that is more flexible, cheaper, simpler, faster and/or more accurate than other methods.

### A. Definitions

According to the present disclosure an "analyte" is the subject to be specifically detected as being present or absent in a sample and, in case of its presence, to encode it. It can be any kind of entity, including a protein, polypeptide, protein or a nucleic acid molecule (e.g. RNA, PNA or DNA) of interest. The analyte provides at least one site for specific binding with analyte-specific probes. Sometimes herein the term "analyte" is replaced by "target". An "analyte" according to the disclosure incudes a complex of subjects, e.g. at least two individual nucleic acid, protein or peptides molecules. In an embodiment of the disclosure an "analyte" excludes a chromosome. In another embodiment of the disclosure an "analyte" excludes DNA.

In some embodiments, an analyte may be a "coding sequence", "encoding sequence", "structural nucleotide sequence" or "structural nucleic acid molecule" which refers to a nucleotide sequence that is translated into a polypeptide, usually via mRNA, when placed under the control of appropriate regulatory sequences. The boundaries of the coding sequence are determined by a translation start codon at the 5'-terminus and a translation stop codon at the 3'-terminus. A coding sequence can include, but is not limited to, genomic DNA, cDNA, EST and recombinant nucleotide sequences.

A "sample" as referred to herein is a composition in liquid or solid form suspected of comprising the analytes to be encoded. In particular, the sample is a biological sample, preferably comprising biological tissue, further preferably comprising biological cells and/or extracts and/or part of cells. For example, thee cell is a prokaryotic cells or a eukaryotic cell, in particular a mammalian cell, in particular a human cell. In some embodiments, the biological tissue, biological cells, extracts and/or part of cells are fixed. In particular, the analytes are fixed in a permeabilized sample, such as a cell-containing sample. In particular, the sample is a pathogen-comprising sample and may be any kind of substance, tissue, biological stuff, organism etc. that comtain a pathogen. The sample can be any kind of a solid, a fluidic sample, a smear, a surface that comprise pathogenic material. The sample may comprise eukaryotic, archeae, procaryotic organisms, or viruses. The sample may derive from an organism, an environmental sample, an excretion from an organism, or a surface.

A "pathogen" includes every type of biological entity (eukaryotic, archeae, procaryotic, virus, viroid, protein, pure RNA) that is defined to have a risk for the life humans, animals, plants, or microorganism communities (e.g. in gut, fermenter, lakes etc.) with a "risk level" higher than 2. The risk level is defined by local authorities and may differ locally (e.g. from country to country). E.g., the risk level in Germany is defined by the BioStoffV. In other countries the risk level is defined by other laws and regulations (e.g., CDC in USA). In any case, the risk level of a pathogen is defined in a process based on scientific experience.

"Inactivation of a pathogen" means in particular that a risk level of a pathogen higher than 2 is reduced to at least a risk level of 2 or lower. Ideally, the pathogen inactivating process do not avoid the detection of the pathogen within the detection process. In some embodiments, the pathogen inactivating process do not reduce the detection efficiency of the pathogen within the detection process. The inactivation includes any kind of physical, chemical, biochemical, or biological treatment of a sample to reduce the risk level from >2 to a risk level of 2 or lower. Inactivation includes also any kind of combination of physical, chemical, biochemical, or biological treatment. Therefore, pathogen inactivation comprises to stop the pathogen in a given sample from contaminating the desired sample by rendering them non-infectious, e.g. viral inactivation renders viruses unable to infect. Examples of methods for measuring the inactivation of pathogens are described in WO1993/015215A1.

A pathogen inactivation method may or may not achieve complete inactivation, it is useful to consider a specific example. A bacterial culture is said to be sterilized if an aliquot of the culture, when transferred to a fresh culture plate and permitted to grow, is undetectable after a certain time period. The time period and the growth conditions (e.g., temperature) define an "amplification factor". This amplification factor along with the limitations of the detection method (e.g., visual inspection of the culture plate for the appearance of a bacterial colony) define the sensitivity of the inactivation method. A minimal number of viable bacteria must be applied to the plate for a signal to be detectable. With the optimum detection method, this minimal number is one bacterial cell. With a suboptimal detection method, the minimal number of bacterial cells applied so that a signal is observed may be much greater than 1. The detection method determines a "threshold" below which the method appears to be completely effective (and above which the method is, in fact, only partially effective) .

Furthermore, inactivation of viruses may be demonstrated by a biological assay such as an enzyme assay (e.g., reduction in reverse transcriptase) or cell culture (e.g., virus-induced host cell death). A more sensitive method has been described by C.V. Hanson er. al. for quantifying HIV. See J. Clin. Microbiol. 23:2030 (1990). The method is a plaque assay employing HIV-sensitive cells in a monolayer. A fluorescent stain is used and detection is made by visualization.

For example, a spiking study is a study done in order to determine the possible methods of pathogen like viral inactivation. The results of these studies are numerical and, based on these numbers, researchers can determine whether or not the process on which the study was conducted will be suitable for the viruses they are trying to extract and the solution from which they are trying to extract them. For example, it may be shown through experimentation, that increasing the viral count (or level of activity) of a sample by a factor of $10^4$ or $10^5$ of the original will only change the virus inactivation ratios by one order of magnitude. From this knowledge, spiking studies have been created in which the virus number (or level of activation) is increased or "spiked" by a factor of $10^4$ or $10^5$ of the original sample. This new high number or level of activity is then run through the process stream and purified. The number or level of activity is taken at the beginning and at the end of the process stream and used in the calculation of Reduction Factor. Reduction factor (RF) for a virus removal or inactivation step is calculated using the following equation (Note for Guidance on Virus Validation Studies: The Design, Contribution and Interpretation of Studies Validating the Inactivation and Removal of Viruses, EMEA CPMP BWP, 268/95 1996): $RF_{step} = \log_{10} [(V1 \times T1)/(V2 \times T2)]$, wherein V1 = volume of spiked feedstock prior to the clearance step; T1 = virus concentration of spiked feedstock prior to the clearance step; V2 = volume of material after the clearance step; and T2 = virus concentration of material after the clearance step.

Furthermore, virucidal activity studies may be performed by using a quantitative suspension test with 30 s exposure time (as descriped in World Health Organization. WHO guidelines on hand hygiene in health care: first global patient safety challenge clean care is safer care. Geneva: the Organization; 2009. [cited 2020 Apr 08]). One part virus suspension may be mixed with one part organic load (0.3% bovine serum albumin as an interfering substance) and 8 parts disinfectant solution of different concentrations. After a 30 s exposure, samples may be serially diluted and and the 50% tissue culture infectious dose (TCID50) per milliliter are determined by using crystal violet staining and subsequently scoring the number of wells displaying cytopathic effects. The TCID50 is calculated by the Spearman-Kärber algorithm, as described (George VG, Hierholzer JC, Ades EW. Cell culture. In: Virology methods manual. Mahy BWJ, Kangro HO, editors. Academic Press: London; 1996. p. 3-24). The cytotoxic effects of disinfectants can be monitored by using crystal violet staining and optical analysis for altered density and morphology of the cellular monolayer in the absence of virus. We quantified cytotoxic effects analogous to the $TCID_{50}$/mL of the virus infectivity. The dose-response curves can be determined as percent normalized virus inactivation versus percent log disinfectant concentration by nonlinear regression using the robust fitting method on the normalized $TCID_{50}$ data implemented in Prism version 8.0.3 (GraphPad, https://www.graphpad.com). The reduction factors (RFs) for each treatment condition can be calculated with the formular:

$$RF = treatment - control = log_{10}\left(\frac{\sum_{i=1}^{n} x_i}{n}\right) - log_{10}\left(\frac{\sum_{j=1}^{m} x_j}{m}\right)$$

Physical inactivation treatments for pathogens according to the present disclosure are not limited to but may include any kind of changing temperature, electromagnetic waves, light (including also visible and invisible light), incubation the sample with radioactive substances, etc. that reduce the risk level of a pathogen higher than 2 to at least to a risk level of 2 or lower. Combinations thereof or other treatments are not excluded.

Some examples without limitations to those:

Treatment by dry temperature: >30°C, >40°C, >50°C, >60°C, >70°C, >80°C, >90°C, >100°C> 110°C etc.
Treatment by temperature in solution >30°C, >40°C, >50°C, >60°C, >70°C, >80°C, >90°C, >100°C> 110°C etc.
Treatment by X-Ray, radioactivity, UV Light, blue light, red lighty, infrared light etc.

Chemical inactivation treatments for pathogens according to the present disclosure are not limited to but may include any kind treating the sample by of changing the pathogen environment like pH, salts, fraction of polar to non-polar solvents, oxidative reagents, reductive reagents, reagents that perform covalent or non-covalent linkage to the pathogen or degradative reagents that cleave at least parts of pathogen. All reactions have in common that they reduce the risk level of a pathogen higher than 2 to at least a risk level of 2 or lower. Combinations thereof or other treatments are not excluded.

Some examples without limitations to those:
Treatment by redox reactive reagents such as but not limited to Beta-Mercaptoethanol, Dithiothreitol (DTT), or Dithioerythrit, metal based such as sodium borohydride (NaBH4) or lithium aluminium hydride (LiAlH4). peroxides (e.g.H2O2), Ozone, potassium permanganate, organic peroxides (e.g. tert-butyl hydroperoxide), or sodium perborate, hypochlorites, and other redoxreactive reagents which may be chosen by a researcher from the galvanic series. Redox reagents are all agents and conditions that are able to oxidize or rduce other chemical compunds. The redox reactivity of such reagents can be measured as an electrode potential compared to the standard electrode potential. Redox reactive agents can change their redox-reactive capability by certain reaction conditions that change temperature, pH, concentration, or other parameters (see text book for redox reactions). From this view, the experienced user can change the agents and reactions parameters to find the right redox-reactive substance. For example, the pathogen containing sample is treated by the redox reactive substance in a way so that the pathogen sticks more tightly to the sample or surface or it inactivates the entry mechanism fro pathogens or any other reaction required by the pathogen to propagate in living organisms. Typical pathogen inactivating reagents that result in crosslinkiung of pathogens are aldehydes, formaldehyde, glutaraldehyde, Boinsche Solution, etc.. Typical pathogen inactivating reagents that result in a change of the lipophily of the solution are alcohols such as methanol, ethanol, propanol, etc.. Typical pathogen inactivating reagents that change of the water tension are detergents such as SDS, Triton, NP etc..

For example, a composition for a chemical inactivation treatment may consist/comprise 80% (vol/vol) ethanol, 1.45% (vol/vol) glycerol, and 0.125% (vol/vol) hydrogen peroxide. A further composition may consist/comprise 75% (vol/vol) 2-propanol, 1.45% (vol/vol) glycerol, and 0.125% (vol/vol) hydrogen peroxide. A further composition may consist/comprise 80% (wt/wt) ethanol, 0.725% (vol/vol) glycerol, and 0.125% (vol/vol) hydrogen peroxide. A further composition may consist/comprise 75% (wt/wt) 2-propanol, 0.725% (vol/vol) glycerol, and 0.125% (vol/vol) hydrogen peroxide.

Biochemical inactivation treatments for pathogens according to the present disclosure are not limited to but may include any kind of enzymatic reactions, competing reactions or reversibel or irreversible reactions, adding biomolecules (e.g. proteins, fatty acids, carbohydrates, nucleic acids or mixture thereof) that reduce the risk level of a pathogen higher than 2 to at least to a risk level of 2 or lower. Combinations thereof or other treatments are not excluded. Typical pathogen inactivating reagents that icludes enzymes such as biomolecule degrading enzymes, protein degrading enzymes, enzymes that degrades lipohilic or amphiphilic biomolecules, enzymes that degrades polymers, antibodies etc..

Biological inactivation treatments for pathogens according to the present disclosure are not limited to but may include any kind treating the sample by a biological component that perform the reduction of the risk level of a pathogen higher than 2 to at least a risk level of 2 or lower. Biological inactivation treatments for pathogens according to the present disclosure are not limited to but may include any kind of treating the sample by counteracting phages, viruses, bacteria, or other organisms or parts of organisms that reduce the the risk level of a pathogen higher than 2 to at least to a risk level of 2 or lower. Combinations thereof or other treatments are not excluded.

Spatial transcriptomics (or Spatial *omics) according to the present disclosure means any kind of analysis where data from the sample are derived in a spatial manner from *in-situ* samples of tissues or whole organisms. The *in situ* sample may be a section of an organ or an organism. The *in-situ* sample may be not pretreated or pretreated in a way that is required for improving the result. Spatial*omics may included the detection of small molecules compunds of tissues or cells, proteins, DNA, and / or RNA. More preferentially, spatial*omics is restricted to proteins, DNA, and/or RNA. More preferentially, spatial*omics is restricted to DNA and / or RNA. Even more preferentially, spatial*omics is restricted to smFISH. Even more preferentially, spatial*omics is restricted to any kind of sequential smFISH.

In particular, the spatial transcriptomics detecting comprises a multiplex method for detecting different analytes in the pathogen-comprising sample by sequential signal-encoding of said analytes, comprising an inactivation of the pathogen within the sample without isolation of RNA and/or DNA from the pathogen or the sample, and

**(A)** contacting the sample with at least twenty (20) different sets of analyte-specific probes for encoding of at least 20 different analytes, each set of analyte-specific probes interacting with a different analyte, wherein if the analyte is a nucleic acid each set of analyte-specific probes comprises at least five (5) analyte-specific probes which specifically interact with different sub-structures of the same analyte, each analyte-specific probe comprising

(aa) a binding element (S) that specifically interacts with one of the different analytes to be encoded, and
(bb) an identifier element (T) comprising a nucleotide sequence which is unique to the analyte to be encoded (unique identifier sequence),

wherein the analyte-specific probes of a particular set of analyte-specific probes differ from the analyte-specific probes of another set of analyte-specific probes in the nucleotide sequence of the identifier element (T), wherein the analyte-specific probes in each set of analyte-specific probes binds to the same analyte and comprises the same nucleotide sequence of the identifier element (T) which is unique to said analyte; and

**(B)** contacting the sample with at least one set of decoding oligonucleotides per analyte, wherein in each set of decoding oligonucleotides for an individual analyte each decoding oligonucleotide comprises:

(aa) an identifier connector element (t) comprising a nucleotide sequence which is essentially complementary to at least a section of the unique identifier sequence of the identifier element (T) of the corresponding analyte-specific probe set, and
(bb) a translator element (c) comprising a nucleotide sequence allowing a specific hybridization of a signal oligonucleotide;

wherein the decoding oligonucleotides of a set for an individual analyte differ from the decoding oligonucleotides of another set for a different analyte in the first connect element (t); and

**(C)** contacting the sample with at least a set of signal oligonucleotides, each signal oligonucleotide comprising:

(aa) a translator connector element (C) comprising a nucleotide sequence which is essentially complementary to at least a section of the nucleotide sequence of a translator element (c) comprised in a decoding oligonucleotide, and
(bb) a signal element.

**(D)** Detecting the signal caused by the signal element;

**(E)** Selectively removing the decoding oligonucleotides and signal oligonucleotides from the sample, thereby essentially maintaining the specific binding of the analyte-specific probes to the analytes to be encoded;

**(F)** Performing at least three (3) further cycles comprising steps B) to E) to generate an encoding scheme with a code word per analyte, wherein in particular the last cycle may stop with step (D).

As used in the present disclosure, "cell", "cell line", and "cell culture" can be used interchangeably and all such designations include progeny. Thus, the words "transformants" or "transformed cells" include the primary subject cell and cultures derived therefrom without regard for the number of transfers. It is also understood that all progeny may not be precisely identical in DNA content, due to deliberate or inadvertent mutations. Mutant progeny that have the same functionality as screened for in the originally transformed cell are included.

One important advantage of the methods according to the present disclosure is that the analysis can be done outside of a BSL3 or BSL4 lab. A biosafety level (BSL), or pathogen/protection level, is a set of biocontainment precautions required to isolate dangerous biological agents in an enclosed laboratory facility. Thee levels of containment range from the lowest biosafety level 1 (BSL-1) to the highest at level 4 (BSL-4). In the United States, the Centers for Disease Control and Prevention (CDC) have specified these levels.] In the European Union, the same biosafety levels are defined in a directive. In Canada the four levels are known as Containment Levels. Facilities with these designations are also sometimes given as P1 through P4 (for pathogen or protection level), as in the term P3 laboratory. For example Biosafety level 3 (BSL-3) is appropriate for work involving pathogens which can cause serious and potentially lethal disease via the inhalation route. Biosafety level 4 (BSL-4) is the highest level of biosafety precautions, and is appropriate for work with agents that could easily be aerosol-transmitted within the laboratory and cause severe to fatal disease in humans for which there are no available vaccines or treatments.

An "encoding scheme" may describe a set of code words that are associated with the analytes to be detected. Each code word refers to one of the analytes and can be distinguished from all other code words. A code word hereby is a sequence of signs provided by the detection cycles of the method. A sign within a code word is a detectable signal or the absence of a signal. A code word does not need to comprise of all different signals used in the method. The number of signs in a code word is defined by the number of detection cycles.

An "oligonucleotide" as used herein, refers to s short nucleic acid molecule, such as DNA, PNA, LNA or RNA. The length of the oligonucleotides is within the range 4-200 nucleotides (nt), preferably 6-80 nt, more preferably 8-60 nt, more preferably 10-50 nt, more preferably 12 to 35 depending on the number of consecutive sequence elements. The nucleic acid molecule can be fully or partially single-stranded. The oligonucleotides may be linear or may comprise hairpin or loop structures. The oligonucleotides may comprise modifications such as biotin, labeling moieties, blocking moieties, or other modifications.

The "analyte-specific probe" consists of at least two elements, namely the so-called binding element (S) which specifically interacts with one of the analytes, and a so-called identifier element (T) comprising the ’unique identifier sequence’. The binding element (S) may be a nucleic acid such as a hybridization sequence or an aptamer, or a peptidic structure such as an antibody.

Also a "probe" consists of at least two elements, namely the so-called binding element (S) which specifically interacts with one of the analytes, and a so-called identifier element (T) comprising the ’unique identifier sequence’. The binding element (S) may be a nucleic acid such as a hybridization sequence or an aptamer, or a peptidic structure such as an antibody.

In particular, in some embodiments the binding element (S) comprises moieties which are affinity moieties from affinity substances or affinity substances in their entirety selected from the group consisting of antibodies, antibody fragments, receptor ligands, enzyme substrates, lectins, cytokines, lymphokines, interleukins, angiogenic or virulence factors, allergens, peptidic allergens, recombinant allergens, allergen-idiotypical antibodies, autoimmune-provoking structures, tissue-rejection-inducing structures, immunoglobulin constant regions and their derivatives, mutants or combinations thereof. In further advantageous embodiments, the antibody fragment is a Fab, an scFv; a single domain, or a fragment thereof, a bis scFv, Fab2, Fab3, minibody, maxibody, diabody, triabody, tetrabody or tandab, in particular a single-chain variable fragment (scFv).

The "unique identifier sequence" as comprised by the analyte-specific probe is unique in its sequence compared to other unique identifiers. "Unique" in this context means that it specifically identifies only one analyte, such as Cyclin A, Cyclin D, Cyclin E etc., or, alternatively, it specifically identifies only a group of ana-lytes, independently whether the group of analytes comprises a gene family or not.

Therefore, the analyte or a group of analytes to be encoded by this unique identifier can be distinguished from all other analytes or groups of analytes that are to be encoded based on the unique identifier sequence of the identifier element (T). Or, in other words, there is only one ’unique identifier sequence’ for a particular analyte or a group of analytes, but not more than one, i.e. not even two. Due to the uniqueness of the unique identifier sequence the identifier element (T) hybridizes to exactly one type of decoding oligonucleotides. The length of the unique identifier sequence is within the range 8-60 nt, preferably 12-40 nt, more preferably 14-20 nt, depending on the number of analytes encoded in parallel and the stability of interaction needed. A unique identifier may be a sequence element of the analyte-specific probe, attached directly or by a linker, a covalent bond or high affinity binding modes, e.g. antibody-antigen interaction, streptavidin-biotin interaction etc. It is understood that the term "analyte specific probe" includes a plurality of probes which may differ in their binding elements (S) in a way that each probe binds to the same analyte but possibly to different parts thereof, for instance to different (e.g. neighboring) or overlapping sections of the nucleotide sequence comprised by the nucleic acid molecule to be encoded-ed. However, each of the plurality of the probes comprises the same identifier element (T).

A "bipartite labeling probe " comprises a binding sequence capable of hybridizing the analyte and a binding probe sequence capable of binding a detectable signal molecule like a fluorophore or a nucleic acid sequence comprising a fluorophore.

A "decoding oligonucleotide" or an "adapter" or a /adapter segment" consists of at least two sequence elements. One sequence element that can specifically bind to a unique identifier sequence, referred to as an "identifier connector element "(t) or "first connector element" (t), and a second sequence element specifically binding to a signal oligonucleotide, referred to as "translator element" (c). The length of the sequence elements is within the range 8-60 nt, preferably 12-40 nt, more preferably 14-20 nt, de-pending on the number of analytes to be encoded in parallel, the stability of interaction needed and the number of different signal oligonucleotides used. The length of the two sequence elements may or may not be the same.

In some advantageous embodiments, the decoding oligonucleotide in the kits and/or methods of the present disclosure may be a "multi-decoder". A "multi-decoder" is a decoding oligonucleotide that consists of at least three sequence elements. One sequence element (the identifier connector element (t)) can specifically bind to a unique identifier sequence (identifier element (T)) and at least two other sequence elements (translator elements (c)) specifically bind different signal oligonucleotides (each of these sequence elements specifically binds a signal oligonucleotide that differs to all other signal oligonucleotides recruited by other elements of the multi-decoder). The length of the sequence elements is within the range 8-60 nt, preferably 12-40 nt, more preferably 14-20 nt, depending on the number of analytes detected in parallel, the stability needed and the number of different signal oligonucleotides used. The length of the sequence elements may or may not be the same.

Therefore, in some advantageous embodiments, the decoding oligonucleotide is a multi-decoder comprising

- an identifier connector element (t) comprising a nucleotide sequence which is essentially complementary to at least a section of the unique identifier sequence of the identifier element (T) of the corresponding analyte-specific probe set, and
- at least two translator elements (c) comprising each a nucleotide sequence allowing a specific hybridization of a different signal oligonucleotide.

Therefore, the first translator element binds a different signal oligonucleotide as the second translator element. In particular, the signal oligonucleotides differ in the signal element comprised in the signal oligonucleotide, e.g. in the kind of the fluorophore.

A "signal oligonucleotide" or a "reporter" as used herein comprises at least two elements, a so-called "translator connector element" (C) or "second connector element" (C) having a nucleotide sequence specifically hybridizable to at least a section of the nucleotide sequence of the translator element (c) of the decoding oligonucleotide, and a "signal element" which provides a detectable signal. This element can either actively generate a detectable signal or provide such a signal via manipulation, e.g. fluorescent excitation. Typical signal elements are, for example, enzymes that catalyze a detectable reaction, fluorophores, radioactive elements or dyes.

A "set" refers to a plurality of moieties or subjects, e.g. analyte-specific probes or decoding oligonucleotides, whether the individual members of said plurality are identical or different from each other. In an analyte specific probe set, the analyte specific probes are identical in the identifier element (T) but may comprise a different binding element (S) for specifically interacting with the same analyte but for specifically interacting with different sub-structures of the same analyte to be encoded.

"Selective denaturation" may be the process of eliminating bound decoding oligonucleotides and signal oligonucleotides with highest efficiency while at the same time the target specific probes have to stay hybridized with the highest efficiency. The total efficiency of these two combined events may to be at least 0.22 for two detection cycles, 0.37 for three detection cycles, 0.47 for four detection cycles, 0.55 for five detection cycles, 0.61 for six detection cycles, 0.65 for seven detection cycles, 0.69 for eight detection cycles, 0.72 for nine detection cycles and 0.74 for 10 detection cycles, 0.76 for 11 detection cycles and 0.78 for 12 detection cycles.

In an embodiment of the disclosure a single set refers to a plurality of oligonucleotides.

An "analyte specific probe set" refers to a plurality of moieties or sub-jects, e.g. analyte-specific probes that are different from each other and bind to independent regions of the analyte. A single analyte specific probe set is further characterized by the same unique identifier.

A "decoding oligonucleotide set" refers to a plurality of decoding oligonucleotides specific for a certain unique identifier needed to realize the encoding independent of the length of the code word. Each and all of the decoding oligonucleotides included in a "decoding oligonucleotide set" bind to the same unique identifier element (T) of the analyte-specific probe.

In certain embodiments, this pattern of binding or hybridization of the decoding oligonucleotides may be converted into a "code word." For example, the code words could be also "101" and "110" for an analyte, where a value of 1 represents binding and a value of 0 represents no binding. The code words may also have longer lengths in other embodiments (see Fig. 13). A code word can be directly related to a specific unique identifier sequence of a analyte-specific probe. Accordingly, different analyte-specific probe may match certain code words, which can then be used to identify the different analytes of the analyte-specific probe based on the binding patterns of the decoding oligonucleotide. However, if no binding is evident, then the code word would be "000" in this example.

The values in each code word can also be assigned in different fashions in some embodiments. For example, a value of 0 could represent binding while a value of 1 represents no binding. Similarly, a value of 1 could represent binding of a secondary nucleic acid probe with one type of signaling entity while a value of 0 could represent binding of a secondary nucleic acid probe with another type of distinguishable signaling entity. These signaling entities could be distinguished, for example, via different colors of fluorescence. In some cases, values in code words need not be confined to 0 and 1. The values could also be drawn from larger alphabets, such as ternary (e.g., 0, 1, and 2) or quaternary (e.g., 0, 1, 2, and 3) systems. Each different value could, for example, be represented by a different distinguishable signaling entity, including (in some cases) one value that may be represented by the absence of signal.

The code words for each analyte may be assigned sequentially, or may be assigned at random. For instance, a first analyte may be assigned to 101, while a second nucleic acid target may be assigned to 110. In addition, in some embodiments, the code words may be assigned using an error-detection system or an error- correcting system, such as a Hamming system, a Golay code, or an extended Hamming system (or a SECDED system, i.e., single error correction, double error detection). Generally speaking, such systems can be used to identify where errors have occurred, and in some cases, such systems can also be used to correct the errors and determine what the correct code word should have been. For example, a code word such as 001 may be detected as invalid and corrected using such a system to 101, e.g., if 001 is not previously assigned to a different target sequence. A variety of different error-correcting codes can be used, many of which have previously been developed for use within the computer industry; however, such error-correcting systems have not typically been used within biological systems. Additional examples of such error-correcting codes are discussed in more detail below.

"Essentially complementary" means, when referring to two nucleotide sequences, that both sequences can specifically hybridize to each other under stringent conditions, thereby forming a hybrid nucleic acid molecule with a sense and an antisense strand connected to each other via hydrogen bonds (Watson-and-Crick base pairs). "Essentially complementary" includes not only perfect base-pairing along the entire strands, i.e. perfect complementary sequences but also imperfect complementary sequences which, however, still have the capability to hybridize to each other under stringent conditions. Among experts it is well accepted that an "essentially complementary" sequence has at least 88% sequence identity to a fully or perfectly complementary sequence.

"Percent sequence identity" or "percent identity" in turn means that a sequence is compared to a claimed or described sequence after alignment of the sequence to be compared (the "Compared Sequence") with the described or claimed sequence (the "Reference Sequence"). The percent identity is then determined according to the following formula: percent identity = 100 [1 -(C/R)]

wherein C is the number of differences between the Reference Sequence and the Compared Sequence over the length of alignment between the Reference Sequence and the Compared Sequence, wherein

(i) each base or amino acid in the Reference Sequence that does not have a corresponding aligned base or amino acid in the Compared Sequence and

(ii) each gap in the Reference Sequence and

(iii) each aligned base or amino acid in the Reference Sequence that is different from an aligned base or amino acid in the Compared Sequence, constitutes a difference and (iiii) the alignment has to start at position 1 of the aligned sequences;

and R is the number of bases or amino acids in the Reference Sequence over the length of the alignment with the Compared Sequence with any gap created in the Reference Sequence also being counted as a base or amino acid.

If an alignment exists between the Compared Sequence and the Reference Sequence for which the percent identity as calculated above is about equal to or greater than a specified minimum Percent Identity then the Compared Sequence has the specified minimum percent identity to the Reference Sequence even though alignments may exist in which the herein above calculated percent identity is less than the specified percent identity.

In the "incubation" steps as understood herein the respective moieties or subjects such as probes or oligonucleotide, are brought into contact with each other under conditions well known to the skilled person allowing a specific binding or hybridization reaction, e.g. pH, temperature, salt conditions etc. Such steps may therefore, be preferably carried out in a liquid environment such as a buffer system which is well known in the art.

The "removing" steps according to the disclosure may include the washing away of the moieties or subjects to be removed such as the probes or oligonucleotides by certain conditions, e.g. pH, temperature, salt conditions etc., as known in the art.

It is understood that in an embodiment of the method according to the present disclosure a plurality of analytes can be encoded in parallel. This requires the use of different sets of analyte-specific probes in step (1). The analyte-specific probes of a particular set differ from the analyte-specific probes of another set. This means that the analyte-specific probes of set 1 bind to analyte 1, the analyte-specific probes of set 2 bind to analyte 2, the analyte-specific probes of set 3 bind to analyte 3, etc. In this embodiment also the use of different sets of decoding oligonucleotides is required in the methods according to the present disclosure.

The decoding oligonucleotides of a particular set differ from the decoding oligonucleotides of another set. This means, the decoding oligonucleotides of set 1 bind to the analyte-specific probes of above set 1 of analyte-specific probes, the decoding oligonucleotides of set 2 bind to the analyte-specific probes of above set 2 of analyte-specific probes, the decoding oligonucleotides of set 3 bind to the analyte-specific probes of above set 3 of analyte-specific probes, etc.

In this embodiment where a plurality of analytes is to be encoded in parallel the different sets of analyte-specific probes may be provided as a premixture of different sets of analyte-specific probes and/or the different sets of decoding oligonucleotides may be provided as a premixture of different sets of decoding oligonucleotides. Each mixture may be contained in a single vial. Alternatively, the different sets of analyte-specific probes and/or the different sets of decoding oligonucleotides may be provided in steps singularly.

A "kit" is a combination of individual elements useful for carrying out the use and/or method of the disclosure, wherein the elements are optimized for use together in the methods. The kits may also contain additional reagents, chemicals, buffers, reaction vials etc. which may be useful for carrying out the method according to the disclosure. Such kits unify all essential elements required to work the method according to the disclosure, thus minimizing the risk of errors. Therefore, such kits also allow semi-skilled laboratory staff to perform the method according to the present disclosure.

The term "quencher" or "quencher dye" or "quencher molecule" refers to a dye or an equivalent molecule, such as nucleoside guanosine (G) or 2'-deoxyguanosine (dG), which is capable of reducing the fluorescence of a fluorescent reporter dye or donor dye. A quencher dye may be a fluorescent dye or non-fluorescent dye. When the quencher is a fluorescent dye, its fluorescence wavelength is typically substantially different from that of the reporter dye and the quencher fluorescence is usually not monitored during an assay. Some embodiments of the present disclosure disclose signal oligonucleotides comprising a quencher and/or a quencher in combination with a signal element (see Fig. 14), and therefore the signal oligonucleotides is not detectable during imaging.

In an embodiment of the disclosure the sample is a biological sample, preferably comprising biological tissue, further preferably comprising biological cells. A biological sample may be derived from an organ, organoids, cell cultures, stem cells, cell suspensions, primary cells, samples infected by viruses, bacteria or fungi, eukaryotic or prokaryotic samples, smears, disease samples, a tissue section.

The method is particularly qualified to encode, identify, detect, count or quantify analytes or single analytes molecules in a biological sample, i.e. such as a sample which contains nucleic acids or proteins as said analytes. It is understood that the biological sample may be in a form as it is in its natural environment (i.e. liquid, semi-liquid, solid etc.), or processed, e.g. as a dried film on the surface of a device which may be re-liquefied before the method is carried out.

In another embodiment of the disclosure prior to step (2) the biological tissue and/or biological cells are fixed. For example, in some embodiments, the cell and/or the tissue is fixed prior to introducing the probes, e.g., to preserve the positions of the analytes like nucleic acids within the cell. Techniques for fixing cells are known to those of ordinary skill in the art. As non-limiting examples, a cell may be fixed using chemicals such as formaldehyde, paraformaldehyde, glutaraldehyde, ethanol, methanol, acetone, acetic acid, or the like. In one embodiment, a cell may be fixed using Hepes-glutamic acid buffer- mediated organic solvent (HOPE).

This measure has the advantage that the analytes to be encoded, e.g. the nuclei acids or proteins, are immobilized and cannot escape. In doing so, the analytes then prepared for a better detection or encoding by the method according to the disclosure.

In yet a further embodiment within the set of analyte-specific probes the individual analyte-specific probes comprise binding elements (S1, S2, S3, S4, S5) which specifically interact with different sub-structures of one of the analytes to be encoded.

By this measure the method becomes even more robust and reliable because the signal intensity obtained at the end of the method or a cycle, respectively, is increased. It is understood, that the individual probes of a set while binding to the same analyte differ in their binding position or binding site at or on the analyte. The binding elements S1, S2, S3, S4, S5 etc. of the first, second, third fourth, fifth etc. analyte-specific probes therefore bind to or at a different position which, however, may or may not overlap.

A multiplex method or assay allow the simultaneously measurement of multiple analytes. According to the present disclosure it may be used to determine the presence or absence of a plurality of predetermined (known) analytes like nucleic acid target sequences in a sample. An analyte may be "predetermined" in that its sequence is known to design a probe that binds to the that target.

In some advantageous embodiments according to the present disclosure at least 20, in particular at least 25, in particular at least 30 different analytes are detected and/or quantified in a sample in parallel. For example, there may be at least 5, at least 10, at least 20, at least 50, at least 75, at least 100, at least 300, at least 1,000, at least 3,000, at least 10,000, or at least 30,000 distinguishable analyte-specific probes that are applied to a sample, e.g., simultaneously or sequentially.

In some advantageous embodiments for the multiplexing twenty (20) or more different sets of analyte-specific probes for encoding of at least 20 different analytes or more are required, in particular more than 50, more than 100 or more than 200. In the multiplexing methods of the present disclosure, in particular at least 20 different groups of analytes (e.g. mRNA molecules) i.e. tags are targeted.

In some advantageous embodiments, at least 4 rounds to collect information for identification of the analyte are carried out, wherein multiple readout increases the accuracy of identification and avoids false positives. The unique tag can be identified by various techniques, including hybridization, e.g. with labeled probes, directly or indirectly or by sequencing (by synthesis, ligation). In particular, the identity of the tag can be encoded with one single signal (binary code), two or more signals, wherein the signal can be a fluorescent label (e.g. attached to an oligonucleotide).

In some advantageous embodiments according to the present disclosure, the kit does not comprise sets of analyte-specific probes as defined under item A).

Preferably, if the analyte in methods according to the present disclosure is a nucleic acid, each set of analyte-specific probes comprises at least five (10) analyte-specific probes, in particular at least fifteen (15) analyte-specific probes, in particular at least twenty (20) analyte-specific probes which specifically interact with different sub-structures of the same analyte. Nucleic acid analyte includes specific DNA molecules, e.g. genomic DNA, nuclear DNA, mitochondrial DNA, viral DNA, bacterial DNA, extra- or intracellular DNA etc., and specific mRNA molecules, e.g. hnRNA, miRNA, viral RNA, bacterial RNA, extra- or intracellular RNA, etc.

Preferably, if the analyte in the methods according to the present disclosure is a peptide, a polypeptide or a protein, each set of analyte-specific probes comprises at least two (2) analyte-specific probes, in particular at least three (3) analyte-specific probes, in particular at least four (4) analyte-specific probes which specifically interact with different sub-structures of the same analyte.

In some advantageous embodiments according to the present disclosure the method comprises the use of at least two different sets of signal oligonucleotides, wherein the signal oligonucleotides in each set comprise a different signal element and comprise a different connector element (C).

In particular, the method comprises the use of at least two different sets of decoding oligonucleotides per analyte, wherein the decoding oligonucleotides comprised in these different sets comprise the same identifier connector element (t) comprising a nucleotide sequence which is essentially complementary to at least a section of the unique identifier sequence of the identifier element (T) of the corresponding analyte-specific probe set, and wherein the decoding oligonucleotides of the different sets per analyte differ in the translator element (c) comprising a nucleotide sequence allowing a specific hybridization of a signal oligonucleotide.

In some embodiments the method comprises the use of least two different sets of decoding oligonucleotides per analyte, wherein the decoding oligonucleotides comprised in these different sets comprise the same identifier connector element (t) comprising a nucleotide sequence which is essentially complementary to at least a section of the unique identifier sequence of the identifier element (T) of the corresponding analyte-specific probe set, and wherein the decoding oligonucleotides of the different sets for at least one analyte differ in the translator element (c) comprising a nucleotide sequence allowing a specific hybridization of a signal oligonucleotide.

In some advantageous embodiments, the number of different sets of decoding oligonucleotides per analyte comprising different translator elements (c) corresponds to the number of different sets of signal oligonucleotides comprising different connector elements (C). However, the decoding oligonucleotides in a particular set of decoding oligonucleotides may interacts with identical identifier elements (T) which are unique to a particular analyte. In particular, all sets of decoding oligonucleotides for the different analytes may comprise the same type(s) of translator element(s) (c).

In particular, the present disclosure is directed to a method for detecting an analyte in a pathogen-comprising sample including acts of exposing the pathogen inactivated sample to a plurality of analyte-specific probes; for each of the analyte-specific probes, determining binding of the analyte-specific probes within the sample; creating code words based on the binding of the analyte-specific probes, the decoding oligonucleotides and the signal oligonucleotides; and for at least some of the code words, matching the code word to a valid code word. In certain embodiments, this pattern of binding or hybridization of the analyte-specific probes, the decoding oligonucleotides and the signal oligonucleotides may be converted into a "code word." For example, for instance, the code words may be "$10^{1}$" and "$1^{10}$" for a first analyte and a second analyte, respectively, where a value of 1 represents binding and a value of 0 represents no binding of decoding oligonucleotides and/or the binding of signal oligonucleotides without and/or quenched signal element. The analyte in the detection round/cycle is therefore not detectable during imaging.

To create such a zero (0) in a code word for an individual analyte the method may comprise the use of:

**(D)** at least a set of non-signal decoding oligonucleotides for binding to a particular identifier element (T) of analyte-specific probes, wherein the decoding oligonucleotides in the same set of non-signal decoding oligonucleotides interacting with the same different identifier element (T),

wherein each non-signal decoding oligonucleotide comprises an identifier connector element (t) comprising a nucleotide sequence which is essentially complementary to at least a section of a unique identifier sequence, and does not comprise a translator element (c) comprising a nucleotide sequence allowing a specific hybridization of a signal oligonucleotide.

To create such a zero (0) in a code word for an individual analyte the method may comprise the use of:
**(D)** at least a set of non-signal decoding oligonucleotides for binding to a particular identifier element (T) of analyte-specific probes, wherein the decoding oligonucleotides in the same set of non-signal decoding oligonucleotides interacting with the same different identifier element (T),
wherein each non-signal decoding oligonucleotide comprises an identifier connector element (t) comprising a nucleotide sequence which is essentially complementary to at least a section of a unique identifier sequence, and comprise a translator element that does not interact/bind to a signal oligonucleotide due to an instable binding sequence and/or due to the translator element is to short (c) comprising a nucleotide sequence allowing a specific hybridization of a signal oligonucleotide.
In some advantageous embodiments, the method may comprise the use of:
**(D)** at least two (2) different sets of non-signal decoding oligonucleotides for binding to at least two different identifier elements (T) of analyte-specific probes, each set of non-signal decoding oligonucleotides interacting with a different identifier element (T),
wherein each non-signal decoding oligonucleotide comprises an identifier connector element (t) comprising a nucleotide sequence which is essentially complementary to at least a section of a unique identifier sequence, and does not comprise a translator element (c) comprising a nucleotide sequence allowing a specific hybridization of a signal oligonucleotide.
Furthermore, in some advantageous embodiments the method may comprise the use of:
**(E)** a set of non-signal oligonucleotides, each non-signal oligonucleotide comprising:

(aa) a translator connector element (C) comprising a nucleotide sequence which is essentially complementary to at least a section of the nucleotide sequence of the translator element (c), and
(bb) a quencher (Q), a signal element and a quencher (Q), or does not comprise a signal element.

In some advantageous embodiments, the method may comprise the use of:
**(E)** at least two sets of non-signal oligonucleotides, each non-signal oligonucleotide comprising:
(aa) a translator connector element (C) comprising a nucleotide sequence which is essentially complementary to at least a section of the nucleotide sequence of the translator element (c), and (bb) a quencher (Q), a signal element and a quencher (Q), or does not comprise a signal element.
In some advantageous embodiments, the different sets of non-signal oligonucleotides may be comprised in a pre-mixture of different sets of non-signal oligonucleotides or exist separately.
Further, in some embodiments the decoding oligonucleotides in a particular set of decoding oligonucleotides interacts with identical identifier elements (T) which are unique to a particular analyte.
In some advantageous embodiments, the different sets of decoding oligonucleotides may be comprised in a pre-mixture of different sets of decoding oligonucleotides or exist separately. In some advantageous embodiments, the different sets of analyte-specific probes may be comprised in a pre-mixture of different sets of analyte-specific probes or exist separately. In some advantageous embodiments, the different sets of signal oligonucleotides may be comprised in a pre-mixture of different sets of signal oligonucleotides or exist separately.
In some advantageous embodiments, a mixture of decoding oligonucleotides and/or multi-decoders is provided that specifically hybridize to the unique identifier sequences of the probe sets. In some embodiments, the decoding oligonucleotides comprise of at least two sequence elements, a first element that is complementary to the unique identifier sequences of the corresponding probe set and a second sequence element (translator element) that provides a sequence for the specific hybridization of a signal oligonucleotide, the translator element defines the type of signal that is recruited to the decoding oligonucleotide. In some embodiments multi-decoders comprising at least three sequence elements are used, a first element that is complementary to the unique identifier sequences of the corresponding probe set and at least to additional sequence elements (translator elements) that provide sequences for the specific hybridization of at least two different signal oligonucleotides. The translator elements define the type of signals that are recruited to the multi-decoder. Different possible structures of a multi-decoder can be seen in Fig. 15. Since a multi-decoder does recruit a full signal oligonucleotide per translator element, the brightness of the signals in each channel is not lower than the brightness of signals with decoding oligonucleotides.
The usage of multi-decoders increases further the efficiency of the encoding scheme. Figure 16 shows a possible encoding scheme using multi-decoders based upon the same conditions used for the examples with the decoding oligonucleotide with two sequence elements. One can clearly see that the multi-decoder based encoding scheme can create a higher hamming distance, with the same number of rounds and the same number of different signal oligonucleotides used in the example of Fig. 5.
As mentioned above the analyte to be encoded may be a nucleic acid, preferably DNA, PNA or RNA, in particular mRNA, a peptide, polypeptide, a protein, and/or mixtures thereof.
In some advantageous embodiments, the binding element (S) comprises an amino acid sequence allowing a specific binding to the analyte to be encoded. The binding element (S) may comprise moieties which are affinity moieties from affinity substances or affinity substances in their entirety selected from the group consisting of antibodies, antibody fragments, anticalin proteins, receptor ligands, enzyme substrates, lectins, cytokines, lymphokines, interleukins, angiogenic or virulence factors, allergens, peptidic allergens, recombinant allergens, allergen-idiotypical antibodies, autoimmune-provoking structures, tissue-rejection-inducing structures, immunoglobulin constant regions and combinations thereof.

In some advantageous embodiments, the binding element (S) may comprise or is an antibody or an antibody fragment selected from the group consisting of Fab, scFv; single domain, or a fragment thereof, bis scFv, F(ab)2, F(ab)3, minibody, diabody, triabody, tetrabody and tandab.

The present disclosure pertains in particular to a method for detecting an analyte in a pathogen-comprising sample comprising

i) Inactivation of the pathogen within the sample without isolation of RNA and/or DNA from the pathogen or the sample;
ii) Detecting the analyte by spatial transcriptomics, wherein

the spatial transcriptomics detecting comprises or is a multiplex method for detecting different analytes in a sample by sequential signal-encoding of said analytes, comprising the steps of:

(A) contacting the sample with at least twenty (20) different sets of analyte-specific probes for encoding of at least 20 different analytes, each set of analyte-specific probes interacting with a different analyte, wherein if the analyte is a nucleic acid each set of analyte-specific probes comprises at least five (5) analyte-specific probes which specifically interact with different sub-structures of the same analyte, each analyte-specific probe comprising

(aa) a binding element (S) that specifically interacts with one of the different analytes to be encoded, and
(bb) an identifier element (T) comprising a nucleotide sequence which is unique to the analyte to be encoded (unique identifier sequence),

wherein the analyte-specific probes of a particular set of analyte-specific probes differ from the analyte-specific probes of another set of analyte-specific probes in the nucleotide sequence of the identifier element (T), wherein the analyte-specific probes in each set of analyte-specific probes binds to the same analyte and comprises the same nucleotide sequence of the identifier element (T) which is unique to said analyte; and

(B) contacting the sample with at least one set of decoding oligonucleotides per analyte, wherein in each set of decoding oligonucleotides for an individual analyte each decoding oligonucleotide comprises:

(aa) an identifier connector element (t) comprising a nucleotide sequence which is essentially complementary to at least a section of the unique identifier sequence of the identifier element (T) of the corresponding analyte-specific probe set, and
(bb) a translator element (c) comprising a nucleotide sequence allowing a specific hybridization of a signal oligonucleotide;

wherein the decoding oligonucleotides of a set for an individual analyte differ from the decoding oligonucleotides of another set for a different analyte in the first connect element (t); and

(C) contacting the sample with at least a set of signal oligonucleotides, each signal oligonucleotide comprising:

(aa) a translator connector element (C) comprising a nucleotide sequence which is essentially complementary to at least a section of the nucleotide sequence of a translator element (c) comprised in a decoding oligonucleotide, and
(bb) a signal element.

(D) Detecting the signal caused by the signal element;

(E) selectively removing the decoding oligonucleotides and signal oligonucleotides from the sample, thereby essentially maintaining the specific binding of the analyte-specific probes to the analytes to be encoded;

(F) Performing at least three (3) further cycles comprising steps B) to E) to generate an encoding scheme with a code word per analyte, wherein in particular the last cycle may stop with step (D).

As mentioned above, the method according to the present disclosure comprises selectively removing the decoding oligonucleotides and signal oligonucleotides from the sample, thereby essentially maintaining the specific binding of the analyte-specific probes to the analyte to be encoded. In particular all steps are performed sequentially. However some steps may be performed simultaneously, in particular the contacting steps A) to C), in particular B) and C).

By this measure the requirements for another round/cycle of binding further decoding oligonucleotides to the same analyte-specific probes are established, thus finally resulting in a code or encoding scheme comprising more than one signal. This step is realized by applying conditions and factors well known to the skilled person, e.g. pH, temperature, salt conditions, oligonucleotide concentration, polymers etc.

In another embodiment of the present disclosure, the method may comprise repeating steps (B)-(E) at least three times to generate an encoding scheme. With this measure a code of four signals in case of four cycles/rounds which are carried out by the user, where 'n' is an integer representing the number of rounds. The encoding capacity of the method according to the disclosure is herewith increased depending on the nature of the analyte and the needs of the operator. In an embodiment of the disclosure said encoding scheme is predetermined and allocated to the analyte to be encoded.

However, this measure enables a precise experimental set-up by providing the appropriate sequential order of the employed decoding and signal oligonucleotides and, therefore, allows the correct allocation of a specific analyte to a respective encoding scheme. The decoding oligonucleotides which are used in repeated steps (B)-(D2) may comprise a translator element (c2) which is identical with the translator element (c1) of the decoding oligonucleotides used in previous steps (B)-(E). In another embodiment of the disclosure decoding oligonucleotides are used in repeated steps (B)-(E) comprising a translator element (c2) which differs from the translator element (c1) of the decoding oligonucleotides used in previous steps (B)-(E). It is understood that the decoding elements may or may not be changed from round to round, i.e. in the second round (B)-(E) comprising the translator element c2, in the third round (B)-(E) comprising the translator element c3, in the fourth round (B)-(E) comprising the translator element c4 etc., wherein 'n' is an integer representing the number of rounds.

The signal oligonucleotides which are used in repeated steps (B)-(E) may comprise a signal element which is identical with the signal element of the decoding oligonucleotides used in previous steps (B)-(E). In a further embodiment of the disclosure signal oligonucleotides are used in repeated steps (B)-(E) comprising a signal element which differs from the signal element of the decoding oligonucleotides used in previous steps (B)-(E). In some embodiments no-signal oligonucleotides and/or no-signal decoding oligonucleotides for an individual analyte are used, resulting to the value 0 in the code word for this cycle/position. In some embodiments in a repeated cycle no decoding oligonucleotides for an individual analyte is contacted with the sample resulting also to the value 0 in the code word for this cycle/position.

By this measure each round the same or a different signal is provided resulting in an encoding scheme characterized by a signal sequence consisting of numerous different signals. This measure allows the creation of a unique code or code word which differs from all other code words of the encoding scheme. In another embodiment of the disclosure, the binding element (S) of the analyte-specific probe comprises a nucleic acid comprising a nucleotide sequence allowing a specific binding to the analyte to be encoded, preferably a specific hybridization to the analyte to be encoded.

In some advantageous embodiments, all steps are automated, in particular wherein steps B) to F) are automated, in particular by using a robotic system and/or an optical multiplexing system according to the present disclosure. In some examples, the steps may be performed in a fluidic system.

As mentioned above, with the methods according to the present disclosure an encoding scheme with a code word per analyte is generated. Therefore, each analyte may be associated with a specific code word, wherein said code word comprise a number of positions, and wherein each position corresponds to one cycle resulting in a plurality of distinguishable encoding schemes with the plurality of code words. In particular, said encoding scheme may be predetermined and allocated to the analyte to be encoded.

In some advantageous embodiments, the code words obtained for the individual analytes in the performed cycles comprise the detected signals and additionally at least one element corresponding to no detected signal like 0,1 or 0,1,2 etc. (see also Fig. 13 and Fig. 14). In particular, no signal is detected for at least one analyte within at least one cycle if using the a non-signal probe according to Figure 14, No. 2 to 4, or a non-signal decoding oligonucleotide as shown in Figure 14 No. 5, or if in one cycle no decoding oligonucleotide is contacted with the corresponding identifier sequence comprised on analyte-specific probe interacting with the corresponding analyte in the sample. In this cycle the position has the value zero (0).

In some advantageous embodiments, at least for one individual analyte a position of the code word is zero (0).In particular, the code word zero (0) is generated by using no decoding oligonucleotides having an identifier connector element (t) comprising a nucleotide sequence which is essentially complementary to at least a section of the unique identifier sequence of the identifier element (T) of a corresponding analyte-specific probe for an individual analyte. As mentioned above, in some embodiments, if at least for one individual analyte a position of the code word is zero (0) in this cycle no corresponding decoding oligonucleotides having an identifier connector element (t) comprising a nucleotide sequence which is essentially complementary to at least a section of the unique identifier sequence of the identifier element (T) of a corresponding analyte-specific probe for an individual analyte are used.

Furthermore, in some advantageous embodiments the sample is contacted with at least two different sets of signal oligonucleotides, wherein the signal oligonucleotides in each set comprise a different signal element and comprise a different connector element (C).

In more particular embodiments, the sample is contacted with at least two different sets of decoding oligonucleotides per analyte,

wherein the decoding oligonucleotides comprised in these different sets comprise the same identifier connector element (t) comprising a nucleotide sequence which is essentially complementary to at least a section of the unique identifier sequence of the identifier element (T) of the corresponding analyte-specific probe set, and

wherein the decoding oligonucleotides of the different sets per analyte differ in the translator element (c) comprising a nucleotide sequence allowing a specific hybridization of a signal oligonucleotide.

In more particular embodiments, the sample is contacted with at least two different sets of decoding oligonucleotides per analyte,

wherein the decoding oligonucleotides comprised in these different sets comprise the same identifier connector element (t) comprising a nucleotide sequence which is essentially complementary to at least a section of the unique identifier sequence of the identifier element (T) of the corresponding analyte-specific probe set, and

wherein the decoding oligonucleotides of the different sets per analyte differ in the translator element (c) comprising a nucleotide sequence allowing a specific hybridization of a signal oligonucleotide;

wherein only one set of decoding oligonucleotides per analyte is used per cycle, and/or wherein different sets of decoding oligonucleotides are used in different cycles in combination with the corresponding set of signal oligonucleotides in the same cycle.

In some advantageous embodiments, the number of different sets of decoding oligonucleotides per analyte comprising different translator elements (c) corresponds to the number of different sets of signal oligonucleotides comprising different connector elements (C). All sets of decoding oligonucleotides for the different analytes may comprise the same type(s) of translator element(s) (c). In some advantageous embodiments of the method according to the present disclosure, the sample is contacted with at least a set of non-signal decoding oligonucleotides for binding to a particular identifier element (T) of analyte-specific probes, wherein the decoding oligonucleotides in the same set of non-signal decoding oligonucleotides interacting with the same different identifier element (T), wherein each non-signal decoding oligonucleotide comprises an identifier connector element (t) comprising a nucleotide sequence which is essentially complementary to at least a section of a unique identifier sequence, and does not comprise a translator element (c) comprising a nucleotide sequence allowing a specific hybridization of a signal oligonucleotide.

As mentioned above, the sample may be contacted with at least two (2) different sets of non-signal decoding oligonucleotides for binding to at least two different identifier elements (T) of analyte-specific probes, each set of non-signal decoding oligonucleotides interacting with a different identifier element (T), wherein each non-signal decoding oligonucleotide comprises an identifier connector element (t) comprising a nucleotide sequence which is essentially complementary to at least a section of a unique identifier sequence, and does not comprise a translator element (c) comprising a nucleotide sequence allowing a specific hybridization of a signal oligonucleotide.

In some advantageous embodiments of the method according to the present disclosure, the different sets of non-signal decoding oligonucleotides may be comprised in a pre-mixture of different sets of non-signal decoding oligonucleotides or exist separately.

Furthermore, in some advantageous embodiments of the method according to the present disclosure, the sample is contacted with a set of non-signal oligonucleotides, each non-signal oligonucleotide comprising:

(aa) a translator connector element (C) comprising a nucleotide sequence which is essentially complementary to at least a section of the nucleotide sequence of the translator element (c), and
(bb) a quencher (Q), a signal element and a quencher (Q), or does not comprise a signal element.

In further embodiments, the sample may be contacted with:
at least two sets of non-signal oligonucleotides, each non-signal oligonucleotide comprising:

(aa) a translator connector element (C) comprising a nucleotide sequence which is essentially complementary to at least a section of the nucleotide sequence of the translator element (c), and
(bb) a quencher (Q), a signal element and a quencher (Q), or does not comprise a signal element.

As mentioned above, the different sets of non-signal oligonucleotides may be comprised in a pre-mixture of different sets of non-signal oligonucleotides or exist separately.

In further embodiments, the decoding oligonucleotides in a particular set of decoding oligonucleotides interacts with identical identifier elements (T) which are unique to a particular analyte.

As mentioned above, the different sets of decoding oligonucleotides may be comprised in a pre-mixture of different sets of decoding oligonucleotides or exist separately as well as the different sets of analyte-specific probes may be comprised in a pre-mixture of different sets of analyte-specific probes or exist separately as well the different sets of signal oligonucleotides may be comprised in a pre-mixture of different sets of signal oligonucleotides or exist separately.

In some advantageous embodiments of the method according to the present disclosure, the binding element (S) comprise a nucleic acid comprising a nucleotide sequence allowing a specific binding to the analyte to be encoded, preferably a specific hybridization to the analyte to be encoded.

In some advantageous embodiments of the method according to the present disclosure, after step A) and before step B) the non-bound analyte-specific probes may be removed, in particular by washing, further after step B) and before step C) the non-bound decoding oligonucleotides may be removed, in particular by washing further, after step C) and before step D) the non-bound signal oligonucleotides may be removed, in particular by washing.

In some advantageous embodiments of the method according to the present disclosure, the analyte specific probes may be incubated with the sample, thereby allowing a specific binding of the analyte specific probes to the analytes to be encoded, further the decoding oligonucleotides may be incubated with the sample, thereby allowing a specific hybridization of the decoding oligonucleotides to identifier elements (T) of the respective analyte-specific probes, further the signal oligonucleotides may be incubated with the sample, thereby allowing a specific hybridization of the signal oligonucleotides to translator elements (T) of the respective decoding oligonucleotides.

As mentioned above, the analyte to be encoded may be a nucleic acid, preferably DNA, PNA, RNA, in particular mRNA, a peptide, polypeptide, a protein or combinations thereof. Therefore, the binding element (S) may comprise an amino acid sequence allowing a specific binding to the analyte to be encoded. Examples for a binding element (S) are moieties which are affinity moieties from affinity substances or affinity substances in their entirety selected from the group consisting of antibodies, antibody fragments, anticalin proteins, receptor ligands, enzyme substrates, lectins, cytokines, lymphokines, interleukins, angiogenic or virulence factors, allergens, peptidic allergens, recombinant allergens, allergen-idiotypical antibodies, autoimmune-provoking structures, tissue-rejection-inducing structures, immunoglobulin constant regions and combinations thereof. In particular, the binding element (S) is an antibody or an antibody fragment selected from the group consisting of Fab, scFv; single domain, or a fragment thereof, bis scFv, Fab 2, Fab 3, minibody, diabody, triabody, tetrabody and tandab.

By this measure the method is further developed to such an extent that the encoded analytes can be detected by any means which is adapted to visualize the signal element. Examples of detectable physical features include e.g. light, chemical reactions, molecular mass, radioactivity, etc.

In some advantageous embodiments, the signal caused by the signal element, therefore in particular the binding of the signal oligonucleotides to the decoding oligonucleotides, interacting with the corresponding analyte probes, bound to the respective analyte is determined by:

(a) Imaging at least a portion of the sample; and/or
(b) Using an optical imaging technique; and/or
(c) Using a fluorescence imaging technique; and/or
(d) Multi-color fluorescence imaging technique; and/or
(e) Super-resolution fluorescence imaging technique.

The method according to the present disclosure may be used ideally for *in vitro* methods for diagnosis of a disease selected from the group comprising cancer, neuronal diseases, cardiovascular diseases, inflammatory diseases, autoimmune diseases, diseases due to a viral or bacterial infection, skin diseases, skeletal muscle diseases, dental diseases and prenatal diseases.

Further, the method according to the present disclosure may be used also ideally for *in vitro* methods for diagnosis of a disease in plants selected from the group comprising: diseases caused by biotic stress, preferably by infectious and/or parasitic origin, or diseases caused by abiotic stress, preferably caused by nutritional deficiencies and/or unfavorable environment.

Further, the method according to the present disclosure may be used also ideally for *in vitro* methods for screening, identifying and/or testing a substance and/or drug comprising:

(a) contacting a test sample comprising a sample with a substance and/or drug

(b) detecting different analytes in a sample by sequential signal-encoding of said analytes with a method according to the present disclosure.

In some advantageous embodiments, the spatial transcriptomics method described herein is used for specific detection of many different analytes in parallel. The technology allows to distinguish a higher number of analytes than different signals are available. The process includes at least four consecutive rounds of specific binding, signal detection and selective denaturation (if a next round is required), eventually producing a signal code. To decouple the dependency between the analyte specific binding and the oligonucleotides providing the detectable signal, a so called "decoding"-oligonucleotide is introduced. The decoding oligonucleotide transcribes the information of the analyte specific probe set to the signal oligonucleotides.

In a specific embodiment, the spatial transcriptomics method method may comprise the steps of 1. providing one or more analyte specific probe sets, the set of analyte specific probes consist of one or more different probes, each differing in the binding moiety that specifically interacts with the analyte, all probes of a single probe set are tethered to a sequence element (unique identifier), that is unique to a single probe set and allows the specific hybridization of a decoding oligonucleotide, 2. specific binding of the probe sets to their target binding sites of the analyte, 3. eliminating non-bound probes (e.g. by a wash step), 4. providing a mixture of decoding oligonucleotides that specifically hybridize to the unique identifier sequences of the probe sets, the decoding oligonucleotides comprise of at least two sequence elements, a first element that is complementary to the unique identifier sequences of the corresponding probe set and a second sequence element (translator element) that provides a sequence for the specific hybridization of a signal oligonucleotide, the translator element defines the type of signal that is recruited to the decoding oligonucleotide, 5. specific hybridization of the decoding oligonucleotides to the unique identifier sequences provided by the bound probe sets, 6. eliminating non-bound decoding oligonucleotides (e.g. by washing step), 7. providing a mixture of signal oligonucleotides, comprising of a signal that can be detected and a nucleic acid sequence that specifically hybridizes to the translator element of one of the decoding oligonucleotides used in the former hybridization step, 8. specific hybridization of the signal oligonucleotides, 9. eliminating non-bound signal oligonucleotides, 10. detection of the signals, 11. selective release of decoding oligonucleotides and signal oligonucleotides while the binding of specific probe sets to the analyte is almost or completely unaffected, 12. eliminating released decoding oligonucleotide and signal oligonucleotides (e.g. by a washing step) while the binding of specific probes sets to the analytes is almost or completely unaffected , repeating the steps 4 to 12 at least three times until the detection of a sufficient number of signals to generate an encoding scheme for each different analyte of interest. It is to be understood that the before-mentioned features and those to be mentioned in the following cannot only be used in the combination indicated in the respective case, but also in other combinations or in an isolated manner without departing from the scope of the disclosure.

The disclosure is now further explained by means of embodiments resulting in additional features, characteristics and advantages of the disclosure. The embodiments are of pure illustrative nature and do not limit the scope or range of the disclosure. The features mentioned in the specific embodiments are general features of the disclosure which are not only applicable in the specific embodiment but also in an isolated manner in the context of any embodiment of the disclosure.

The spatial transcriptomics method method disclosed herein is used for specific detection of many different analytes in parallel. The technology allows distinguishing a higher number of analytes than different signals are available. The process preferably includes at least two consecutive rounds of specific binding, signal detection and selective denaturation (if a next round is required), eventually producing a signal code. To decouple the dependency between the analyte specific binding and the oligonucleotides providing the detectable signal, a so called "decoding" oligonucleotide is introduced. The decoding oligonucleotide transcribes the information of the analyte specific probe set to the signal oligonucleotides.

The present disclosure pertains further to methods for detecting an analyte in a pathogen-comprising sample, comprising an inactivation of the pathogen within the sample without isolation of RNA and/or DNA from the pathogen or the sample, and

- attaching a plurality of analyte-specific probes to the analyte, wherein the analyte-specific probes independently attach to the analyte and wherein the analyte-specific probes share a common identifier segment (T);

- annealing a plurality of first decoding oligonucleotides to the analyte-specific probes, wherein the first decoding oligonucleotides share a first common region that is reverse complementary to the common identifier segment and a second common region;

- annealing a first signal oligonucleotide to at least one of the plurality of first decoding oligonucleotides such that an oligo tethered to the first signal oligonucleotide is reverse complementary to the second common region;

- detecting the first signal oligonucleotide;

- removing the plurality of first decoding oligonucleotides;

- annealing a plurality of second decoding oligonucleotides to the analyte-specific probes, wherein the second decoding oligonucleotides share a first common region that is reverse complementary to the common identifier segment and a second decoding oligonucleotides second common region that differs from the second common region of the first decoding;

- annealing a second signal oligonucleotide to at least one of the plurality of second decoding oligonucleotides such that an oligo tethered to the signal oligonucleotide is reverse complementary to the second decoding oligonucleotide second common region; and detecting the second signal oligonucleotide.

In particular, in the above mentioned embodiment a second aliquot of a plurality of first decoding oligonucleotides is annealed to the analyte-specific probes. Furthermore, a first aliquot of a plurality of first decoding oligonucleotides is annealed to the analyte-specific probes.
In some embodiments, no second signal oligonucleotide to the at least one of the plurality of first decoding oligonucleotides is annealed. In particular, no third signal oligonucleotide to the at least one of the plurality of first decoding oligonucleotides is annealed.

**Methods and Examples**

In an application variant, the analyte or target is nucleic acid, e.g. DNA or RNA, and the probe set comprises oligonucleotides that are partially or completely complementary to the whole sequence or a subsequence of the nucleic acid sequence to be detected (Figure 1). The nucleic acid sequence specific oligonucleotide probe sets comprising analyte-specific probes (1) including a binding element (S) that specifically hybridizes to the target nucleic acid sequence to be detected, and an identifier element (T) comprising a nucleotide sequence which is unique to said set of analyte-specific probes (unique identifier sequence).
In a further application variant, the analyte or target is a protein and the probe set comprises one or more proteins, e.g. antibodies (Figure 2). The protein specific probe set comprising analyte-specific probes (1) including a binding element (T) such as the (hyper-)variable region of an antibody, that specifically interacts with the target protein to be detected, and the identifier element (T).
In a further application variant, at least one analyte is a nucleic acid and at least a second analyte is a protein and at least the first probe set binds to the nucleic acid sequence and at least the second probe set binds specifically to the protein analyte. Other combinations are possible as well.
An Embodiments of the general method of the present disclosure may be:

Step 1: Applying the at least 20 analyte- or target-specific probe sets. The target nucleic acid sequence is incubated with a probe set consisting of oligonucleotides with sequences complementary to the target nucleic acid. In this example, a probe set of 5 different probes is shown, each comprising a sequence element complementary to an individual subsequence of the target nucleic acid sequence (S1 to S5). In this example, the regions do not overlap. Each of the oligonucleotides targeting the same nucleic acid sequence comprises the identifier element or unique identifier sequence (T), respectively.

Step 2: Hybridization of the probe set. The probe set is hybridized to the target nucleic acid sequence under conditions allowing a specific hybridization. After the incubation, the probes are hybridized to their corresponding target sequences and provide the identifier element (T) for the next steps.

Step 3: Eliminating non-bound probes. After hybridization, the unbound oligonucleotides are eliminated, e.g. by washing steps.

Step 4: Applying the decoding oligonucleotides. The decoding oligonucleotides consisting of at least two sequence elements (t) and (c) are applied. While sequence element (t) is complementary to the unique identifier sequence (T), the sequence element (c) provides a region for the subsequent hybridization of signal oligonucleotides (translator element).

Step 5: Hybridization of decoding oligonucleotides. The decoding oligonucleotides are hybridized with the unique identifier sequences of the probes (T) via their complementary first sequence elements (t). After incubation, the decoding oligonucleotides provide the translator sequence element (c) for a subsequent hybridization step.

Step 6: Eliminating the excess of decoding oligonucleotides. After hybridization, the unbound decoding oligonucleotides are eliminated, e.g. by washing steps.

Step 7: Applying the signal oligonucleotide. The signal oligonucleotides are applied. The signal oligonucleotides comprise at least one second connector element (C) that is essentially complementary to the translator sequence element (c) and at least one signal element that provides a detectable signal (F).

Step 8: Hybridization of the signal oligonucleotides. The signal oligonucleotides are hybridized via the complementary sequence connector element (C) to the translator element (c) of decoding oligonucleotide. After incubation, the signal oligonucleotides are hybridized to their corresponding decoding oligonucleotides and provide a signal (F) that can be detected.

Step 9: Eliminating the excess of signal oligonucleotides. After hybridization, the unbound signal oligonucleotides are eliminated, e.g. by washing steps.

Step 10: Signal detection. The signals provided by the signal oligonucleotides are detected.
The following steps (steps 11 and 12) are unnecessary for the last detection round.

Step 11: Selective denaturation. The hybridization between the unique identifier sequence (T) and the first sequence element (t) of the decoding oligonucleotides is dissolved. The destabilization can be achieved via different mechanisms well known to the trained person like for example: increased temperature, denaturing agents, etc. The target- or analyte-specific probes are not affected by this step.

Step 12: Eliminating the denatured decoding oligonucleotides. The denatured decoding oligonucleotides and signal oligonucleotides are eliminated (e.g. by washing steps) leaving the specific probe sets with free unique identifier sequences, reusable in a next round of hybridization and detection (steps 4 to 10). This detection cycle (steps 4 to 12) is repeated at least four times until the planed encoding scheme is completed.

Another Embodiment of the general method of the present disclosure using multi-decoders may be (Fig. 16):

Step 1: Target nucleic acids: In this example three different target nucleic acids (A), (B) and (C) have to be detected and differentiated by using only two different types of signal oligonucleotides. Before starting the experiment, a certain encoding scheme is set. In this example, the three different nucleic acid sequences are encoded by three rounds of detection with three different signal types (1), (2) and (1/2) and a resulting hamming distance of 3 to allow for error detection. The planed code words are:

sequence A: (1) - (1) - (2)
sequence B: (2) - (2) - (1/2)
sequence C: (1/2) - (1/2) - (1)

Step 2: Hybridization of the probe sets: For each target nucleic acid, an own probe set is applied, specifically hybridizing to the corresponding nucleic acid sequence of interest. Each probe set provides a unique identifier sequence (T1), (T2) or (T3). This way each different target nucleic acid is uniquely labeled. In this example sequence (A) is labeled with (T1), sequence (B) with (T2) and sequence (C) with (T3). The illustration in Fig. 16 summarizes Steps 1 to 3 of Fig. 3.

Step 3: Hybridization of the decoding oligonucleotides and multi-decoders: For each unique identifier present, a certain decoding oligonucleotide or multi-decoder is applied specifically hybridizing to the corresponding unique identifier sequence by its first sequence element (here (t1) to (T1), (t2) to (T2) and (t3) to (T3)). Each of the decoding oligonucleotides or multi-decoders provides a translator or two translator elements that define the signals that will be generated after hybridization of signal oligonucleotides. Here nucleic acid sequence (A) is labeled with (c1), (B) is labeled with (c2) and (C) is labeled with both translator elements (c1) and (c2) resulting in the signal (1/2). The illustration in Fig. 16 summarizes steps 4 to 6 of Fig. 3.

Step 4: Hybridization of signal oligonucleotides: For each type of translator element, a signal oligonucleotide with a certain signal, differentiable from signals of other signal oligonucleotides, is applied. This signal oligonucleotide can specifically hybridize to the corresponding translator element. The illustration in Fig. 16 summarizes steps 7 to 9 of Fig. 3.

Step 5: Signal detection for the encoding scheme: The different signals are detected. Note that in this example the nucleic acids (A), (B) and (C) can already be distinguished after the first round of detection. This is in contrast to the step 5 of Fig. 5 explained by the additional signal type (1/2) that can be realized due to multi-decoders. Although nucleic acid sequences can already be distinguished, the additional rounds contribute to the planned hamming distance of 3. The illustration in Fig. 16 corresponds to step 10 of Fig. 3.

Step 6: Selective denaturation: The decoding (and signal) oligonucleotides and/or multi-decoders of all nucleic acid sequences to be detected are selectively denatured and eliminated as described in steps 11 and 12 of Fig.3. Afterwards the unique identifier sequences of the different probe sets can be used for the next round of hybridization and detection.

Step 7: Second round of detection: A next round of hybridization and detection is done as described in steps 3 to 5. Note that in this new round the mix of different decoding oligonucleotides and multi-decoders is changed. For example, decoding oligonucleotide of nucleic acid sequence (A) used in the first round comprised of sequence elements (t1) and (c1) while the new multi decoder of round 2 comprises of the sequence elements (t1), (c1) and (c2). Note that now a hamming distance of 2 is already given after 2 rounds, which is the final result of the example in Fig. 3 after 3 rounds.

Step 8: Third round of detection: Again, a new combination of decoding oligonucleotides and/or multi-decoders is used leading to new signal combinations. After signal detection, the resulting code words for the three different nucleic acid sequences are not only unique and therefore distinguishable but comprise a hamming distance of 3 to other code words. Due to the hamming distance, an error in the detection of the signals (signal exchange) would not result in a valid code word and therefore could be detected and because of hamming distance 3 also corrected, in contrast to the encoding scheme of Fig. 3. This way three different nucleic acids can be distinguished in three detection rounds with two different signals, allowing an error detection and correction.

Note that in every round of detection, the type of signal provided by a certain unique identifier is controlled by the use of a certain decoding oligonucleotide. As a result, the sequence of decoding oligonucleotides applied in the detection cycles transcribes the binding specificity of the probe set into a unique signal sequence.

The steps of decoding oligonucleotide hybridization (steps 4 to 6) and signal oligonucleotide hybridization (steps 7 to 9) can also be combined in two alternative ways as shown in Figure 4.

Opt. 1: Simultaneous hybridization. Instead of the steps 4 to 9 of Figure 3, specific hybridization of decoding oligonucleotides and signal oligonucleotides can also be done simultaneously leading to the same result as shown in step 9 of Figure 3, after eliminating the excess decoding- and signal oligonucleotides.

Opt. 2: Preincubation. Additionally to option 1 of Figure 3, decoding- and signal oligonucleotides can be preincubated in a separate reaction before being applied to the target nucleic acid with the already bound specific probe set.

<u>1. Example for signal encoding of three different nucleic acid sequences by two different signal types and three detection rounds</u>

Figure 3 shows the general concept of generation and detection of specific signals mediated by decoding oligonucleotides. It does not show the general concept of encoding that can be achieved by this procedure. To illustrate the use of the process shown in Figure 3 for the generation of an encoding scheme, Figure 5 shows a general example for a multiple round encoding experiment with three different nucleic acid sequences. In this example, the encoding scheme includes error detection.

Step 1: Target nucleic acids. In this example three different target nucleic acids (A), (B) and (C) have to be detected and differentiated by using only two different types of signal. Before starting the experiment, a certain encoding scheme is set. In this example, the three different nucleic acid sequences are encoded by three rounds of detection with two different signals (1) and (2) and a resulting hamming distance of 2 to allow for error detection. The planed code words are:

sequence A: (1) - (2) - (2);
sequence B: (1) - (1) - (1);
sequence C: (2) - (1) - (2).

Step 2: Hybridization of the probe sets. For each target nucleic acid, an own probe set is applied, specifically hybridizing to the corresponding nucleic acid sequence of interest. Each probe set provides a unique identifier sequence (T1), (T2) or (T3). This way each different target nucleic acid is uniquely labeled. In this example sequence (T) is labeled with (T1), sequence (B) with (T2) and sequence (C) with (T3). The illustration summarizes steps 1 to 3 of Figure 3.

Step 3: Hybridization of the decoding oligonucleotides. For each unique identifier present, a certain decoding oligonucleotide is applied specifically hybridizing to the corresponding unique identifier sequence by its first sequence element (here (t1) to (T1), (t2) to (T2) and (t3) to (T3)). Each of the decoding oligonucleotides provides a translator element that defines the signal that will be generated after hybridization of signal oligonucleotides. Here nucleic acid sequences (A) and (B) are labeled with the translator element (c1) and sequence (C) is labeled with (c2). The illustration summarizes steps 4 to 6 of Figure 3.

Step 4: Hybridization of signal oligonucleotides. For each type of translator element, a signal oligonucleotide with a certain signal (2), differentiable from signals of other signal oligonucleotides, is applied. This signal oligonucleotide can specifically hybridize to the corresponding translator element. The illustration summarizes steps 7 to 9 of Figure 3.

Step 5: Signal detection for the encoding scheme. The different signals are detected. Note that in this example the nucleic acid sequence (C) can be distinguished from the other sequences by the unique signal (2) it provides, while sequences (A) and (B) provide the same kind of signal (1) and cannot be distinguished after the first cycle of detection. This is due to the fact, that the number of different nucleic acid sequences to be detected exceeds the number of different signals available. The illustration corresponds to step 10 of Figure 3.

Step 6: Selective denaturation. The decoding (and signal) oligonucleotides of all nucleic acid sequences to be detected are selectively denatured and eliminated as described in steps 11 and 12 of Figure 3. Afterwards the unique identifier sequences of the different probe sets can be used for the next round of hybridization and detection.

Step 7: Second round of detection. A next round of hybridization and detection is done as described in steps 3 to 5. Note that in this new round the mix of different decoding oligonucleotides is changed. For example, decoding oligonucleotide of nucleic acid sequence (A) used in the first round comprised of sequence elements (t1) and (c1) while the new decoding oligonucleotide comprises of the sequence elements (t1) and (c2). Note that now all three sequences can clearly be distinguished due to the unique combination of first and second round signals.

Step 8: Third round of detection. Again, a new combination of decoding oligonucleotides is used leading to new signal combinations. After signal detection, the resulting code words for the three different nucleic acid sequences are not only unique and therefore distinguishable but comprise a hamming distance of 2 to other code words. Due to the hamming distance, an error in the detection of the signals (signal exchange) would not result in a valid code word and therefore could be detected. By this way three different nucleic acids can be distinguished in three detection rounds with two different signals, allowing error detection.

## 2. Advantages over prior art technologies

### *Coding strategy*

Compared to state-of-the-art methods, one particular advantage of the method according to the disclosure is the use of decoding oligonucleotides breaking the dependencies between the target specific probes and the signal oligonucleotides.

Without decoupling target specific probes and signal generation, two different signals can only be generated for a certain target if using two different molecular tags. Each of these molecular tags can only be used once. Multiple readouts of the same molecular tag do not increase the information about the target. In order to create an encoding scheme, a change of the target specific probe set after each round is required (SeqFISH) or multiple molecular tags must be present on the same probe set (like merFISH, intronSeqFISH).

Following the method according to the disclosure, different signals are achieved by using different decoding oligonucleotides reusing the same unique identifier (molecular tag) and a small number of different, mostly cost-intensive signal oligonucleotides. This leads to several advantages in contrast to the other methods.

(1) The encoding scheme is not defined by the target specific probe set as it is the case for all other methods of prior art. Here the encoding scheme is transcribed by the decoding oligonucleotides. This leads to a much higher flexibility concerning the number of rounds and the freedom in signal choice for the code words. Looking on the methods of prior art (e.g. merFISH or intronSeqFISH), the encoding scheme (number, type and sequence of detectable signals) for all target sequences is predefined by the presence of the different tag sequences on the specific probe sets (4 of 16 different tags per probe set in the case of merFISH and 5 of 60 different tags in the case of intron FISH). In order to produce a sufficient number of different tags per probe set, the methods use rather complex oligonucleotide designs with several tags present on one target specific oligonucleotide. In order to change the encoding scheme for a certain target nucleic acid, the specific probe set has to be replaced. The method according to the disclosure describes the use of a single unique tag sequence (unique identifier) per analyte, because it can be reused in every detection round to produce a new information. The encoding scheme is defined by the order of decoding oligonucleotides that are used in the detection rounds. Therefore, the encoding scheme is not predefined by the specific probes (or the unique tag sequence) but can be adjusted to different needs, even during the experiment. This is achieved by simply changing the decoding oligonucleotides used in the detection rounds or adding additional detection rounds.

(2) The number of different signal oligonucleotides must match the number of different tag sequences with methods of prior art (16 in the case of merFISH and 60 in the case of intronSeqFISH). Using the method according to the disclosure, the number of different signal oligonucleotides matches the number of different signals used. Due to this, the number of signal oligonucleotides stays constant for the method described here and never exceeds the number of different signals but increases with the complexity of the encoding scheme in the methods of prior art (more detection rounds more different signal oligonucleotides needed). As a result, the method described here leads to a much lower complexity (unintended interactions of signal oligonucleotides with environment or with each other) and dramatically reduces the cost of the assay since the major cost factor are the signal oligonucleotides.

(3) In the methods of prior art, the number of different signals generated by a target specific probe set is restricted by the number of different tag sequences the probe set can provide. Since each additional tag sequence increases the total size of the target specific probe, there is a limitation to the number of different tags a single probe can provide. This limitation is given by the size dependent increase of several problems (unintended inter- and intramolecular interactions, costs, diffusion rate, stability, errors during synthesis etc.). Additionally, there is a limitation of the total number of target specific probes that can be applied to a certain analyte. In case of nucleic acids, this limitation is given by the length of the target sequence and the proportion of suitable binding sites. These factors lead to severe limitations in the number of different signals a probe set can provide (4 signals in the case of merFISH and 5 signals in the case of intronSeqFISH). This limitation substantially affects the number of different code words that can be produced with a certain number of detection rounds. In the approach of the disclosure only one tag is needed and can be freely reused in every detection round. This leads to a low oligonucleotide complexity/length and at the same time to the maximum encoding efficiency possible (number of colors$^{\text{number of rounds}}$). The vast differences of coding capacity of our method compared to the other methods is shown in Figures 1 and 5. Due to this in approach of the disclosure a much lower number of detection rounds is needed to produce the same amount of information. A lower number of detection rounds is connected to lower cost, lower experimental time, lower complexity, higher stability and success rate, lower amount of data to be collected and analyzed and a higher accuracy of the results.

*Coding capacity*

All three methods compared in the Table 1 below use specific probe sets that are not denatured between different rounds of detection. For intronSeqFISH there are four detection rounds needed to produce the pseudo colors of one coding round, therefore data is only given for rounds 4, 8,12,16 and 20. The merFISH-method uses a constant number of 4 signals, therefore the data starts with the smallest number of rounds possible. After 8 detection rounds our method exceeds the maximum coding capacity reached with 20 rounds of merFISH (depicted with one asterisk) and after 12 rounds of detection the maximum coding capacity of intron FISH is exceeded (depicted with two asterisks). For the method according to the disclosure usage of 3 different signals is assumed (as is with intronSeqFISH).

Table 1: Comparison of coding capacity

| NUMBER OF ROUNDS: | CODING CAPACITY | | |
|---|---|---|---|
| | **Method of the present disclosure** | **intron FISH** | **merFISH** |
| 1 | 3 | - | - |
| 2 | 9 | - | - |
| 3 | 27 | - | - |
| 4 | 81 | 12 | 1 |
| 5 | 243 | - | 5 |
| 6 | 729 | - | 15 |
| 7 | 2187 | - | 35 |
| 8* | 6561 | 144 | 70 |
| 9 | 19683 | - | 126 |
| 10 | 59049 | - | 210 |
| 11 | 177147 | - | 330 |
| 12** | 531441 | 1728 | 495 |
| 13 | 1594323 | - | 715 |

(continued)

| NUMBER OF ROUNDS: | CODING CAPACITY | | |
|---|---|---|---|
| | Method of the present disclosure | intron FISH | merFISH |
| 14 | 4782969 | - | 1001 |
| 15 | 14348907 | - | 1365 |
| 16 | 43046721 | 20736 | 1820 |
| 17 | 129140163 | - | 2380 |
| 18 | 387420489 | - | 3060 |
| 19 | 1162261467 | - | 3876 |
| 20 | 3486784401 | 248832 | 4845 |

As shown in Figure 6 the number of code words for merFISH does not exponentially increase with the number of detection cycles but gets less effective with each added round. In contrast, the number of code words for intronSeqFISH in the method according to the disclosure increases exponentially. The slope of the curve for the proposed method is much higher than that of intron FISH, leading to more than 10,000 times more code words usable after 20 rounds of detection.

Note that this maximum efficiency of coding capacity is also reached in case of seqFISH, where specific probes are denatured after every detection round and a new probe set is specifically hybridized to the target sequence for each detection round. However, this method has major downsides to technologies using only one specific hybridization for their encoding scheme (all other methods):

(1) For the efficient denaturation of the specific probes, rather crude conditions must be used (high temperatures, high concentrations of denaturing agent, long incubation times) leading to much higher probability for the loss or the damage of the analyte.

(2) For each round of detection an own probe set has to be used for every target nucleic acid sequence. Therefore, the number of specific probes needed for the experiment scales with the number of different signals needed for the encoding scheme. This dramatically increases the complexity and the cost of the assay.

(3) Because the hybridization efficiency of every target nucleic acid molecule is subject to some probabilistic effects, the fluctuations of signal intensity between the different detection rounds is much higher than in methods using only one specific hybridization event, reducing the proportion of complete codes.

(4) The time needed for the specific hybridization is much longer than for the hybridization of signal oligonucleotides or decoding oligonucleotides (as can be seen in the method parts of the intronSeqFISH, merFISH and seqFISH publications), which dramatically increases the time needed to complete an experiment.

Due to these reasons all other methods use a single specific hybridization event and accept the major downside of lower code complexity and therefore the need of more detection rounds and a higher oligonucleotide design complexity.

The method according to the disclosure combines the advantages of seqFISH (mainly complete freedom concerning the encoding scheme) with all advantages of methods using only one specific hybridization event while eliminating the major problems of such methods.

Note that the high numbers of code words produced after 20 rounds can also be used to introduce higher hamming distances (differences) between different code words, allowing error detection of 1, 2 or even more errors and even error corrections. Therefore, even very high coding capacities are still practically relevant.

As mentioned above, the usage of multi-decoders further increases the coding capacity of the encoding scheme. Instead of being limited to the having exactly the same number of different signal types as different signal oligonucleotides and corresponding translator elements, the use of multi-decoders increases the signal types that can be used to: (N x (N+1))/2 (with N being the number of different signal oligonucleotides used). For the code used in table 1 with 3 different signal oligonucleotides this would mean the following 7 different signal types could be used: (S1),(S2),(S3),(S1/S2),(S1/S3),(S2/S3),(S1/S2/S3). The effect to the coding efficiency can be seen in Table 1b and Figure 17.

| NUMBER OF ROUNDS: | CODING CAPACITY | | | |
|---|---|---|---|---|
| | Method of the present disclosure | Method of the present disclosure with multi-decoders | intron FISH | merFISH |
| 1 | 3 | 7 | - | - |
| 2 | 9 | 49 | - | - |
| 3 | 27 | 343 | - | - |
| 4 | 81 | 2401 | 12 | 1 |
| 5* | 243 | 16807 | - | 5 |
| 6 | 729 | 117649 | - | 15 |
| 7** | 2187 | 823543 | - | 35 |
| 8 | 6561 | 5764801 | 144 | 70 |
| 9 | 19683 | 40353607 | - | 126 |
| 10 | 59049 | 282475249 | - | 210 |
| 11 | 177147 | 1977326743 | - | 330 |
| 12*** | 531441 | 1,3841 x 10^10 | 1728 | 495 |
| 13 | 1594323 | 9,6889 x 10^10 | - | 715 |
| 14 | 4782969 | 6,7822 x 10^11 | - | 1001 |
| 15 | 14348907 | 4,7476 x 10^12 | - | 1365 |
| 16 | 43046721 | 3,3233 x 10^13 | 20736 | 1820 |
| 17 | 129140163 | 2,3263 x 10^14 | - | 2380 |
| 18 | 387420489 | 1,6284 x 10^15 | - | 3060 |
| 19 | 1162261467 | 1,1399 x 10^16 | - | 3876 |
| 20 | 3486784401 | 7,9792 x 10^16 | 248832 | 4845 |

Table 1b shows the coding capacity of the four methods. All four methods compared in the table use specific probe sets that are not denatured between different rounds of detection. For intronSeqFISH there are four detection rounds needed to produce the pseudo colors of one coding round, therefore data is only given for rounds 4, 8,12,16 and 20. The merFISH-method uses a constant number of 4 signals, therefore the data starts with the smallest number of rounds possible. After 4 detection rounds the method with multi-decoders as described here exceeds the maximum coding capacity reached with 20 rounds of merFISH (depicted with one asterisk), after 7 rounds of detection the maximum coding capacity of intron FISH is exceeded (depicted with two asterisks) and after 12 rounds of detection the maximum coding capacity of the method of the present disclosure is exceeded (depicted with three asterisks). The usage of 3 different signal oligonucleotides is assumed (as is with intronSeqFISH).

3. Selective denaturation, oligonucleotide assembly and reuse of unique identifiers are surprisingly efficient

A key factor of the method according to the disclosure is the consecutive process of decoding oligonucleotide binding, signal oligonucleotide binding, signal detection and selective denaturation. In order to generate an encoding scheme, this process has to be repeated several times (depending on the length of the code word). Because the same unique identifier is reused in every detection cycle, all events from the first to the last detection cycle are depending on each other. Additionally, the selective denaturation depends on two different events: While the decoding oligonucleotide has to be dissolved from the unique identifier with highest efficiency, specific probes have to stay hybridized with highest efficiency.

Due to this the efficiency E of the whole encoding process can be described by the following equation:

$$E = B_{sp} \times (B_{de} \times B_{si} \times E_{de} \times S_{sp})^n$$

E = total efficiency
$B_{sp}$= binding of specific probes
$B_{de}$= binding of decoding oligonucleotides
$S_{si}$ = binding of signal oligonucleotides
$E_{de}$= elimination of decoding oligonucleotides
$S_{sp}$= stability of specific probes during elimination process
n = number of detection cycles

Based on this equation the efficiency of each single step can be estimated for a given total efficiency of the method. The calculation is hereby based on the assumption, that each process has the same efficiency. The total efficiency describes the portion of successfully decodable signals of the total signals present.

The total efficiency of the method is dependent on the efficiency of each single step of the different factors described by the equation. Under the assumption of an equally distributed efficiency the total efficiency can be plotted against the single step efficiency as shown in Figure 7. As can be seen, a practically relevant total efficiency for an encoding scheme with 5 detection cycles can only be achieved with single step efficiencies clearly above 90%. For example, to achieve a total efficiency of 50% an average efficiency within each single step of 97.8% is needed. These calculations are even based on the assumption of a 100% signal detection and analysis efficiency. Due to broad DNA melting curves of oligonucleotides of a variety of sequences, the inventors assumed prior to experiments that the selective denaturation would work less efficient for denaturation of decoding oligonucleotides and that sequence specific binding probes are not stable enough. In contrast to this assumption, we found a surprising effectiveness of all steps and a high stability of sequence specific probes during selective denaturation.

Experimentally, the inventors achieved a total decoding efficiency of about 30% to 65% based on 5 detection cycles. A calculation of the efficiency of each single step (Bsp, Bde, Bsi, Ede, Ssp) by the formula given above revealed an average efficiency of about 94.4% to 98%. These high efficiencies are very surprising and cannot easily be anticipated by a well-trained person in this field.

4. Experimental data

Background

The experiment shows the specific detection of 10 to 50 different mRNAs species in parallel with single molecule resolution. It is based on 5 detection cycles, 3 different fluorescent signals and an encoding scheme without signal gaps and a hamming distance of 2 (error detection). The experiment proofs the enablement and functionality of the method according to the disclosure.

Oligonucleotides and their sequences

All oligonucleotide sequences used in the experiment (target specific probes, decoding oligonucleotides, signal oligonucleotides) are listed in the sequence listing of the appendix. The signal oligonucleotide R:ST05*O_Atto594 was ordered from biomers.net GmbH. All other oligonucleotides were ordered from Integrated DNA Technologies. Oligonucleotides were dissolved in water. The stock solutions (100 μM) were stored at -20°C.

Experimental overview

The 50 different target specific probe sets are divided into 5 groups. The name of the transcript to be detected and the name of the target specific probe set are the same (transcript variant names of www.ensemble.org). The term "new" indicates a revised probe design. All oligonucleotide sequences of the probe sets can be found in the sequence listing. The table lists the unique identifier name of the probe set as well as the names of the decoding oligonucleotides used in the different detection cycles. The resulting code shows the sequence of fluorescent signals generated during the 5 detection cycles (G(reen)=Alexa Fluor 488, O(range)= Atto 594, Y(ellow)= Alexa Fluor 546).

Table 2: Experimental overview

| | | Decoding oligonucleotides in detection cycle: | | | | | |
|---|---|---|---|---|---|---|---|
| **target transcript** | **unique identifier** | **1** | **2** | **3** | **4** | **5** | **resulting code** |
| **Group 1** | | | | | | | |
| DDX5-201 | ST21 | ST21-ST07 | ST21-ST05 | ST21-ST07 | ST21-ST05 | ST21-ST06 | GOGOY |
| RAD17-208 | ST02 | ST02-ST06 | ST02-ST07 | ST02-ST06 | ST02-ST06 | ST02-ST07 | YGYYG |
| SPOCK1-202 | ST03 | ST03-ST06 | ST03-ST06 | ST03-ST07 | ST03-ST05 | ST03-ST05 | YYGOO |
| FBXO32-203 | ST04 | ST04-ST07 | ST04-ST06 | ST04-ST06 | ST04-ST06 | ST04-ST05 | GYYYO |
| THRAP3-203 | ST14 | ST14-ST07 | ST14-ST05 | ST14-ST05 | ST14-ST07 | ST14-ST05 | GOOGO |
| GART-203 | ST11 | ST11-ST06 | ST11-ST07 | ST11-ST06 | ST11-ST05 | ST11-ST05 | YGYOO |
| KAT2A-201 | ST13 | ST13-ST06 | ST13-ST06 | ST13-ST07 | ST13-ST06 | ST13-ST07 | YYGYG |
| HPRT1-201 | ST12 | ST12-ST06 | ST12-ST07 | ST12-ST07 | ST12-ST06 | ST12-ST06 | YGGYY |
| CCNA2-201 | ST22 | ST22-ST05 | ST22-ST07 | ST22-ST06 | ST22-ST06 | ST22-ST05 | OGYYO |
| NKRF-201 | ST23 | ST23-ST05 | ST23-ST06 | ST23-ST07 | ST23-ST06 | ST23-ST05 | OYGYO |
| **Group 2** | | | | | | | |
| CCNE1-201-new | NT01 | NT01-ST07 | NT01-ST07 | NT01-ST06 | NT01-ST06 | NT01-ST06 | GGYYY |
| COG5-201 | NT03 | NT03-ST06 | NT03-ST06 | NT03-ST05 | NT03-ST05 | NT03-ST06 | YYOOY |
| FBN1-201 | NT04 | NT04-ST05 | NT04-ST07 | NT04-ST06 | NT04-ST07 | NT04-ST07 | OGYGG |
| DYNC1H1-201 | NT05 | NT05-ST07 | NT05-ST06 | NT05-ST07 | NT05-ST06 | NT05-ST06 | GYGYY |
| CKAP5-202 | NT06 | NT06-ST05 | NT06-ST06 | NT06-ST07 | NT06-ST05 | NT06-ST06 | OYGOY |
| KRAS-202 | NT07 | NT07-ST06 | NT07-ST05 | NT07-ST06 | NT07-ST06 | NT07-ST05 | YOYYO |
| EGFR-207 | NT08 | NT08-ST07 | NT08-ST06 | NT08-ST05 | NT08-ST05 | NT08-ST05 | GYOOO |
| TP53-205 | NT09 | NT09-ST06 | NT09-ST05 | NT09-ST06 | NT09-ST07 | NT09-ST07 | YOYGG |
| NF1-204 | XT01 | XT01-ST06 | XT01-ST06 | XT01-ST07 | XT01-ST07 | XT01-ST06 | YYGGY |
| NF2-204 | XT02 | XT02-ST07 | XT02-ST06 | XT02-ST05 | XT02-ST06 | XT02-ST07 | GYOYG |

(continued)

| | | | | | | | |
|---|---|---|---|---|---|---|---|
| **Group 3** | | | | | | | |
| ACO2-201 | XT03 | XT03-ST06 | XT03-ST06 | XT03-ST06 | XT03-ST07 | XT03-ST05 | YYYGO |
| AKT1-211 | XT04 | XT04-ST07 | XT04-ST06 | XT04-ST07 | XT04-ST07 | XT04-ST05 | GYGGO |
| LYPLAL1-202 | XT05 | XT05-ST07 | XT05-ST05 | XT05-ST06 | XT05-ST05 | XT05-ST05 | GOYOO |
| PKD2-201 | XT06 | XT06-ST06 | XT06-ST07 | XT06-ST05 | XT06-ST05 | XT06-ST07 | YGOOG |
| ENG-204 | XT09 | XT09-ST05 | XT09-ST05 | XT09-ST06 | XT09-ST06 | XT09-ST06 | OOYYY |
| FANCE-201 | XT10 | XT10-ST05 | XT10-ST07 | XT10-ST06 | XT10-ST05 | XT10-ST06 | OGYOY |
| MET-201 | XT12 | XT12-ST05 | XT12-ST06 | XT12-ST05 | XT12-ST06 | XT12-ST06 | OYOYY |
| NOTCH2-201 | XT13 | XT13-ST05 | XT13-ST05 | XT13-ST06 | XT13-ST07 | XT13-ST05 | OOYGO |
| SPOP-206 | XT14 | XT14-ST05 | XT14-ST07 | XT14-ST05 | XT14-ST07 | XT14-ST06 | OGOGY |
| ABL1-202 | XT16 | XT16-ST05 | XT16-ST06 | XT16-ST06 | XT16-ST05 | XT16-ST05 | OYYOO |
| **Group 4** | | | | | | | |
| ATP11C-202 | XT17 | XT17-ST07 | XT17-ST06 | XT17-ST06 | XT17-ST05 | XT17-ST06 | GYYOY |
| BCR-202 | XT18 | XT18-ST05 | XT18-ST06 | XT18-ST06 | XT18-ST07 | XT18-ST06 | OYYGY |
| CAV1-205 | XT19 | XT19-ST07 | XT19-ST05 | XT19-ST06 | XT19-ST07 | XT19-ST06 | GOYGY |
| CDK2-201 | XT20 | XT20-ST05 | XT20-ST05 | XT20-ST07 | XT20-ST07 | XT20-ST06 | OOGGY |
| DCAF1-202 | XT201 | XT201-ST06 | XT201-ST06 | XT201-ST05 | XT201-ST07 | XT201-ST07 | YYOGG |
| FHOD1-201 | XT202 | XT202-ST05 | XT202-ST07 | XT202-ST07 | XT202-ST05 | XT202-ST07 | OGGOG |
| GMDS-202 | XT203 | XT203-ST07 | XT203-ST05 | XT203-ST07 | XT203-ST06 | XT203-ST05 | GOGYO |
| IFNAR1-201 | XT204 | XT204-ST06 | XT204-ST07 | XT204-ST05 | XT204-ST07 | XT204-ST05 | YGOGO |
| NSMF-203 | XT206 | XT206-ST07 | XT206-ST06 | XT206-ST07 | XT206-ST05 | XT206-ST07 | GYGOG |
| POLA2-201 | XT208 | XT208-ST06 | XT208-ST06 | XT208-ST06 | XT208-ST05 | XT208-ST07 | YYYOG |
| **Group 5** | | | | | | | |
| BRCA1-210new | NT10 | NT10-ST07 | NT10-ST05 | NT10-ST06 | NT10-ST06 | NT10-ST07 | GOYYG |

(continued)

| Group 5 | | | | | | | |
|---|---|---|---|---|---|---|---|
| JAK1-201new | XT11 | XT11-ST05 | XT11-ST05 | XT11-ST07 | XT11-ST06 | XT11-ST07 | OOGYG |
| STRAP-202 | XT207 | XT207-ST07 | XT207-ST07 | XT207-ST06 | XT207-ST05 | XT207-ST07 | GGYOG |
| SERPINB5-201 | XT209 | XT209-ST07 | XT209-ST05 | XT209-ST05 | XT209-ST05 | XT209-ST07 | GOOOG |
| SETX-201 | XT210 | XT210-ST06 | XT210-ST07 | XT210-ST06 | XT210-ST07 | XT210-ST06 | YGYGY |
| WDFY1-201 | XT212 | XT212-ST05 | XT212-ST07 | XT212-ST05 | XT212-ST06 | XT212-ST07 | OGOYG |
| TACC1-201 | XT213 | XT213-ST05 | XT213-ST07 | XT213-ST07 | XT213-ST07 | XT213-ST05 | OGGGO |
| KIF2A-203 | XT214 | XT214-ST07 | XT214-ST07 | XT214-ST06 | XT214-ST07 | XT214-ST05 | GGYGO |
| CDT1-201 | XT215 | XT215-ST07 | XT215-ST07 | XT215-ST07 | XT215-ST05 | XT215-ST05 | GGGOO |
| CENPE-202 | XT216 | XT216-ST07 | XT216-ST06 | XT216-ST05 | XT216-ST07 | XT216-ST06 | GYOGY |

Variations of the experiment

Some variations of the experiment have been performed. Experiments 1 to 4 mainly differ in the number of transcripts detected in parallel. The groups listed as target specific probe sets refer to table 6. Experiments 5 to 8 are single round, single target controls for comparison with the decoded signals.

Table 3: Variations of the experiment

| Experiment | Target specific probe sets used | Nr. of detection cycles | Imaging with trolox |
|---|---|---|---|
| 1. 50 transcripts_T+ | Groups 1 to 5 of table 6 | 5 | + |
| 2. 50 transcripts_T- | Groups 1 to 5 of table 6 | 5 | - |
| 3. 30 transcripts_T+ | Groups 2 to 4 of table 6 | 5 | + |
| 4. 10 transcripts_T+ | Group 1 of table 6 | 5 | + |
| 5. DDX5 | DDX5-ST21 | 1 | - |
| 6. RAD17 | RAD17-ST02 | 1 | - |
| 7. SPOCK1 | SPOCK1-ST03 | 1 | - |
| 8. THRAP3 | THRAP3-ST14 | 1 | - |

Experimental details

*A. Seeding and cultivation of cells*

HeLa cells were grown in HeLa cell culture medium to nearly 100% confluency. The HeLa cell culture medium comprises DMEM (Thermo Fisher, Cat.: 31885) with 10% FCS (Biochrom, Cat.: S0415), 1% Penicillin-Streptomycin (Sigma-Adrich, Cat.: P0781) and 1% MEM Non-Essential Amino Acids Solution (Thermo Fisher, Cat.: 11140035). After aspiration of cell culture medium, cells were trypsinized by incubation with trypsin EDTA solution (Sigma-Aldrich, Cat.: T3924) for 5 min at 37°C after a washing step with PBS (1,424 g/l $Na_2HPO_4$*$2H_2O$, 0,276 g/l, $NaH_2PO_4$*$2H_2O$, 8,19 g/l NaCl in water, pH 7,4). Cells were then seeded on the wells of a μ-Slide 8 Well ibidiTreat (Ibidi, Cat.: 80826). The number of cells per well was adjusted to reach about 50% confluency after adhesion of the cells. Cells were incubated over night with 200 µl HeLa cell culture medium per well.

*B. Fixation of cells*

After aspiration of cell culture medium and two washing steps with 200 µl 37°C warm PBS per well, cells were fixed with 200 µl precooled methanol (-20°C, Roth, Cat.: 0082.1) for 10 min at -20°C.

*C. Counterstaining with Sudan Black*

Methanol was aspirated and 150 µl of 0.2% Sudan Black-solution diluted in 70% ethanol were added to each well. Wells were incubated for 5 min in the dark at room temperature. After incubation cells were washed three times with 400 µl 70% ethanol per well to eliminate the excess of Sudan Black-solution.

*D. Hybridization of analyte/target-specific probes*

Before hybridization, cells were equilibrated with 200 µl sm-wash-buffer. The sm-wash-buffer comprises 30 mM $Na_3$Citrate, 300 mM NaCl, pH7, 10% formamide (Roth, Cat.:P040.1) and 5mM Ribonucleoside Vanadyl Complex (NEB, Cat.: S1402S). For each target-specific probe set 1 µl of a 100 µM oligonucleotide stock solution was added to the mixture. The oligonucleotide stock solution comprises equimolar amounts of all target specific oligonucleotides of the corresponding target specific probe set. The total volume of the mixture was adjusted to 100 µl with water and mixed with 100 µl of a 2x concentrated hybridization buffer solution. The 2x concentrated hybridization buffer comprises 120 mM $Na_3$Citrate, 1200 mM NaCl, pH7, 20% formamide and 20 mM Ribonucleoside Vanadyl Complex. The resulting 200 µl hybridization mixture was added to the corresponding well and incubated at 37°C for 2 h. Afterwards cells were washed three times with 200 µl per well for 10 min with target probe wash buffer at 37°C. The target probe wash buffer comprises 30 mM $Na_3$Citrate, 300 mM NaCl, pH7, 20% formamide and 5 mM Ribonucleoside Vanadyl Complex.

*E. Hybridization of decoding oligonucleotides*

Before hybridization, cells were equilibrated with 200 µl sm-wash-buffer. For each decoding oligonucleotide 1,5 µl of a 5 µM stock solution were added to the mixture. The total volume of the mixture was adjusted to 75 µl with water and mixed with 75 µl of a 2x concentrated hybridization buffer solution. The resulting 150 µl decoding oligonucleotide hybridization mixture was added to the corresponding well and incubated at room temperature for 45 min. Afterwards cells were washed three times with 200 µl per well for 2 min with sm-wash-buffer at room temperature.

*F. Hybridization of signal oligonucleotides*

Before hybridization, cells were equilibrated with 200 µl sm-wash-buffer. The signal oligonucleotide hybridization mixture was the same for all rounds of experiments 1 to 4 and comprised 0,3 µM of each signal oligonucleotide (see table A3) in 1x concentrated hybridization buffer solution. In each round 150 µl of this solution were added per well and incubated at room temperature for 45 min. The procedure was the same for experiments 5 to 8 with the exception that the final concentration of each signal oligonucleotide was 0,15 µM. Afterwards cells were washed three times with 200 µl per well for 2 min with sm-wash-buffer at room temperature.

*G. Fluorescence and white light imaging*

Cells were washed once with 200 µl of imaging buffer per well at room temperature. In experiments without Trolox (see table 7, last column) imaging buffer comprises 30 mM $Na_3$Citrate, 300 mM NaCl, pH7 and 5mM Ribonucleoside Vanadyl Complex. In experiments with Trolox, imaging buffer additionally contains 10 % VectaCell Trolox Antifade Reagent (Vector laboratories, Cat.: CB-1000), resulting in a final Trolox concentration of 10 mM.

A Zeiss Axiovert 200M microscope with a 63x immersion oil objective (Zeiss, apochromat) with numerical aperture of 1.4, a pco.edge 4.2 CMOS camera (PCO AG) and an LED-light source (Zeiss, colibri 7) was used for imaging of the regions. Filter sets and LED-wavelengths were adjusted to the different optima of the fluorophores used. Illumination times per image were 1000 ms for Alexa Fluor 546 and Atto 594 and 400 ms for Alexa Fluor 488.

In each experiment, three regions were randomly chosen for imaging. For each region, a z-stack of 32 images was detected with a z-step size of 350 nm. Additionally, one white light image was taken from the regions. In experiments with more than one detection cycle, the regions of the first detection round were found back and imaged in every subsequent round.

*H. Selective denaturation*

For selective denaturation, every well was incubated with 200 μl of sm-wash-buffer at 42°C for 6 min. This procedure was repeated six times.

Steps (E) to (H) were repeated 5 times in experiments 1 to 4. Step (H) was omitted for the 5th detection cycle.

*I. Analysis*

Based on custom ImageJ-plugins a semi-automated analysis of the raw data was performed to distinguish the specific fluorescent signals from the background. The resulting 3D-point clouds of all three fluorescent channels were combined in silico with a custom VBA script. The resulting combined 3D-point clouds of the 5 detection cycles were aligned to each other on the basis of a VBA script. The resulting alignments revealed the code words for each unique signal detected. Successfully decoded signals were used for quantitative and spatial analysis of the experiments based on custom VBA-scripts and ImageJ-plugins.

Results

*1. Absolute numbers of decoded signals*

The absolute numbers of successfully decoded signals for all transcripts are listed for each region of each experiment in the following Table 4. In summary, the sum of correct codes depicts the total number of decoded signals that were assigned to transcripts detectable in the corresponding experiment, while the sum of incorrect codes it the total number of decoded signals not detectable in the corresponding experiment. The total number of signals comprises successfully decoded as well as unsuccessfully decoded signals.

Table 4: Absolute numbers of decoded signals

| | Experiment 1: region | | | Experiment 2: region: | | | Experiment 3: region: | | | Experiment 4: region: | | |
|---|---|---|---|---|---|---|---|---|---|---|---|---|
| **transcript name:** | **1** | **2** | **3** | **1** | **2** | **3** | **1** | **2** | **3** | **1** | **2** | **3** |
| **Group 1** | | | | | | | | | | | | |
| **DDX5-201** | 1214 | 1136 | 927 | 1144 | 1509 | 1176 | 2964 | 2034 | 2141 | 8 | 2 | 2 |
| **RAD17-208** | 40 | 126 | 50 | 26 | 55 | 62 | 22 | 30 | 33 | 7 | 9 | 10 |
| **SPOCK1-202** | 581 | 655 | 301 | 483 | 875 | 149 | 1349 | 621 | 539 | 2 | 10 | 8 |
| **FBXO32-203** | 153 | 175 | 68 | 113 | 106 | 78 | 301 | 160 | 269 | 5 | 13 | 9 |
| **THRAP3-203** | 1079 | 2180 | 1035 | 810 | 1179 | 1047 | 2318 | 1609 | 1609 | 6 | 8 | 16 |
| **GART-203** | 422 | 397 | 346 | 350 | 333 | 202 | 766 | 658 | 569 | 5 | 3 | 2 |
| **KAT2A-201** | 141 | 310 | 166 | 174 | 307 | 186 | 382 | 315 | 340 | 5 | 1 | 3 |
| **HPRT1-201** | 63 | 79 | 34 | 44 | 116 | 71 | 85 | 88 | 112 | 1 | 0 | 6 |
| **CCNA2-201** | 91 | 248 | 205 | 95 | 134 | 138 | 241 | 151 | 238 | 10 | 20 | 9 |
| **NKRF-201** | 162 | 318 | 153 | 101 | 99 | 135 | 313 | 254 | 209 | 7 | 5 | 12 |
| **Group 2** | | | | | | | | | | | | |
| **CCNE1-201-new** | 75 | 180 | 38 | 57 | 110 | 97 | 0 | 0 | 1 | 122 | 104 | 120 |
| **COG5-201** | 57 | 21 | 38 | 26 | 45 | 23 | 0 | 0 | 1 | 56 | 86 | 60 |
| **FBN1-201** | 202 | 1338 | 499 | 456 | 513 | 571 | 0 | 1 | 3 | 450 | 778 | 895 |
| **DYNC1H1-201** | 554 | 892 | 398 | 664 | 1026 | 666 | 4 | 3 | 7 | 823 | 1148 | 1248 |
| **CKAP5-202** | 43 | 23 | 77 | 51 | 98 | 74 | 1 | 2 | 1 | 94 | 190 | 212 |
| **KRAS-202** | 331 | 417 | 355 | 302 | 333 | 230 | 0 | 0 | 1 | 279 | 637 | 371 |
| **EGFR-207** | 527 | 252 | 372 | 293 | 519 | 322 | 0 | 1 | 0 | 411 | 719 | 818 |
| **TP53-205** | 116 | 324 | 194 | 138 | 198 | 169 | 0 | 1 | 4 | 182 | 280 | 181 |
| **NF1-204** | 381 | 676 | 320 | 347 | 522 | 416 | 3 | 2 | 0 | 507 | 642 | 659 |
| **NF2-204** | 434 | 638 | 361 | 336 | 468 | 401 | 0 | 0 | 2 | 508 | 523 | 636 |
| **Group 3** | | | | | | | | | | | | |
| **ACO2-201** | 456 | 759 | 444 | 333 | 367 | 345 | 0 | 0 | 0 | 556 | 681 | 681 |
| **AKT1-211** | 351 | 710 | 301 | 230 | 437 | 297 | 0 | 0 | 3 | 558 | 614 | 690 |

(continued)

| | | | | | | | | | | | | |
|---|---|---|---|---|---|---|---|---|---|---|---|---|
| **Group 3** | | | | | | | | | | | | |
| **LYPLAL1-202** | 65 | 62 | 51 | 33 | 58 | 51 | 7 | 6 | 8 | 28 | 34 | 42 |
| **PKD2-201** | 72 | 194 | 124 | 95 | 129 | 69 | 1 | 0 | 0 | 122 | 164 | 169 |
| **ENG-204** | 472 | 890 | 458 | 446 | 494 | 558 | 0 | 2 | 0 | 1145 | 799 | 1119 |
| **FANCE-201** | 24 | 82 | 61 | 56 | 67 | 56 | 0 | 0 | 2 | 119 | 131 | 153 |
| **MET-201** | 268 | 744 | 333 | 426 | 275 | 462 | 0 | 0 | 0 | 790 | 662 | 782 |
| **NOTCH2-201** | 344 | 823 | 404 | 172 | 256 | 208 | 4 | 0 | 3 | 402 | 779 | 772 |
| **SPOP-206** | 43 | 377 | 139 | 68 | 117 | 100 | 0 | 0 | 0 | 215 | 289 | 300 |
| **ABL1-202** | 224 | 72 | 218 | 107 | 122 | 153 | 4 | 1 | 1 | 302 | 393 | 480 |
| **Group 4** | | | | | | | | | | | | |
| **ATP11C-202** | 170 | 116 | 121 | 86 | 165 | 128 | 2 | 1 | 1 | 130 | 206 | 234 |
| **BCR-202** | 180 | 401 | 185 | 177 | 149 | 169 | 1 | 0 | 0 | 321 | 372 | 485 |
| **CAV1-205** | 728 | 777 | 644 | 328 | 653 | 567 | 2 | 0 | 5 | 613 | 997 | 852 |
| **CDK2-201** | 306 | 937 | 367 | 297 | 385 | 358 | 4 | 1 | 3 | 568 | 742 | 888 |
| **DCAF1-202** | 119 | 292 | 187 | 59 | 67 | 65 | 0 | 1 | 0 | 108 | 171 | 131 |
| **FHOD1-201** | 60 | 233 | 143 | 132 | 185 | 157 | 1 | 0 | 1 | 194 | 294 | 300 |
| **GMDS-202** | 67 | 124 | 49 | 56 | 80 | 63 | 7 | 2 | 3 | 59 | 73 | 114 |
| **IFNAR1-201** | 81 | 221 | 135 | 99 | 104 | 55 | 1 | 0 | 1 | 159 | 266 | 238 |
| **NSMF-203** | 448 | 583 | 386 | 293 | 453 | 331 | 8 | 4 | 11 | 525 | 608 | 616 |
| **POLA2-201** | 74 | 111 | 62 | 45 | 72 | 67 | 2 | 4 | 2 | 41 | 75 | 57 |
| **Group 5** | | | | | | | | | | | | |
| **BRCA1-210new** | 230 | 704 | 248 | 324 | 439 | 374 | 2 | 1 | 0 | 2 | 3 | 3 |
| **JAK1-201new** | 157 | 554 | 223 | 185 | 259 | 263 | 0 | 1 | 0 | 5 | 4 | 2 |
| **STRAP-202** | 42 | 75 | 31 | 18 | 52 | 40 | 3 | 1 | 0 | 6 | 4 | 4 |
| **SERPINB5-201** | 324 | 598 | 343 | 286 | 344 | 364 | 6 | 13 | 9 | 0 | 1 | 2 |
| **SETX-201** | 212 | 540 | 254 | 291 | 439 | 336 | 2 | 2 | 2 | 12 | 9 | 5 |
| **WDFY1-201** | 43 | 516 | 218 | 176 | 206 | 147 | 0 | 0 | 0 | 4 | 2 | 8 |

(continued)

| Group 5 | | | | | | | | | | | | |
|---|---|---|---|---|---|---|---|---|---|---|---|---|
| **TACC1-201** | 69 | 373 | 131 | 80 | 156 | 118 | 1 | 0 | 0 | 21 | 29 | 32 |
| **KIF2A-203** | 442 | 879 | 445 | 298 | 519 | 430 | 5 | 1 | 2 | 6 | 11 | 9 |
| **CDT1-201** | 31 | 27 | 33 | 19 | 61 | 28 | 15 | 8 | 9 | 0 | 8 | 1 |
| **CENPE-202** | 192 | 246 | 215 | 94 | 262 | 225 | 0 | 1 | 0 | 8 | 9 | 9 |
| **Summary** | | | | | | | | | | | | |
| **sum of correct codes:** | 1296 0 | 2340 5 | 1289 0 | 1131 9 | 1591 7 | 1279 7 | 8741 | 5920 | 6059 | 1038 7 | 1345 7 | 1430 3 |
| **sum of incorrect codes:** | 0 | 0 | 0 | 0 | 0 | 0 | 86 | 60 | 86 | 120 | 151 | 152 |
| **total number of signals:** | 4295 9 | 7215 7 | 3218 5 | 3203 7 | 5879 3 | 3547 0 | 1354 9 | 9182 | 1010 9 | 2670 1 | 3296 6 | 3545 1 |
| **% successfully decoded:** | 30,2 | 32,4 | 40,0 | 35,3 | 27,1 | 36,1 | 64,5 | 64,5 | 59,9 | 38,9 | 40,8 | 40,3 |

Table 4 shows a very low number of incorrectly decoded signals compared to the number of correctly decoded signals. The absolute values for decoded signals of a certain transcript are very similar between different regions of one experiment. The fraction of the total number of signals that can be successfully decoded is between 27.1 % and 64.5 %. This fraction depends on the number of transcripts and/or the total number of signals present in the respective region/experiment.

*Conclusion*

The method according to the disclosure produces a low amount of incorrectly assigned code words and can therefore be considered specific. The fraction of successfully decodable signals is very high, even with very high numbers of signals per region and very high numbers of transcripts detected in parallel. The high fraction of assignable signals and the high specificity make the method practically useful.

*Comparison of relative transcript abundancies between different experiments*

As shown in Figure 8 for both comparisons (A and B) the overlap of detected transcripts between the experiments is used for the analysis. Each bar represents the mean abundance of all three regions of an experiment. The standard deviation between these regions is also indicated.

*Correlation of relative transcript abundancies between different experiments*

As can be seen in Figure 9 the mean relative abundances of transcripts from experiment 1 are correlated to the abundances of the overlapping transcripts of experiment 3, 4 and 2. The correlation coefficient as well as the formula for the linear regression are indicated for each correlation.

Figure 8 shows low standard deviations, indicating low variations of relative abundances between different regions of one experiment. The differences of relative abundances between transcripts from different experiments are also very low. This is the case for the comparison of transcripts from group 1 (Fig. 8A), that were detected in experiments 1, 2 and 3. It is also the case for the comparison of the transcripts from groups 2, 3 and 4 that were overlapping between experiments 1, 2 and 4. The very high correlation of these abundances can also be seen in Figure 9. The abundances of transcripts from experiment 1 correlate very well with the abundances of the other multi round experiments. The correlation factors are between 0.88 and 0.91, while the slope of the linear regressions is between 0.97 and 1.05.

*Conclusion*

The relative abundancies of transcripts correlate very well between different regions of one experiment but also between different experiments. This can be clearly seen by the comparisons of Figures 3 and 4. The main difference between the experiments is the number of different targets and hence the total number of signals detected. Therefore, the number of transcripts detected as well as the number and density of signals does not interfere with the ability of the method to accurately quantify the number of transcripts. The very good correlations further support the specificity and stability of the method, even with very high numbers of signals.

*Comparison of intercellular distribution of signals*

In Figure 10 the maximum projections of image stacks are shown. A: region 1 of experiment 7 (single round, single transcript experiment detecting SPOCK1), B: 2D-projection of all selected signals from experiment 1, region 1 assigned to SPOCK1, C: region 1 of experiment 8 (single round, single transcript experiment detecting THRAP3), D: 2D-projection of all selected signals from experiment 1, region 1 assigned to THRAP3.

*Comparison of intracellular distribution of signals*

In Figure 11 the maximum projections of image stacks are shown. Magnified sub regions of the corresponding regions are shown. A: region 1 of experiment 8 (single round, single transcript experiment detecting THRAP3), B: 2D-projection of selected signals from experiment 1, region 1 assigned to THRAP3, C: region 1 of experiment 5 (single round, single transcript experiment detecting DDX5), D: 2D-projection of all selected signals from experiment 1, region 1 assigned to DDX5.

Figure 10 shows huge differences of intercellular distributions between different transcripts. SPOCK1 seems to be highly abundant in some cells but nearly absent in other cells (Figure 10 A). THRAP3 shows a more uniform distribution over all cells of a region (Figure 10 C). These spatial distribution patterns can also clearly be observed with the point clouds assigned to the corresponding transcripts from experiment 1 (Figure 10 B and D).

Figure 11 shows huge differences of intracellular distributions between different transcripts. THRAP3 can be mainly observed in the periphery (cytoplasm) of the cells (Figure 11 A), while DDX5 shows a higher abundance in the center (nucleus) of the cells (Figure 11 C). These intracellular distributions can also be observed with the point clouds of experiment 1 assigned to THRAP3 and DDX5 (Figure 11 B and D).

*Conclusion*

Next to the reliability of quantification, the point clouds of multi round experiments also show the same intracellular and intercellular distribution patterns of transcripts. This is clearly proven by the direct comparison of the assigned point clouds with signals from single round experiments detecting only one characteristic mRNA-species.

*Distribution pattern of different cell cycle dependent transcripts*

All images of Figure 12 show region 1 of experiment 1. In each image, a point cloud is shown, that is assigned to a certain transcript, A: CCNA2, B: CENPE, C: CCNE1, D: all transcripts. Figure 12 shows the transcripts of three different cell cycle dependent proteins. CENPE (Figure 12 B) is also known as Centromere protein E and accumulates during G2 phase. It is proposed to be responsible for spindle elongation and for chromosome movement. It is not present during interphase. CCNA2 (Figure 12 A) is also known as Cyclin A2. It regulates the cell cycle progression by interacting with CDK1 during transition from G2 to M-phase. Interestingly there is an obvious colocalization of both mRNA-species. They are mainly present in the three central cells of region 1. CCNE1 (Figure 12 C) is also known as Cyclin E1. This cyclin interacts with CDK2 and is responsible for the transition from G1 to S-phase. Figure 12 shows clearly, that the transcripts of this gene are not present in the three central cells, but quite equally distributed over the other cells. It therefore shows an anti-localization to the other two transcripts. The data for the corresponding point-clouds are derived from a point cloud with a very high number of points and a very high point density (Figure 12 D gives an impression).

*Conclusion*

The three decoded point clouds of cell cycle dependent proteins shown in Figure 12, show distribution patterns that can be explained by their corresponding function. These data strongly suggest, that our method reliably produces biological relevant data, even with a low number of signals per cell (Figure 12 C) and with very high signal densities (Figure 12 D).

<u>Sequence listing</u>

In the accompanying sequence listing SEQ ID Nos. 1-1247 refer to nucleotide sequences of exemplary target-specific oligonucleotides. The oligonucleotides listed consist of a target specific binding site (5'- end) a spacer/linker sequence (gtaac or tagac) and the unique identifier sequence, which is the same for all oligonucleotides of one probe set.

In the accompanying sequence listing SEQ ID Nos. 1248-1397 refer to nucleotide sequences of exemplary decoding oligonucleotides.

In the accompanying sequence listing SEQ ID Nos. 1398-1400 refer to the nucleotide sequences of exemplary signal oligonucleotides. For each signal oligonucleotide the corresponding fluorophore is present twice. One fluorophore is covalently linked to the 5'-end and one fluorophore is covalently linked to the 3'-end. SEQ ID No. 1398 comprises at its 5' terminus "5Alex488N", and at its 3' terminus "3AlexF488N". SEQ ID No. 1399 comprises at its 5' terminus "5Alex546", and at its 3' terminus 3Alex546N. SEQ ID No. 1400 comprises at its 5' terminus and at its 3' terminus "Atto594".

## Claims

**1.** A method for detecting an analyte in a pathogen-comprising sample comprising:

i) Inactivation of the pathogen within the sample without isolation of RNA and/or DNA from the pathogen or the sample;
ii) Detecting the analyte by spatial transcriptomics.
wherein the pathogen-comprising sample is selected from the group consisting of a solid, a fluidic sample, a smear and a surface comprising pathogenic material,
wherein the pathogen-comprising sample is a biological sample, preferably comprising biological tissue, further preferably comprising biological cells and/or extracts and/or part of cells containing a pathogen, and

wherein the spatial transcriptomics detection of step ii) comprises a multiplex method for detecting different analytes in the pathogen-comprising sample by sequential signal-encoding of said analytes, comprising the steps of:

**(A)** contacting the sample with at least twenty (20) different sets of analyte-specific probes for encoding of at least 20 different analytes, each set of analyte-specific probes interacting with a different analyte, wherein if the analyte is a nucleic acid each set of analyte-specific probes comprises at least five (5) analyte-specific probes which specifically interact with different substructures of the same analyte, each analyte-specific probe comprising

(aa) a binding element (S) that specifically interacts with one of the different analytes to be encoded, and
(bb) an identifier element (T) comprising a nucleotide sequence which is unique to the analyte to be encoded (unique identifier sequence),
wherein the analyte-specific probes of a particular set of analyte-specific probes differ from the analyte-specific probes of another set of analyte-specific probes in the nucleotide sequence of the identifier element (T),
wherein the analyte-specific probes in each set of analyte-specific probes binds to the same analyte and comprises the same nucleotide sequence of the identifier element (T) which is unique to said analyte; and

**(B)** contacting the sample with at least one set of decoding oligonucleotides per analyte, wherein in each set of decoding oligonucleotides for an individual analyte each decoding oligonucleotide comprises:

(aa) an identifier connector element (t) comprising a nucleotide sequence which is essentially complementary to at least a section of the unique identifier sequence of the identifier element (T) of the corresponding analyte-specific probe set, and
(bb) a translator element (c) comprising a nucleotide sequence allowing a specific hybridization of a signal oligonucleotide;

wherein the decoding oligonucleotides of a set for an individual analyte differ from the decoding oligonucleotides of another set for a different analyte in the first connect element (t); and
**(C)** contacting the sample with at least a set of signal oligonucleotides, each signal oligonucleotide comprising:

(aa) a translator connector element (C) comprising a nucleotide sequence which is essentially complementary to at least a section of the nucleotide sequence of a translator element (c) comprised in a decoding oligonucleotide, and
(bb) a signal element.

**(D)** Detecting the signal caused by the signal element;
**(E)** Selectively removing the decoding oligonucleotides and signal oligonucleotides from the sample, thereby essentially maintaining the specific binding of the analyte-specific probes to the analytes to be encoded;
**(F)** Performing at least three (3) further cycles comprising steps B) to E) to generate an encoding scheme with a code word per analyte, wherein in particular the last cycle may stop with step (D);
wherein the code words per analyte in the performed cycles comprise additionally at least one element corresponding to no detected signal (code word zero (0)).

**2.** The method according to claim 1, wherein the pathogen-comprising sample comprise eukaryotic, archaea, prokaryotic organisms, and/or viruses.

**3.** The method according to of any one of claims 1 to 2, wherein the pathogen-comprising sample comprises formalin-fixed, paraffin-embedded tissues containing a pathogen and/or wherein the pathogen-comprising sample is frozen or alcoholic stabilized.

**4.** The method according to of any of the previous claims, wherein the pathogen is inactivated by a physical, chemical, biochemical and/or biological treatment.

**5.** The method according to claim 4, wherein the physical inactivation treatment is selected from the group consisting of temperature change, electromagnetic waves and light including visible and invisible light like UV irradiation, wherein the treatment with a temperature change is selected from a treatment at a temperature higher than 30°C, 40°C, 50°C, 60°C, 70°C, 80°C, 90°C, 100°C or 110°C,

**6.** The method according to claim 4, wherein the treatment with electromagnetic waves is selected from a treatment with X-ray, radioactivity, UV-light, blue light red light and/or infrared light, or combinations thereof.

**7.** The method according to claim 4, wherein the pathogen is inactivated by a chemical inactivation treatment, wherein the chemical inactivation treatment is selected from the group consisting of pH change, salt treatment, treatment with a fraction of a polar or nonpolar solvent, treatment with an oxidative or reductive reagent, treatment with a reagent that perform covalent or non-covalent linkage to the pathogen and treatment with a degenerative reagent that cleaves at least parts of the pathogen, or combinations thereof.

**8.** The method according to claim 4, wherein the biochemical inactivation treatment is a treatment with enzymes and/or biomolecules.

**9.** The method according to any one of claims 1 to 8, wherein all steps are automated, in particular wherein steps B) to F) are automated, in particular by using a robotic system.

**10.** The method according to any one of claims 1 to 9, wherein the code words obtained for the individual analytes in the performed cycles comprise the detected signals and additionally at least one element corresponding to no detected signal.

**11.** The method according to any one of claims 1 to 10, wherein no signal is detected for at least one analyte within at least one cycle.

**12.** The method according to of any one of claims 1 to 11, wherein for at least for one individual analyte a position of the code word is zero (0).

**13.** The method according to of any one of claims 1 to 12, wherein the code word zero (0) is generated by using no decoding oligonucleotides having an identifier connector element (t) comprising a nucleotide sequence which is essentially complementary to at least a section of the unique identifier sequence of the identifier element (T) of a corresponding analyte-specific probe for an individual analyte.

**14.** The method according to of any one of claims 1 to 13, wherein if at least for one individual analyte a position of the code word is zero (0) in this cycle no corresponding decoding oligonucleotides having an identifier connector element (t) comprising a nucleotide sequence which is essentially complementary to at least a section of the unique identifier sequence of the identifier element (T) of a corresponding analyte-specific probe for an individual analyte are used.

**15.** The method according to any one of claims 1 to 14, wherein the sample is contacted with at least two different sets of decoding oligonucleotides per analyte,

wherein the decoding oligonucleotides comprised in these different sets comprise the same identifier connector element (t) comprising a nucleotide sequence which is essentially complementary to at least a section of the unique identifier sequence of the identifier element (T) of the corresponding analyte-specific probe set, and
wherein the decoding oligonucleotides of the different sets per analyte differ in the translator element (c) comprising a nucleotide sequence allowing a specific hybridization of a signal oligonucleotide; and
wherein at least a set of non-signal decoding oligonucleotides for binding to a particular identifier element (T) of analyte-specific probes, wherein the decoding oligonucleotides in the same set of non-signal decoding oligonucleotides interacting with the same different identifier element (T),
wherein each non-signal decoding oligonucleotide comprises an identifier connector element (t) comprising a nucleotide sequence which is essentially complementary to at least a section of a unique identifier sequence, and does not comprise a translator element (c) comprising a nucleotide sequence allowing a specific hybridization of a signal oligonucleotide.

## Patentansprüche

1. Verfahren zum Nachweisen eines Analyten in einer pathogenhaltigen Probe, umfassend:

   i) Inaktivierung des Pathogens in der Probe ohne Isolierung von RNA und/oder DNA aus dem Pathogen oder der Probe;
   ii) Nachweisen des Analyten durch räumliche Transkriptomik.
   wobei die pathogenhaltige Probe aus der Gruppe ausgewählt ist, bestehend aus einem Feststoff, einer flüssigen Probe, einem Abstrich und einer Oberfläche, die pathogenes Material umfasst,
   wobei die pathogenhaltige Probe eine biologische Probe ist, vorzugsweise umfassend biologisches Gewebe, ferner vorzugsweise umfassend biologische Zellen und/oder Extrakte und/oder Teile von Zellen, die ein Pathogen enthalten, und
   wobei der räumliche Transkriptomik-Nachweis von Schritt ii) ein Multiplex-Verfahren zum Nachweisen verschiedener Analyten in der pathogenhaltigen Probe durch sequentielle Signalcodierung der Analyten umfasst, umfassend die folgenden Schritte:

   **(A)** Inkontaktbringen der Probe mit mindestens zwanzig (20) verschiedenen Sätzen analytspezifischer Sonden zum Kodieren von mindestens 20 verschiedenen Analyten, wobei jeder Satz analytspezifischer Sonden mit einem anderen Analyten interagiert, wobei, wenn der Analyt eine Nukleinsäure ist, jeder Satz analytspezifischer Sonden mindestens fünf (5) analytspezifische Sonden umfasst, die spezifisch mit verschiedenen Unterstrukturen desselben Analyten interagieren, wobei jede analytspezifische Sonde Folgendes umfasst

   (aa) ein Bindungselement (S), das spezifisch mit einem der verschiedenen zu kodierenden Analyten interagiert, und
   (bb) ein Identifikatorelement (T), umfassend eine Nukleotidsequenz, die für den zu kodierenden Analyten einzigartig ist (eindeutige Identifikatorsequenz),
   wobei sich die analytspezifischen Sonden eines bestimmten Satzes analytspezifischer Sonden von den analytspezifischen Sonden eines anderen Satzes analytspezifischer Sonden in der Nukleotidsequenz des Identifikatorelements (T) unterscheiden,
   wobei die analytspezifischen Sonden in jedem Satz analytspezifischer Sonden an denselben Analyten binden und dieselbe Nukleotidsequenz des Identifikatorelements (T) umfassen, die für den Analyten eindeutig ist; und

   **(B)** Inkontaktbringen der Probe mit mindestens einem Satz dekodierenden Oligonukleotiden pro Analyt, wobei in jedem Satz von dekodierenden Oligonukleotiden für einen einzelnen Analyten jedes dekodierende Oligonukleotid Folgendes umfasst:

   (aa) ein Identifikatorverbindungselement (t), umfassend eine Nukleotidsequenz, die im Wesentlichen komplementär zu mindestens einem Abschnitt der eindeutigen Identifikatorsequenz des Identifikatorelements (T) des entsprechenden analytspezifischen Sondensatzes ist, und
   (bb) ein Translatorelement (c), umfassend eine Nukleotidsequenz, die eine spezifische Hybridisierung eines Signal-Oligonukleotids ermöglicht;

   wobei sich die dekodierenden Oligonukleotide eines Satzes für einen individuellen Analyten von den dekodierenden Oligonukleotiden eines anderen Satzes für einen anderen Analyten in dem ersten Verbindungselement (t) unterscheiden; und
   **(C)** Inkontaktbringen der Probe mit mindestens einem Satz von Signal-Oligonukleotiden, wobei jedes Signal-Oligonukleotid Folgendes umfasst:

   (aa) ein Translator-Verbindungselement (C), umfassend eine Nukleotidsequenz, die im Wesentlichen komplementär zu mindestens einem Abschnitt der Nukleotidsequenz eines Translatorelements (c) ist, das in einem dekodierenden Oligonukleotid umfasst ist, und
   (bb) ein Signalelement.

   **(D)** Nachweisen des Signals, das durch das Signalelement verursacht wird;
   **(E)** Selektives Entfernen der dekodierenden Oligonukleotide und Signal-Oligonukleotide aus der Probe, wodurch im Wesentlichen die spezifische Bindung der analytspezifischen Sonden an die zu kodierenden Analyten aufrechterhalten wird;
**(F)** Durchführen von mindestens drei (3) weiteren Zyklen, umfassend die Schritte B) bis E), um ein Kodierungsschema mit einem Codewort pro Analyt zu erzeugen, wobei insbesondere der letzte Zyklus mit Schritt (D) enden kann;
wobei die Codewörter pro Analyt in den durchgeführten Zyklen zusätzlich mindestens ein Element umfassen, das keinem detektierten Signal entspricht (Codewort Null (0)).

**2.** Verfahren nach Anspruch 1, wobei die pathogenhaltige Probe eukaryotische, archaeische, prokaryotische Organismen und/oder Viren umfasst.

**3.** Verfahren nach einem der Ansprüche 1 bis 2, wobei die pathogenhaltige Probe formalinfixierte, in Paraffin eingebettete Gewebe umfasst, die ein Pathogen enthalten, und/oder wobei die pathogenhaltige Probe gefroren oder alkoholisch stabilisiert ist.

**4.** Verfahren nach einem der vorstehenden Ansprüche, wobei das Pathogen durch eine physikalische, chemische, biochemische und/oder biologische Behandlung inaktiviert wird.

**5.** Verfahren nach Anspruch 4, wobei die physikalische Inaktivierungsbehandlung ausgewählt ist aus der Gruppe bestehend aus Temperaturänderung, elektromagnetischen Wellen und Licht, einschließlich sichtbarem und unsichtbarem Licht, wie UV-Bestrahlung, wobei die Behandlung mit einer Temperaturänderung ausgewählt ist aus einer Behandlung bei einer Temperatur von mehr als 30 °C, 40 °C, 50 °C, 60 °C, 70 °C, 80 °C, 90 °C, 100 °C oder 110 °C,

**6.** Verfahren nach Anspruch 4, wobei die Behandlung mit elektromagnetischen Wellen ausgewählt ist aus einer Behandlung mit Röntgenstrahlen, Radioaktivität, UV-Licht, blauem Licht, rotem Licht und/oder Infrarotlicht oder Kombinationen davon.

**7.** Verfahren nach Anspruch 4, wobei das Pathogen durch eine chemische Inaktivierungsbehandlung inaktiviert wird, wobei die chemische Inaktivierungsbehandlung ausgewählt ist aus der Gruppe bestehend aus pH-Änderung, Salzbehandlung, Behandlung mit einer Fraktion eines polaren oder nicht-polaren Lösungsmittels, Behandlung mit einem oxidativen oder reduktiven Reagenz, Behandlung mit einem Reagenz, das kovalente oder nicht-kovalente Bindung mit dem Pathogen durchführt, und Behandlung mit einem degenerativen Reagenz, das zumindest Teile des Pathogens spaltet, oder Kombinationen davon.

**8.** Verfahren nach Anspruch 4, wobei die biochemische Inaktivierungsbehandlung eine Behandlung mit Enzymen und/oder Biomolekülen ist.

**9.** Verfahren nach einem der Ansprüche 1 bis 8, wobei alle Schritte automatisiert sind, insbesondere wobei die Schritte B) bis F) automatisiert sind, insbesondere unter Verwendung eines Robotersystems.

**10.** Verfahren nach einem der Ansprüche 1 bis 9, wobei die für die einzelnen Analyten in den durchgeführten Zyklen erhaltenen Codewörter die nachgewiesenen Signale und zusätzlich mindestens ein Element umfassen, das keinem nachgewiesenen Signal entspricht.

**11.** Verfahren nach einem der Ansprüche 1 bis 10, wobei für mindestens einen Analyten innerhalb mindestens eines Zyklus kein Signal nachgewiesen wird.

**12.** Verfahren nach einem der Ansprüche 1 bis 11, wobei für mindestens einen einzelnen Analyten eine Position des Codeworts Null (0) ist.

**13.** Verfahren nach einem der Ansprüche 1 bis 12, wobei das Codewort Null (0) unter Verwendung von nicht dekodierenden Oligonukleotiden mit einem Identifikatorverbindungselement (t) umfassend eine Nukleotidsequenz erzeugt wird, die im Wesentlichen komplementär zu mindestens einem Abschnitt der eindeutigen Identifikatorsequenz des Identifikatorelements (T) einer entsprechenden analytspezifischen Sonde für einen einzelnen Analyten ist.

**14.** Verfahren nach einem der Ansprüche 1 bis 13, wobei, wenn mindestens für einen einzelnen Analyten eine Position des Codewortes in diesem Zyklus Null (0) ist, keine entsprechenden dekodierenden Oligonukleotide mit einem Identifikatorverbindungselement (t) umfassend eine Nukleotidsequenz verwendet werden, die im Wesentlichen komplementär zu mindestens einem Abschnitt der eindeutigen Identifikatorsequenz des Identifikatorelements (T) einer entsprechenden analytspezifischen Sonde für einen einzelnen Analyten ist.

**15.** Verfahren nach einem der Ansprüche 1 bis 14, wobei die Probe mit mindestens zwei verschiedenen Sätzen von dekodierenden Oligonukleotiden pro Analyt in Kontakt gebracht wird,

wobei die in diesen verschiedenen Sätzen umfassten dekodierenden Oligonukleotide das gleiche Identifikatorverbindungselement (t) umfassen, das eine Nukleotidsequenz umfasst, die im Wesentlichen komplementär zu mindestens einem Abschnitt der eindeutigen Identifikatorsequenz des Identifikatorelements (T) des entsprechenden analytspezifischen Sondensatzes ist, und
wobei sich die dekodierenden Oligonukleotide der verschiedenen Sätze pro Analyt darin unterscheiden, dass das Translatorelement (c) eine Nukleotidsequenz umfasst, die eine spezifische Hybridisierung eines Signal-Oligonukleotids ermöglicht; und
wobei mindestens ein Satz von nicht-signaldekodierenden Oligonukleotiden zum Binden an ein bestimmtes Identifikatorelement (T) von analytspezifischen Sonden, wobei die decodierenden Oligonukleotide in demselben Satz von nicht-signaldekodierenden Oligonukleotiden mit demselben unterschiedlichen Identifikatorelement (T) interagieren,
wobei jedes nicht-signaldecodierende Oligonukleotid ein Identifikatorverbindungselement (t) umfasst, das eine Nukleotidsequenz umfasst, die im Wesentlichen komplementär zu mindestens einem Abschnitt einer eindeutigen Identifikatorsequenz ist, und kein Translatorelement (c) umfasst, das eine Nukleotidsequenz umfasst, die eine spezifische Hybridisierung eines Signal-Oligonukleotids ermöglicht.

## Revendications

**1.** Procédé de détection d'un analyte dans un échantillon comprenant un agent pathogène comprenant :

i) l'inactivation de l'agent pathogène dans l'échantillon sans isolement de l'ARN et/ou de l'ADN de l'agent pathogène ou de l'échantillon ;
ii) la détection de l'analyte par transcriptomique spatiale.
dans lequel l'échantillon comprenant un agent pathogène est choisi dans le groupe constitué d'un solide, d'un échantillon fluidique, d'un frottis et d'une surface comprenant un matériau pathogène,
dans lequel l'échantillon comprenant un agent pathogène est un échantillon biologique, comprenant de préférence un tissu biologique, comprenant en outre de préférence des cellules biologiques et/ou des extraits et/ou une partie de cellules contenant un agent pathogène, et
dans lequel la détection transcriptomique spatiale de l'étape ii) comprend un procédé multiplex pour détecter différents analytes dans l'échantillon comprenant un agent pathogène par codage séquentiel des signaux desdits analytes, comprenant les étapes consistant à :

**(A)** mettre en contact l'échantillon avec au moins vingt (20) ensembles différents de sondes spécifiques à l'analyte pour coder au moins 20 analytes différents, chaque ensemble de sondes spécifiques à l'analyte interagissant avec un analyte différent, dans lequel si l'analyte est un acide nucléique, chaque ensemble de sondes spécifiques à l'analyte comprend au moins cinq (5) sondes spécifiques à l'analyte qui interagissent spécifiquement avec différentes sous-structures du même analyte, chaque sonde spécifique à l'analyte comprenant

(aa) un élément de liaison (S) qui interagit spécifiquement avec l'un des différents analytes à coder, et
(bb) un élément identificateur (T) comprenant une séquence nucléotidique unique à l'analyte à coder (séquence d'identificateur unique),
dans lequel les sondes spécifiques à l'analyte d'un ensemble particulier de sondes spécifiques à l'analyte diffèrent des sondes spécifiques à l'analyte d'un autre ensemble de sondes spécifiques à l'analyte dans la séquence nucléotidique de l'élément identificateur (T),
dans lequel les sondes spécifiques à l'analyte dans chaque ensemble de sondes spécifiques à l'analyte se lient au même analyte et comprennent la même séquence nucléotidique de l'élément identificateur (T) qui est unique audit analyte ; et

**(B)** mettre en contact l'échantillon avec au moins un ensemble d'oligonucléotides de décodage par analyte, dans lequel dans chaque ensemble d'oligonucléotides de décodage pour un analyte individuel, chaque oligonucléotide de décodage comprend :

(aa) un élément connecteur identificateur (t) comprenant une séquence nucléotidique qui est essentiellement complémentaire d'au moins une section de la séquence d'identificateur unique de l'élément identificateur (T) de l'ensemble de sondes spécifique à l'analyte correspondant, et
(bb) un élément traducteur (c) comprenant une séquence nucléotidique permettant une hybridation spécifique d'un signal d'oligonucléotide ;

dans lequel les oligonucléotides de décodage d'un ensemble pour un analyte individuel diffèrent des oligonucléotides de décodage d'un autre ensemble pour un analyte différent dans le premier élément de connexion (t) ; et
**(C)** mettre en contact l'échantillon avec au moins un ensemble de signaux d'oligonucléotides, chaque signal d'oligonucléotide comprenant :

(aa) un élément connecteur traducteur (C) comprenant une séquence nucléotidique qui est essentiellement complémentaire d'au moins une section de la séquence nucléotidique d'un élément traducteur (c) compris dans un oligonucléotide de décodage, et
(bb) un élément de signal.

**(D)** Détecter le signal causé par l'élément de signal ;
**(E)** retirer sélectivement les oligonucléotides de décodage et les signaux d'oligonucléotides de l'échantillon, maintenant ainsi essentiellement la liaison spécifique des sondes spécifiques à l'analyte aux analytes à coder ;
**(F)** effectuer au moins trois (3) autres cycles comprenant les étapes B) à E) pour générer un schéma de codage avec un mot de code par analyte, dans lequel en particulier le dernier cycle peut s'arrêter avec l'étape (D) ;
dans lequel les mots de code par analyte dans les cycles effectués comprennent en outre au moins un élément correspondant à aucun signal détecté (mot de code zéro (0)).

**2.** Procédé selon la revendication 1, dans lequel l'échantillon comprenant un agent pathogène comprend des organismes eucaryotes, archées, procaryotes et/ou des virus.

**3.** Procédé selon l'une quelconque des revendications 1 à 2, dans lequel l'échantillon comprenant un agent pathogène comprend des tissus fixés au formol et inclus en paraffine contenant un agent pathogène et/ou dans lequel l'échantillon comprenant un agent pathogène est congelé ou stabilisé à l'alcool.

**4.** Procédé selon l'une quelconque des revendications précédentes, dans lequel l'agent pathogène est inactivé par un traitement physique, chimique, biochimique et/ou biologique.

**5.** Procédé selon la revendication 4, dans lequel le traitement d'inactivation physique est choisi dans le groupe constitué par le changement de température, les ondes électromagnétiques et la lumière, y compris la lumière visible et invisible comme l'irradiation UV, dans lequel le traitement avec un changement de température est choisi parmi un traitement à une température supérieure à 30°C, 40°C, 50°C, 60°C, 70°C, 80°C, 90°C, 100°C ou 110°C,

**6.** Procédé selon la revendication 4, dans lequel le traitement par ondes électromagnétiques est choisi parmi un traitement par rayons X, radioactivité, lumière UV, lumière bleue, lumière rouge et/ou lumière infrarouge, ou des combinaisons de ceux-ci.

**7.** Procédé selon la revendication 4, dans lequel l'agent pathogène est inactivé par un traitement d'inactivation chimique, dans lequel le traitement d'inactivation chimique est choisi dans le groupe constitué par un changement de pH, un traitement au sel, un traitement avec une fraction d'un solvant polaire ou non polaire, un traitement avec un réactif oxydant ou réducteur, un traitement avec un réactif qui effectue une liaison covalente ou non covalente avec l'agent pathogène et un traitement avec un réactif dégénératif qui clive au moins des parties de l'agent pathogène, ou des combinaisons de ceux-ci.

**8.** Procédé selon la revendication 4, dans lequel le traitement d'inactivation biochimique est un traitement avec des enzymes et/ou des biomolécules.

**9.** Procédé selon l'une quelconque des revendications 1 à 8, dans lequel toutes les étapes sont automatisées, en particulier dans lequel les étapes B) à F) sont automatisées, en particulier en utilisant un système robotique.

**10.** Procédé selon l'une quelconque des revendications 1 à 9, dans lequel les mots de code obtenus pour les analytes individuels dans les cycles effectués comprennent les signaux détectés et en outre au moins un élément correspondant à aucun signal détecté.

**11.** Procédé selon l'une quelconque des revendications 1 à 10, dans lequel aucun signal n'est détecté pour au moins un analyte au cours d'au moins un cycle.

**12.** Procédé selon l'une quelconque des revendications 1 à 11, dans lequel, pour au moins un analyte individuel, une position du mot de code est zéro (0).

**13.** Procédé selon l'une quelconque des revendications 1 à 12, dans lequel le mot de code zéro (0) est généré en n'utilisant pas d'oligonucléotides de décodage ayant un élément connecteur identificateur (t) comprenant une séquence nucléotidique qui est essentiellement complémentaire d'au moins une section de la séquence d'identificateur unique de l'élément identificateur (T) d'une sonde spécifique à un analyte correspondant pour un analyte individuel.

**14.** Procédé selon l'une quelconque des revendications 1 à 13, dans lequel si au moins pour un analyte individuel une position du mot de code est zéro (0) dans ce cycle, aucun oligonucléotide de décodage correspondant ayant un élément connecteur identificateur (t) comprenant une séquence nucléotidique qui est essentiellement complémentaire d'au moins une section de la séquence d'identificateur unique de l'élément identificateur (T) d'une sonde spécifique à l'analyte correspondant pour un analyte individuel n'est utilisé.

**15.** Procédé selon l'une quelconque des revendications 1 à 14, dans lequel l'échantillon est mis en contact avec au moins deux ensembles différents d'oligonucléotides de décodage par analyte,

dans lequel les oligonucléotides de décodage compris dans ces différents ensembles comprennent le même élément connecteur identificateur (t) comprenant une séquence nucléotidique qui est essentiellement complémentaire d'au moins une section de la séquence d'identificateur unique de l'élément identificateur (T) de l'ensemble de sondes spécifique à l'analyte correspondant, et
dans lequel les oligonucléotides de décodage des différents ensembles par analyte diffèrent dans l'élément traducteur (c) comprenant une séquence nucléotidique permettant une hybridation spécifique d'un signal d'oligonucléotide ; et
dans lequel au moins un ensemble d'oligonucléotides de décodage de non-signal pour se lier à un élément identificateur particulier (T) de sondes spécifiques à l'analyte, dans lequel les oligonucléotides de décodage dans le même ensemble d'oligonucléotides de décodage de non-signal interagissent avec le même élément identificateur différent (T),
dans lequel chaque oligonucléotide de décodage de non-signal comprend un élément connecteur identificateur (t) comprenant une séquence nucléotidique qui est essentiellement complémentaire d'au moins une section d'une séquence d'identificateur unique, et ne comprend pas d'élément traducteur (c) comprenant une séquence nucléotidique permettant une hybridation spécifique d'un signal d'oligonucléotide.

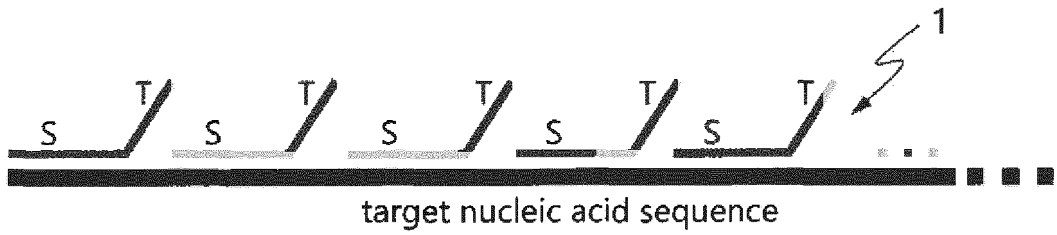


FIG. 1

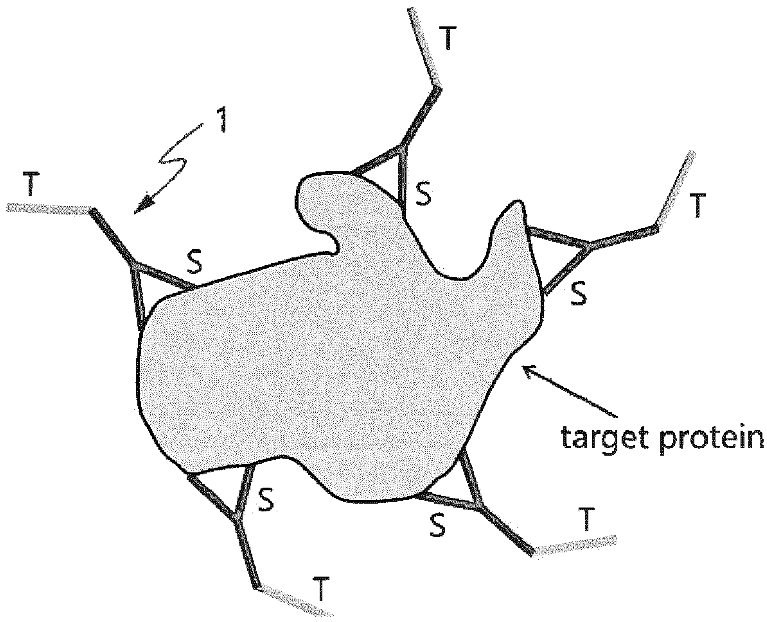


FIG. 2

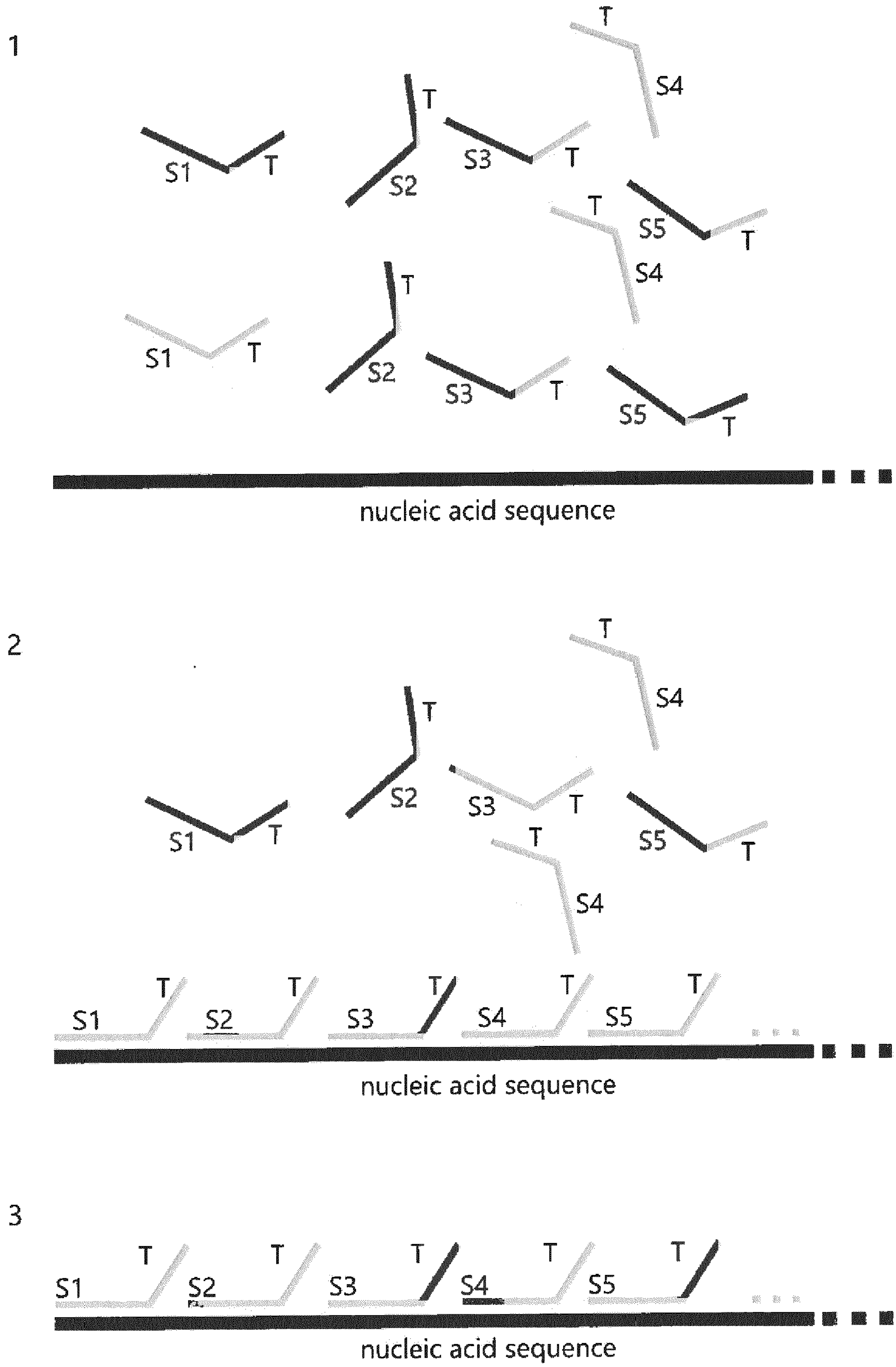
1
T
S4
T
S1
T
S2
S3
T
T
S5
T
S4
T
S1
T
S2
S3
T
S5
T
nucleic acid sequence
2
T
S4
T
S1
T
S2
S3
T
T
S5
T
S4
S1 T
S2 T
S3 T
S4 T
S5 T
nucleic acid sequence
3
S1 T
S2 T
S3 T
S4 T
S5 T
nucleic acid sequence

Fig. 3

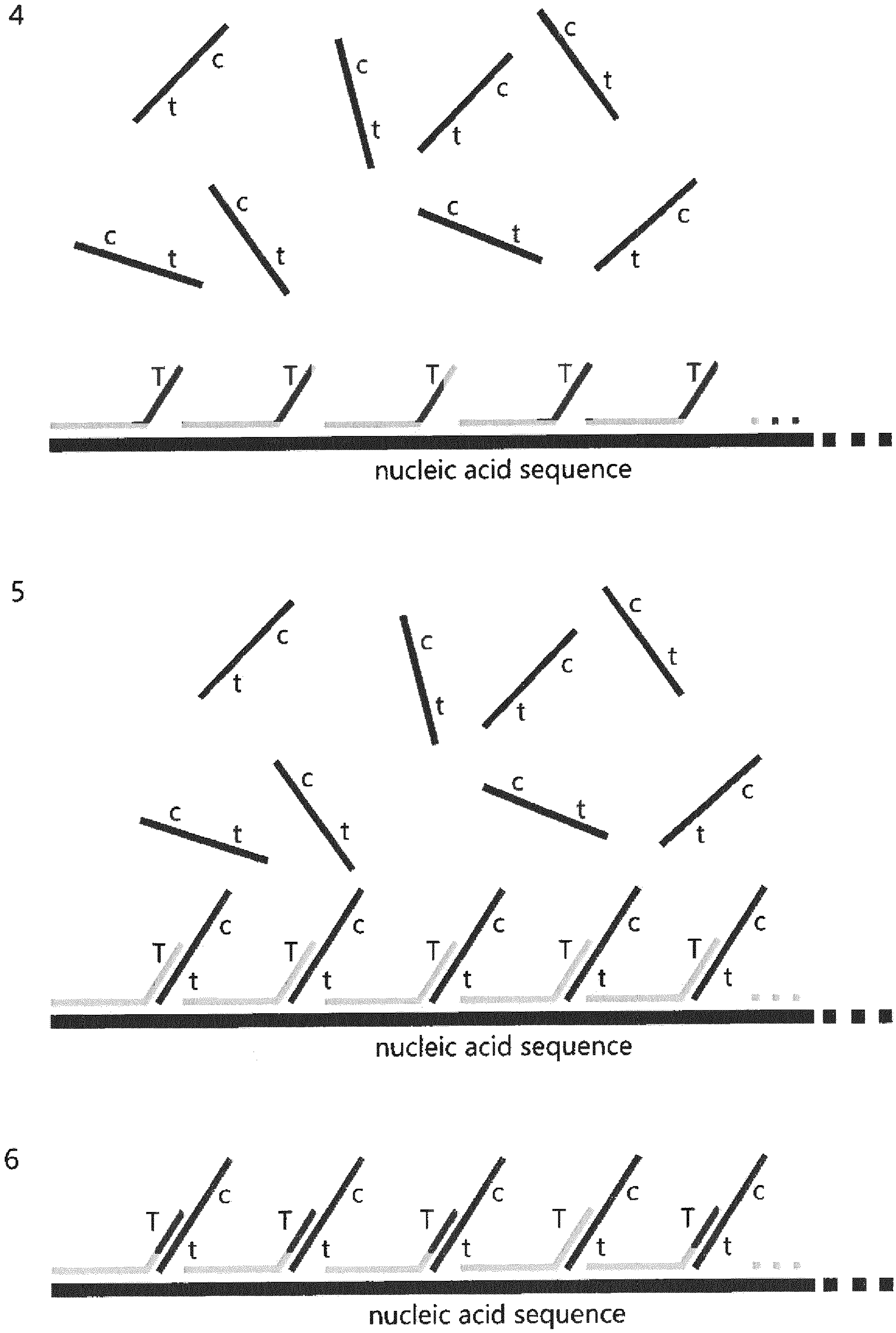


Fig. 3 continued

7

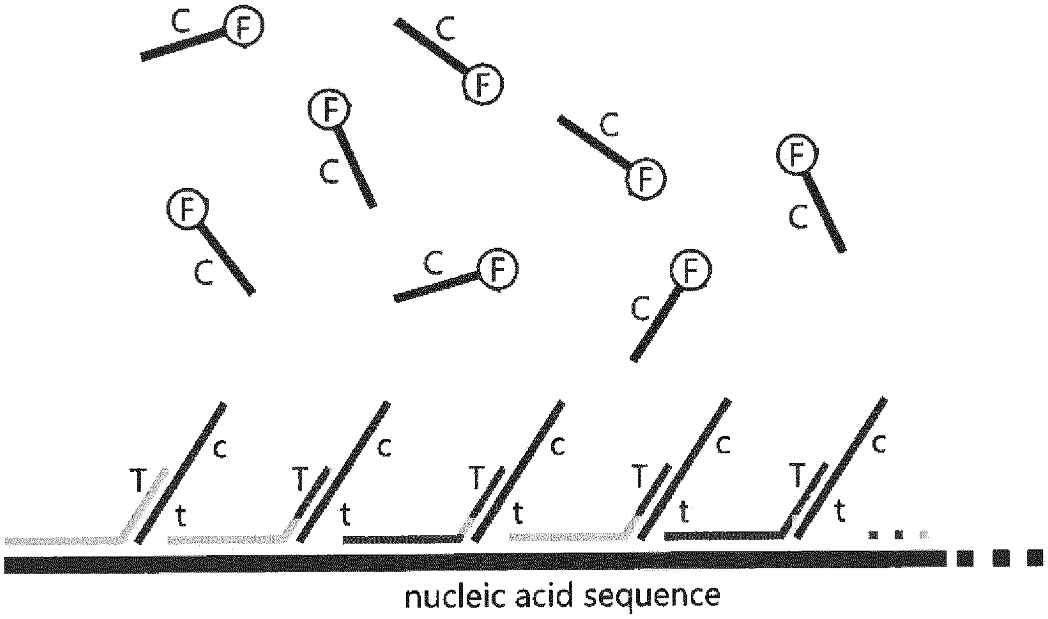


8

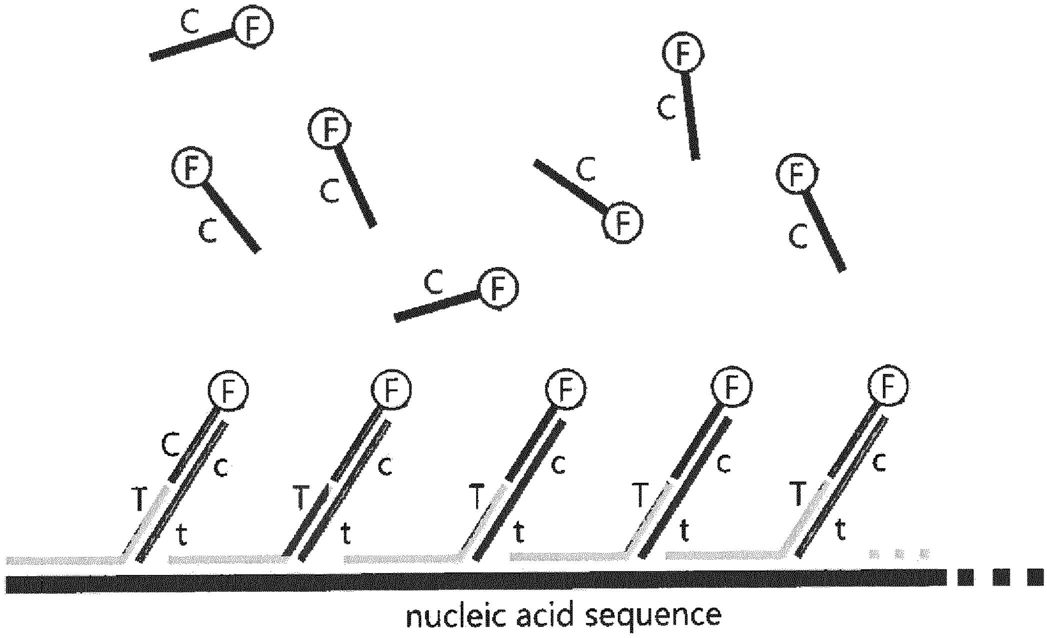


9

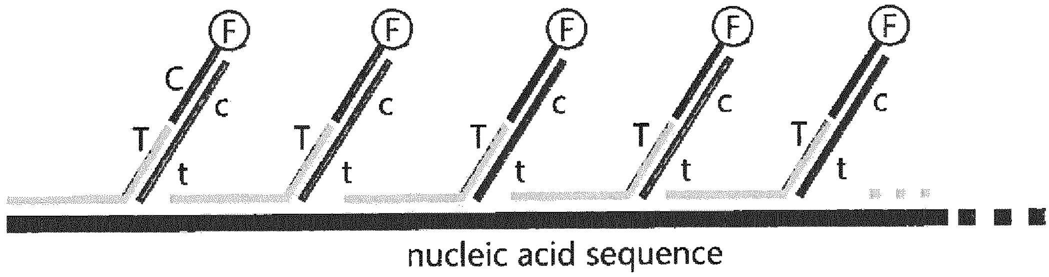


Fig. 3 continued

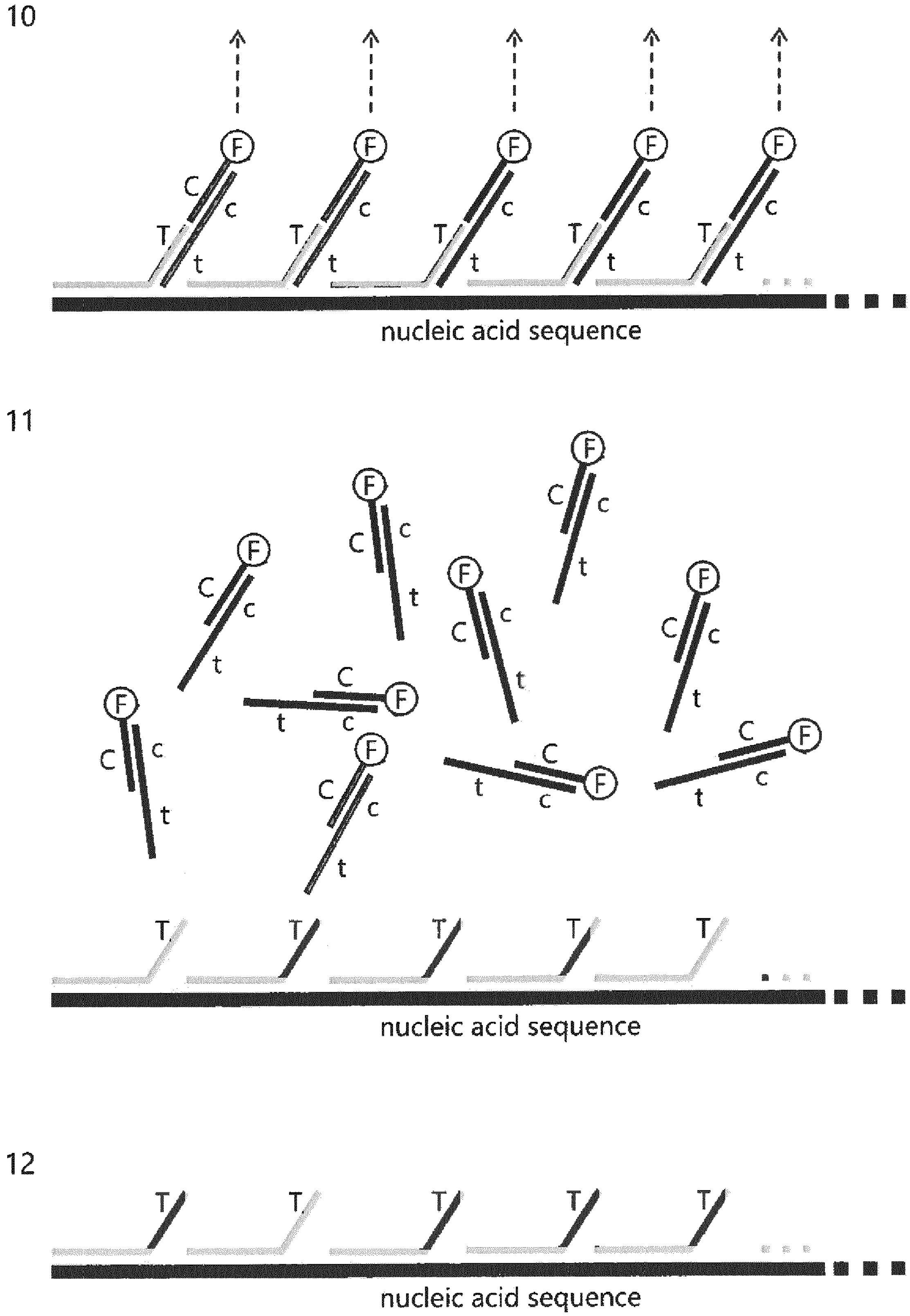


Fig. 3 continued

Opt. 1

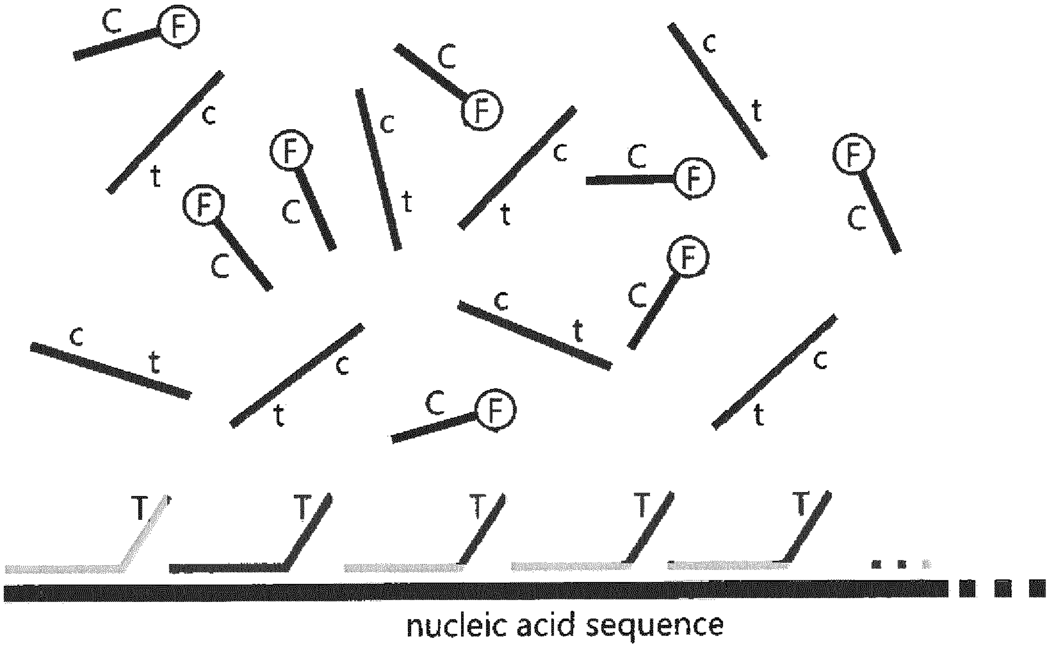


Opt. 2

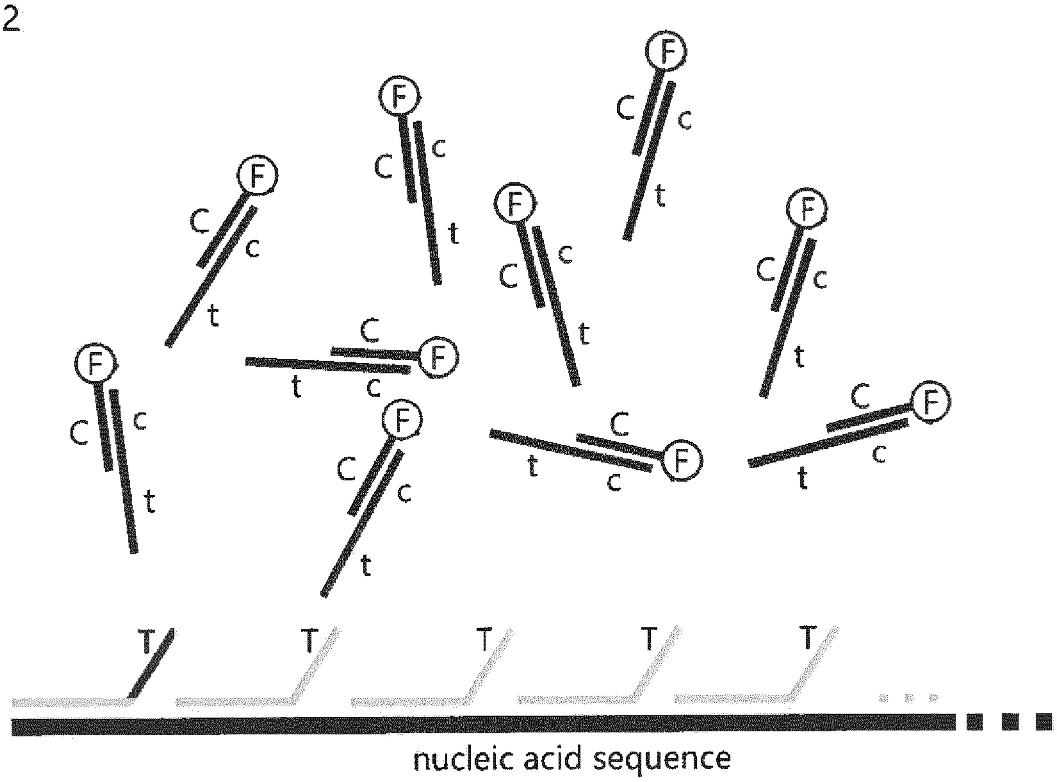


FIG. 4

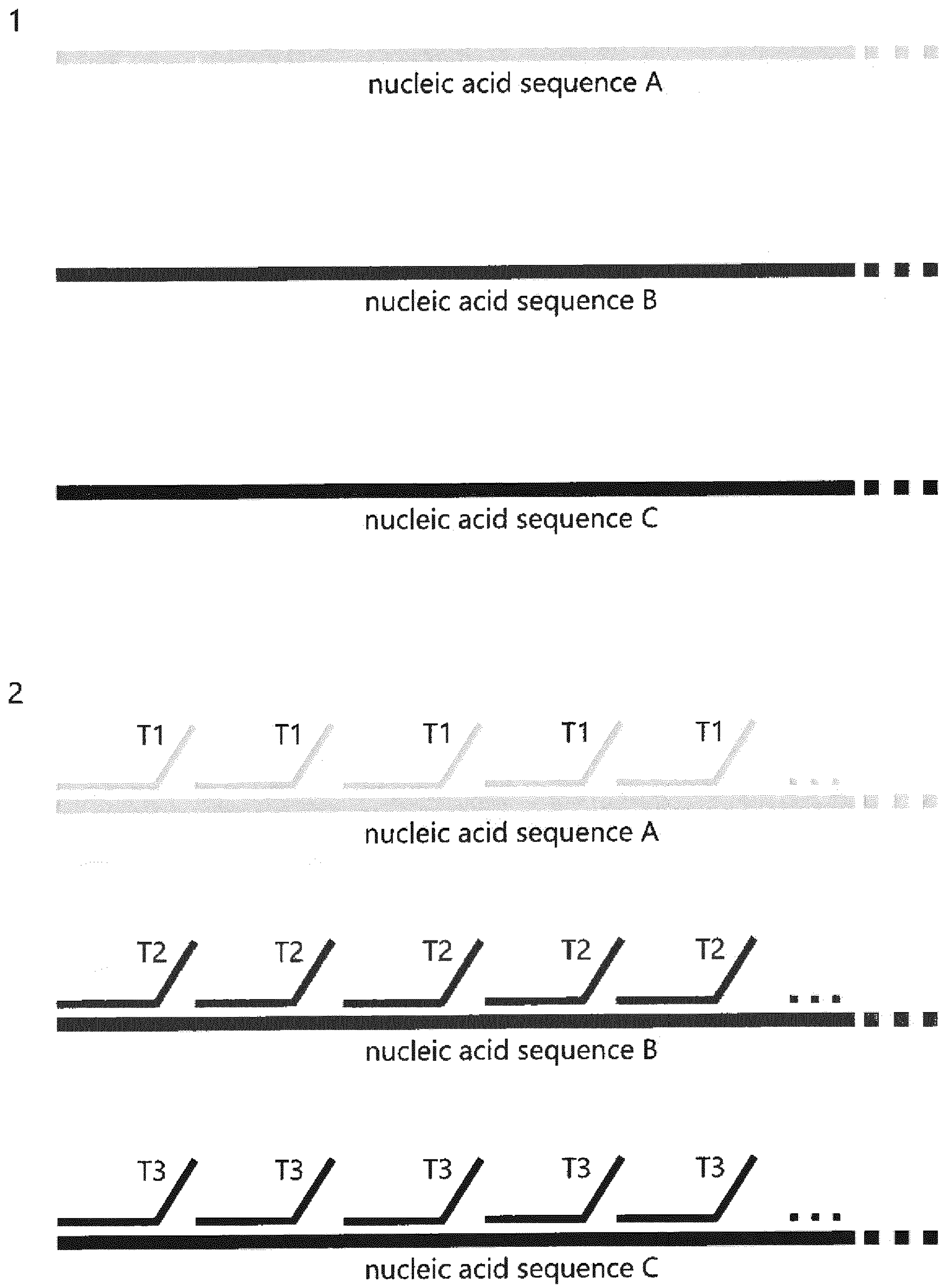
1
nucleic acid sequence A
nucleic acid sequence B
nucleic acid sequence C
2
T1
T1
T1
T1
T1
nucleic acid sequence A
T2
T2
T2
T2
T2
nucleic acid sequence B
T3
T3
T3
T3
T3
nucleic acid sequence C

FIG. 5

3

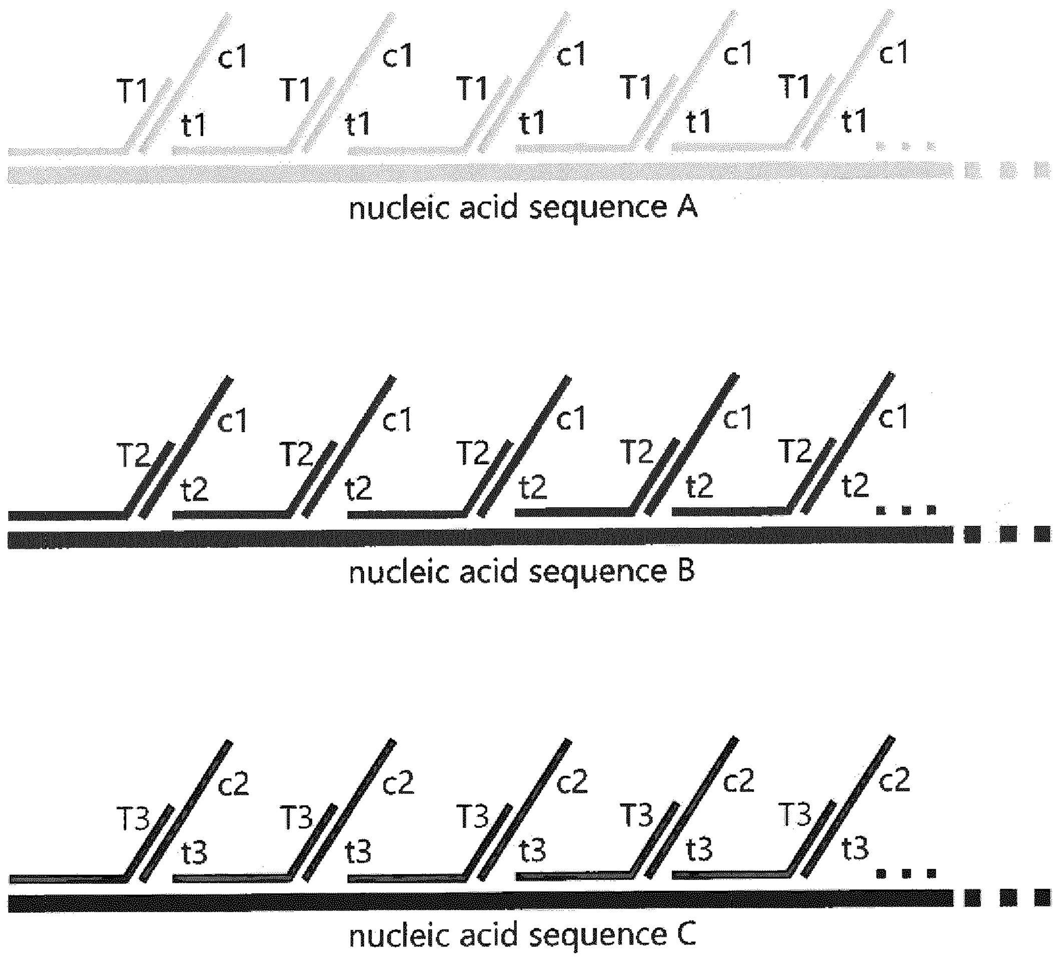


FIG. 5 continued

4

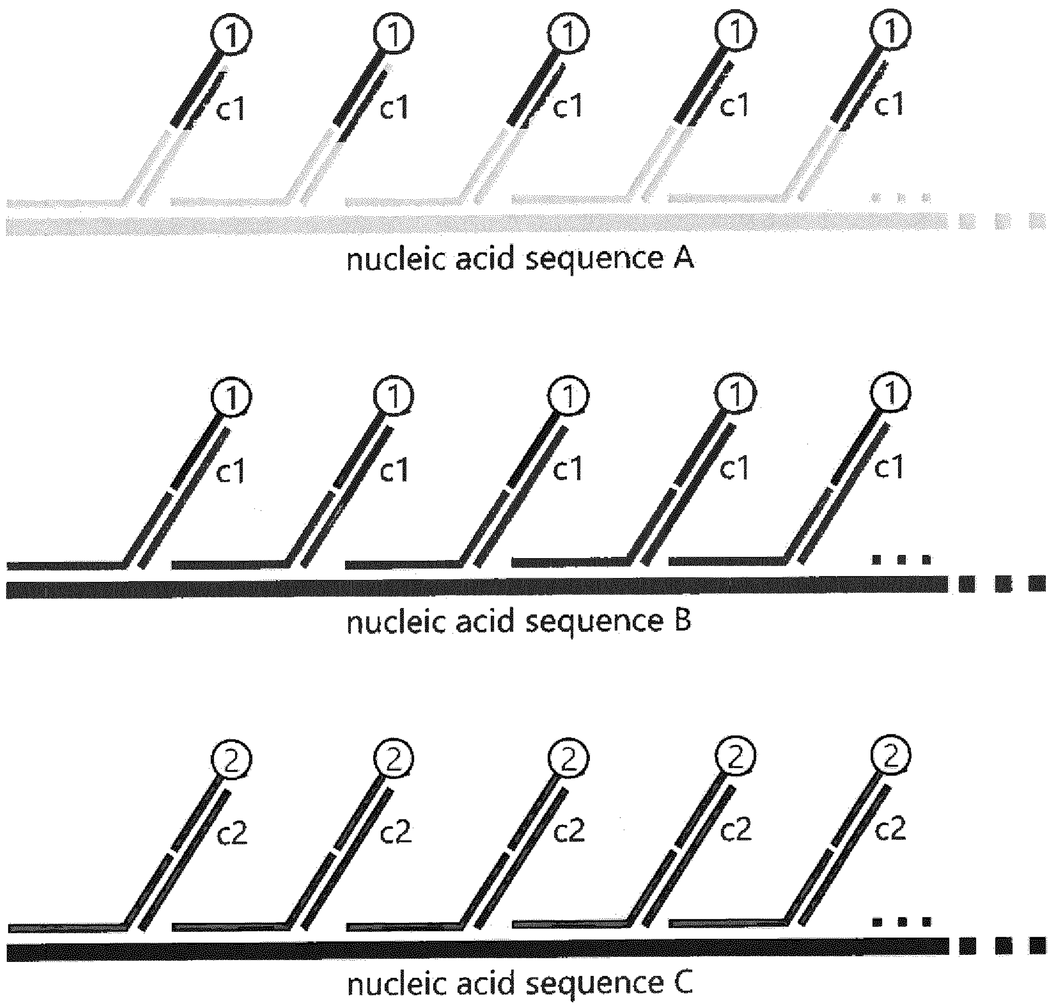


FIG. 5 continued

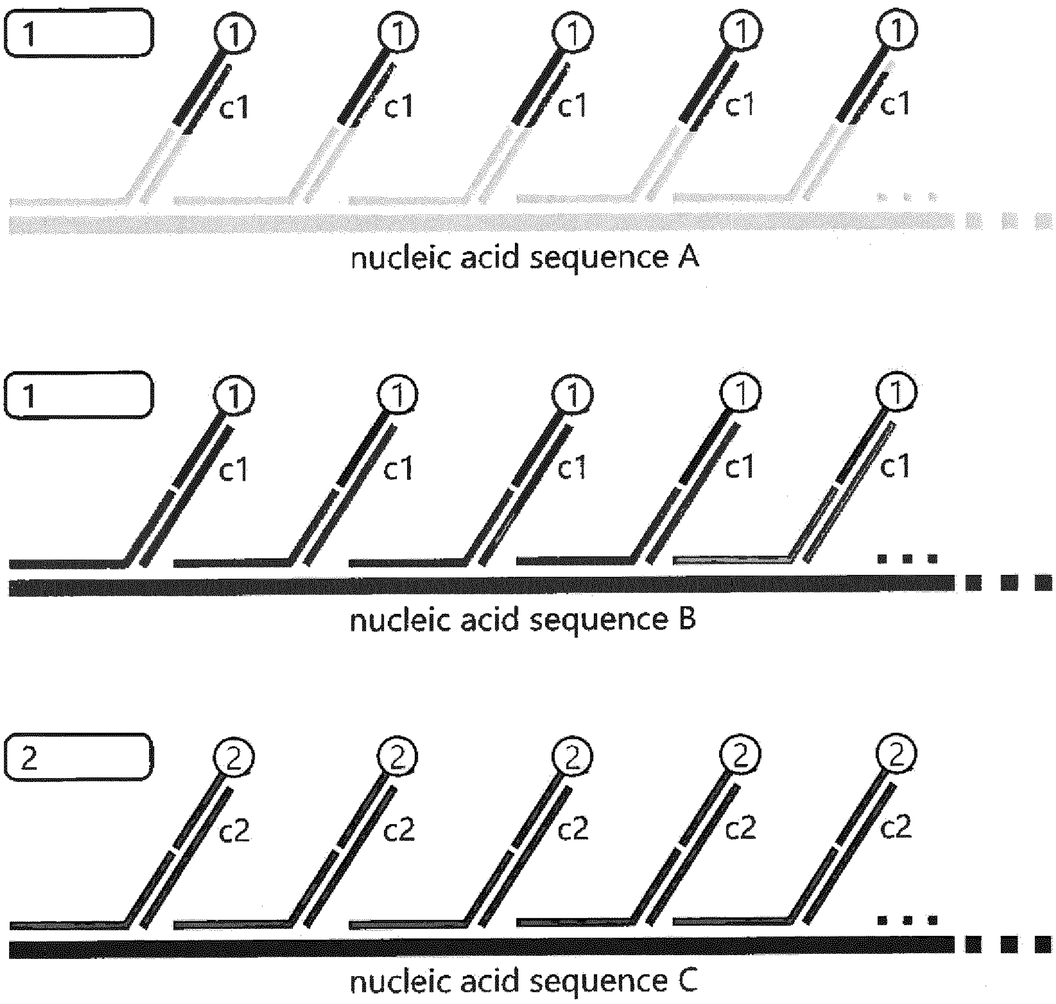


FIG. 5 continued

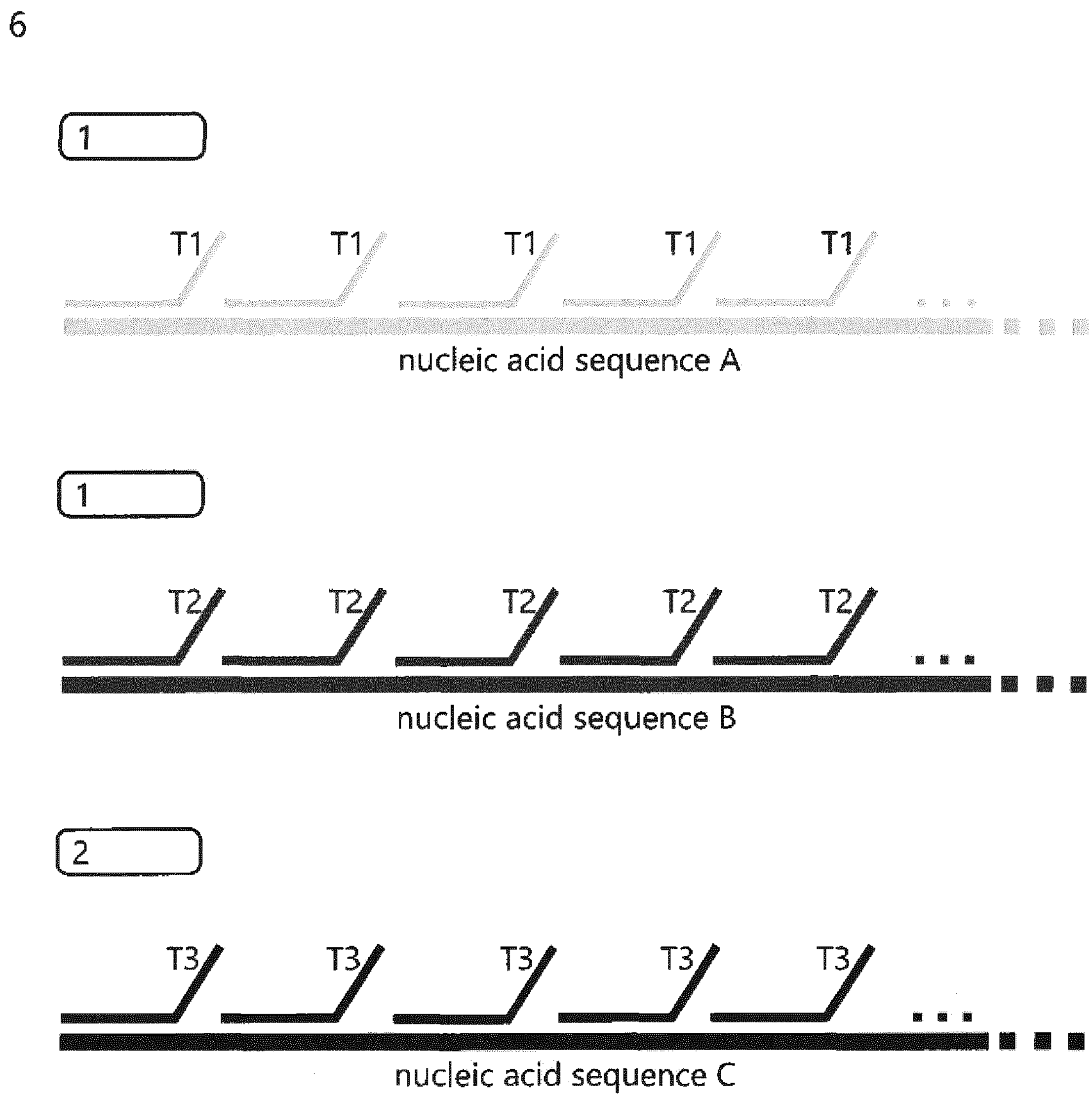


FIG. 5 continued

7

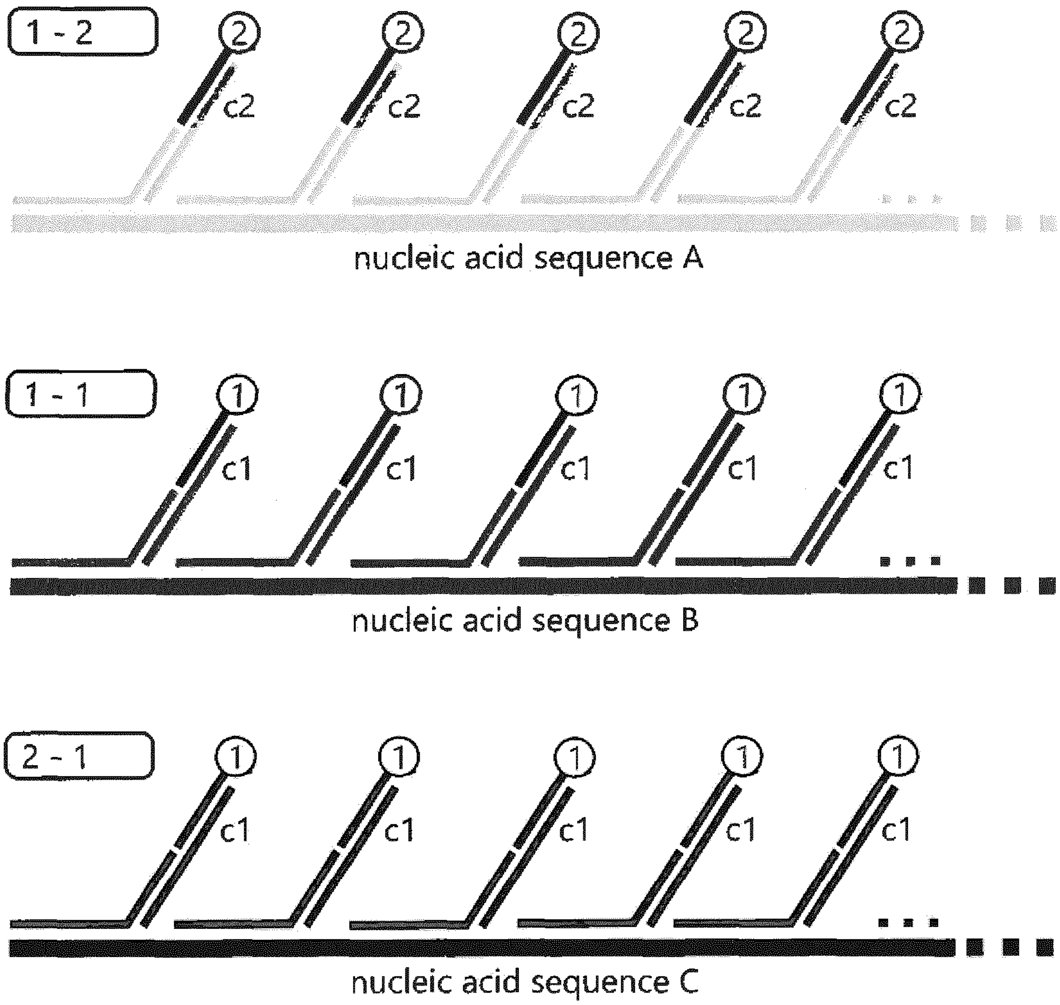


FIG. 5 continued

8

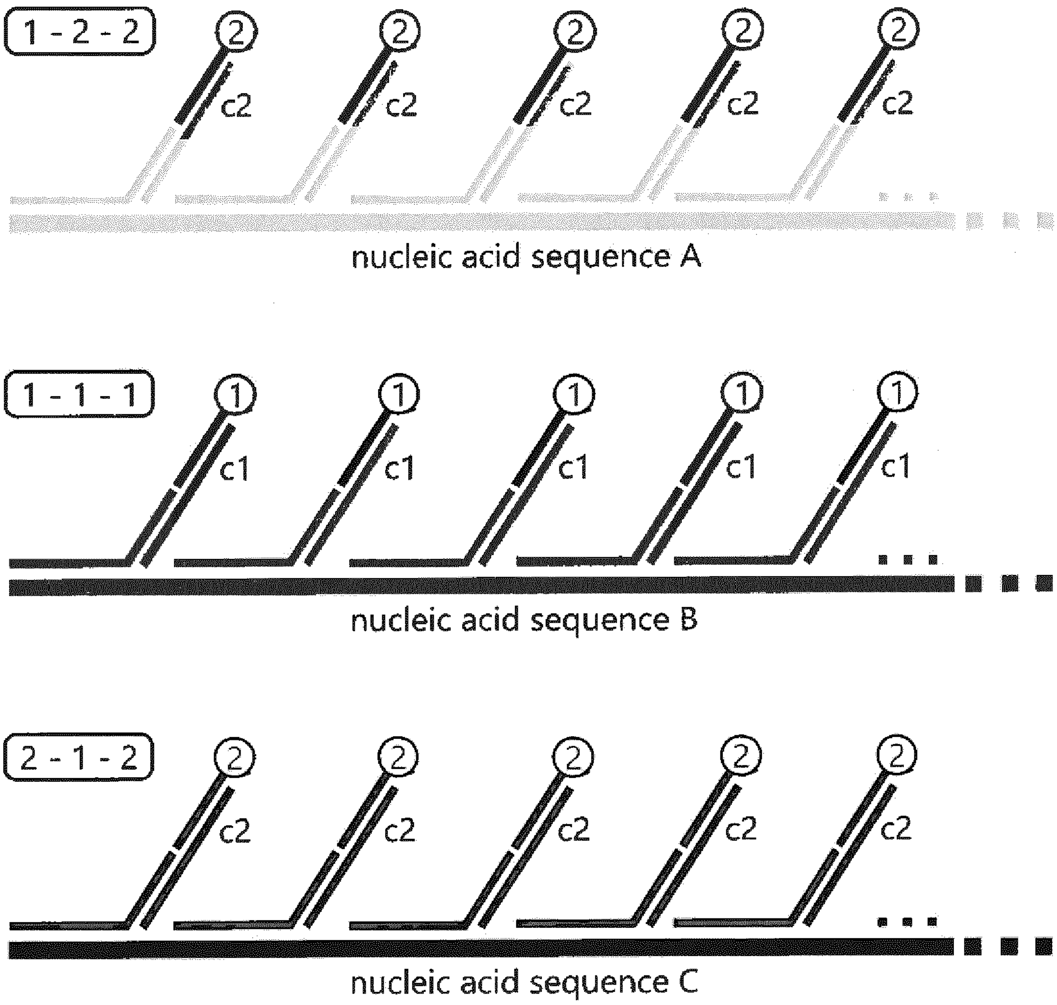


FIG. 5 continued

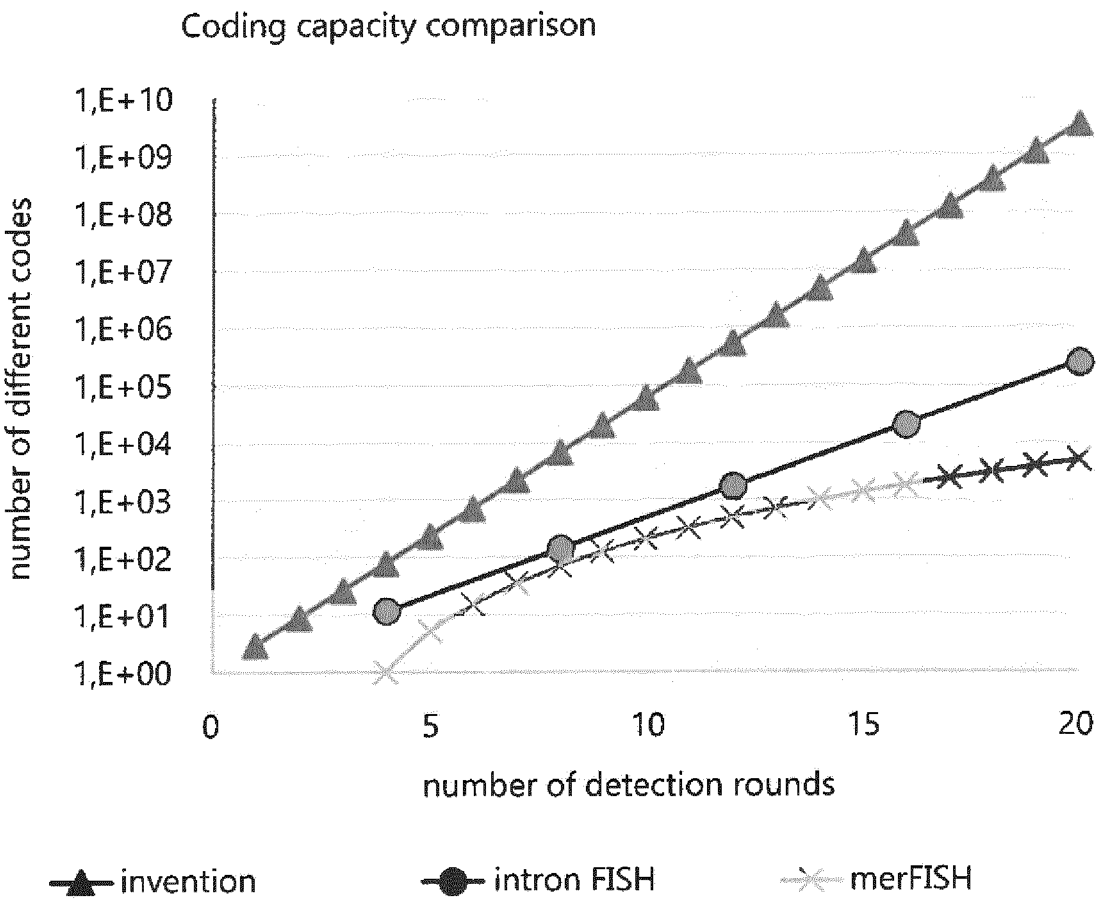
Coding capacity comparison
1,E+10
1,E+09
1,E+08
1,E+07
1,E+06
1,E+05
1,E+04
1,E+03
1,E+02
1,E+01
1,E+00
number of different codes
0
5
10
15
20
number of detection rounds
invention
intron FISH
merFISH

FIG. 6

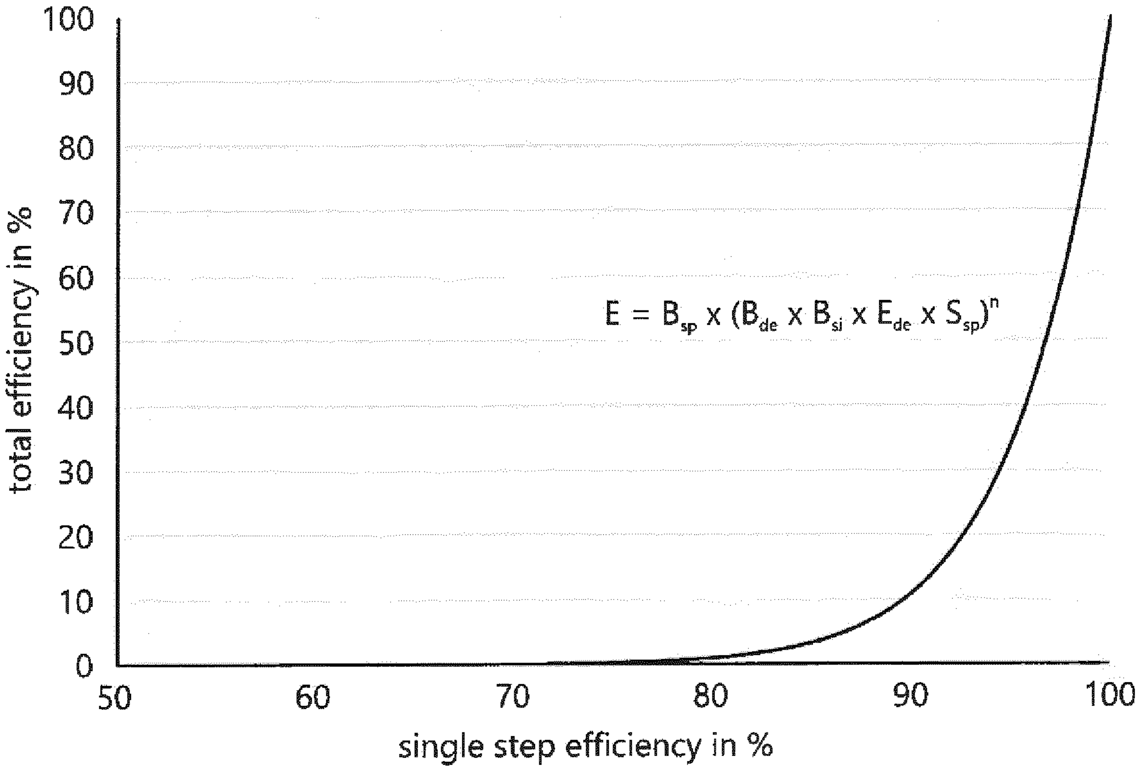
100
90
80
70
60
50
40
30
20
10
0
total efficiency in %
E = Bsp x (Bde x Bsi x Ede x Ssp)n
50
60
70
80
90
100
single step efficiency in %

FIG. 7

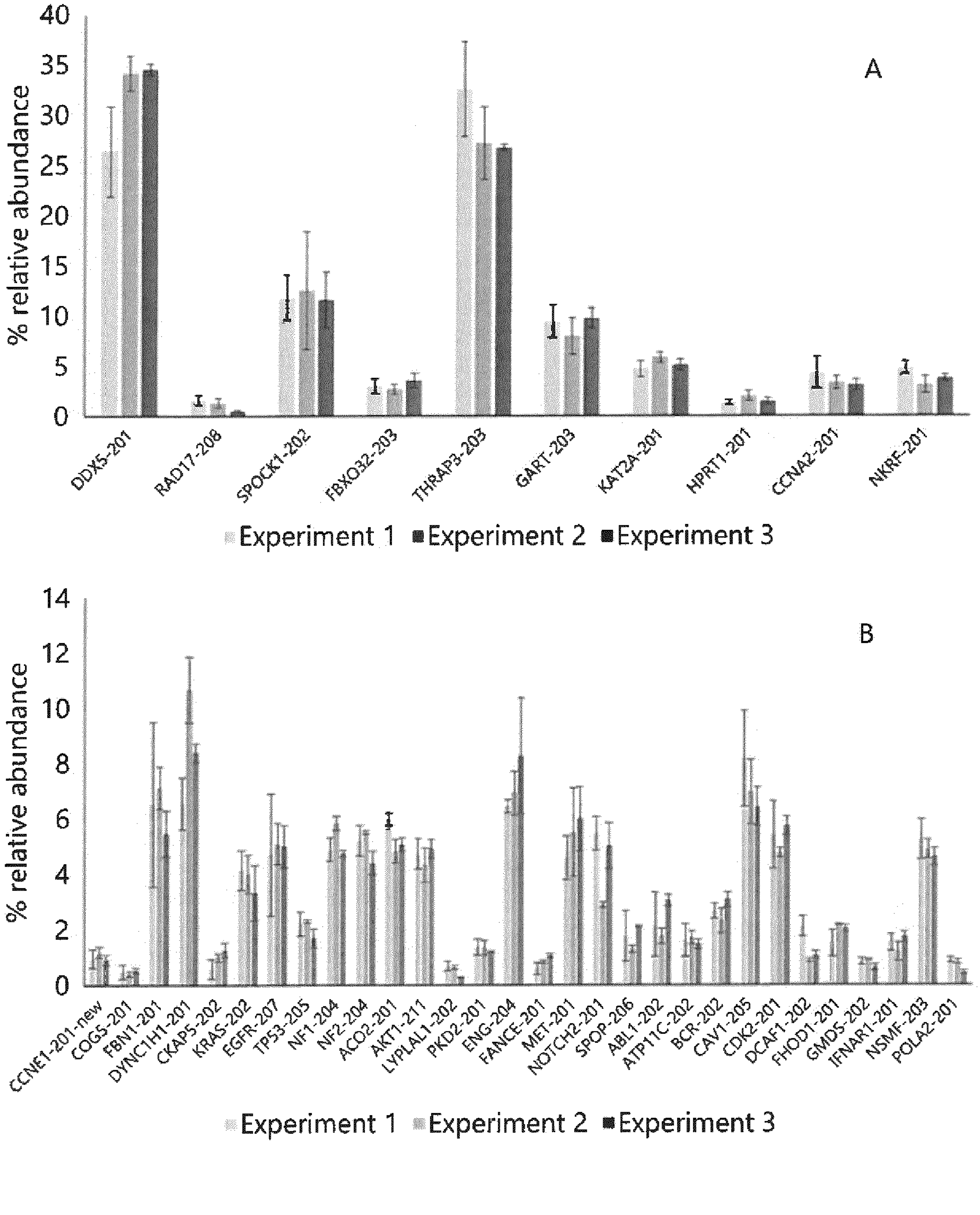
40
35
30
25
20
15
10
5
0
% relative abundance
A
DDX5-201
RAD17-208
SPOCK1-202
FBXO32-203
THRAP3-203
GART-203
KAT2A-201
HPRT1-201
CCNA2-201
NKRF-201
Experiment 1 Experiment 2 Experiment 3
14
12
10
8
6
4
2
0
% relative abundance
B
CCNE1-201-new
COG5-201
FBN1-201
DYNC1H1-201
CKAP5-202
KRAS-202
EGFR-207
TP53-205
NF1-204
NF2-204
ACO2-201
AKT1-211
LYPLAL1-202
PKD2-201
ENG-204
FANCE-201
MET-201
NOTCH2-201
SPOP-206
ABL1-202
ATP11C-202
BCR-202
CAV1-205
CDK2-201
DCAF1-202
FHOD1-201
GMDS-202
IFNAR1-201
NSMF-203
POLA2-201
Experiment 1 Experiment 2 Experiment 3

FIG. 8

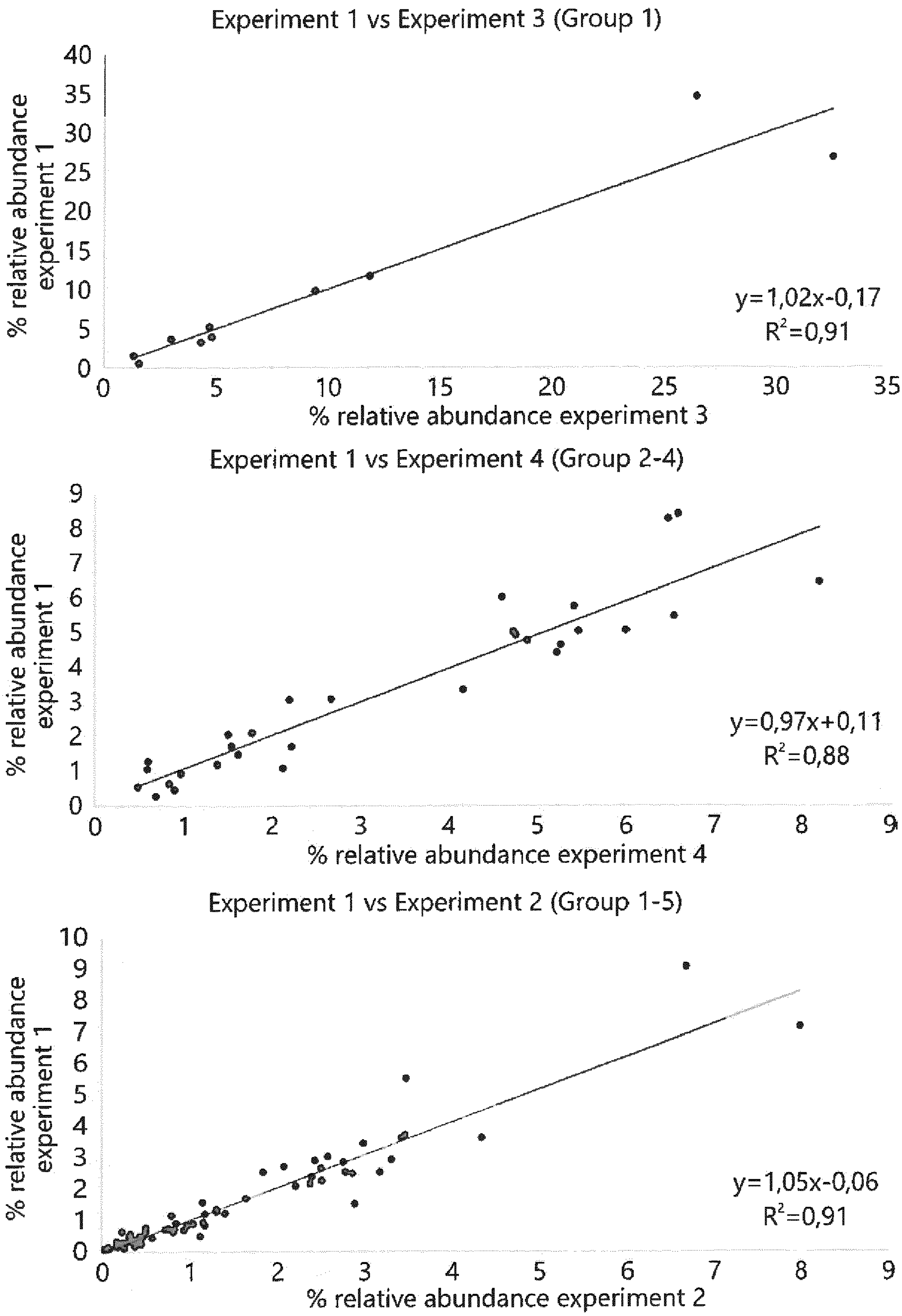
Experiment 1 vs Experiment 3 (Group 1)
% relative abundance experiment 1
% relative abundance experiment 3
0
5
10
15
20
25
30
35
40
y=1,02x-0,17
$R^2$=0,91
Experiment 1 vs Experiment 4 (Group 2-4)
% relative abundance experiment 4
0
1
2
3
4
5
6
7
8
9
y=0,97x+0,11
$R^2$=0,88
Experiment 1 vs Experiment 2 (Group 1-5)
% relative abundance experiment 2
10
y=1,05x-0,06
$R^2$=0,91

Fig. 9

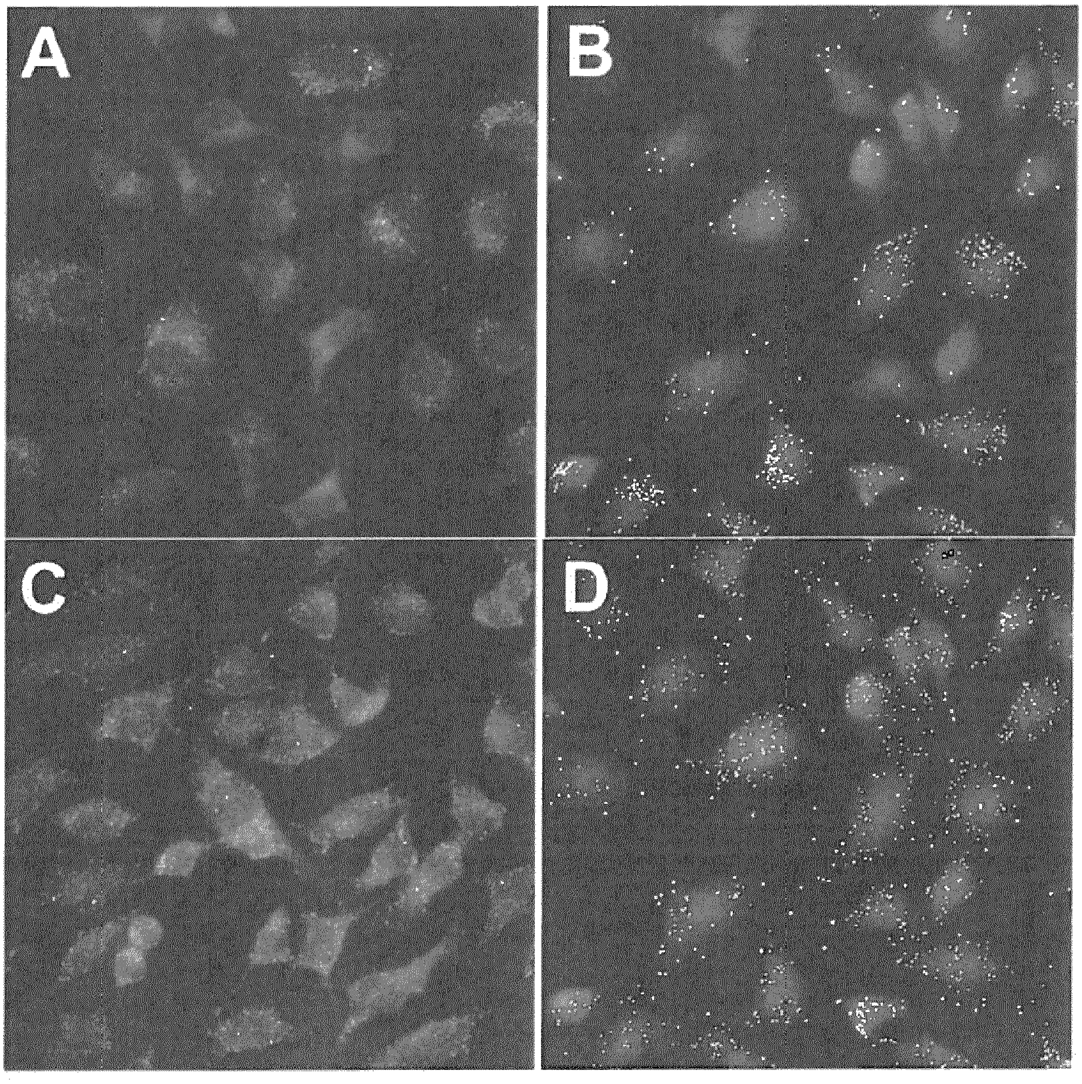


FIG. 10

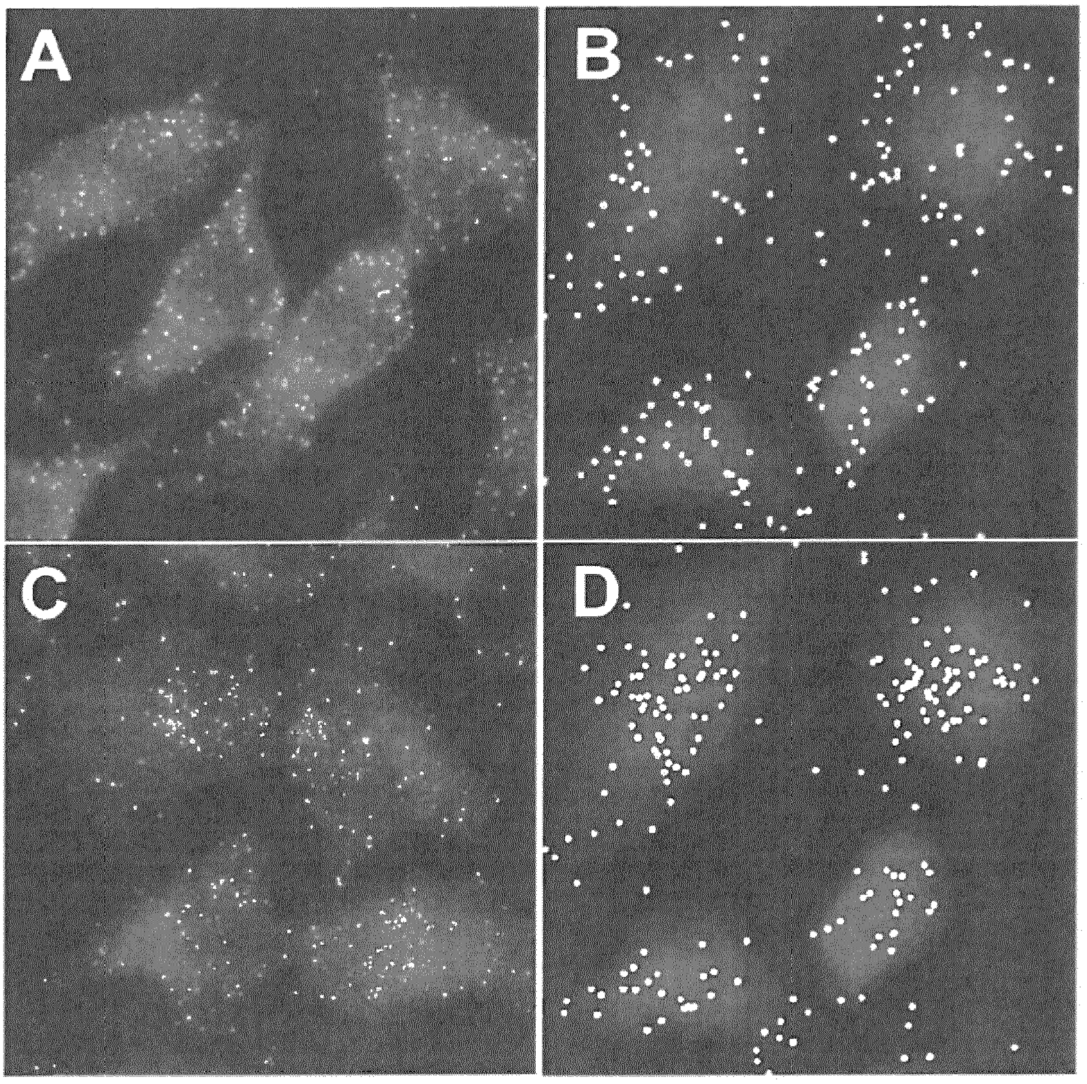


FIG. 11

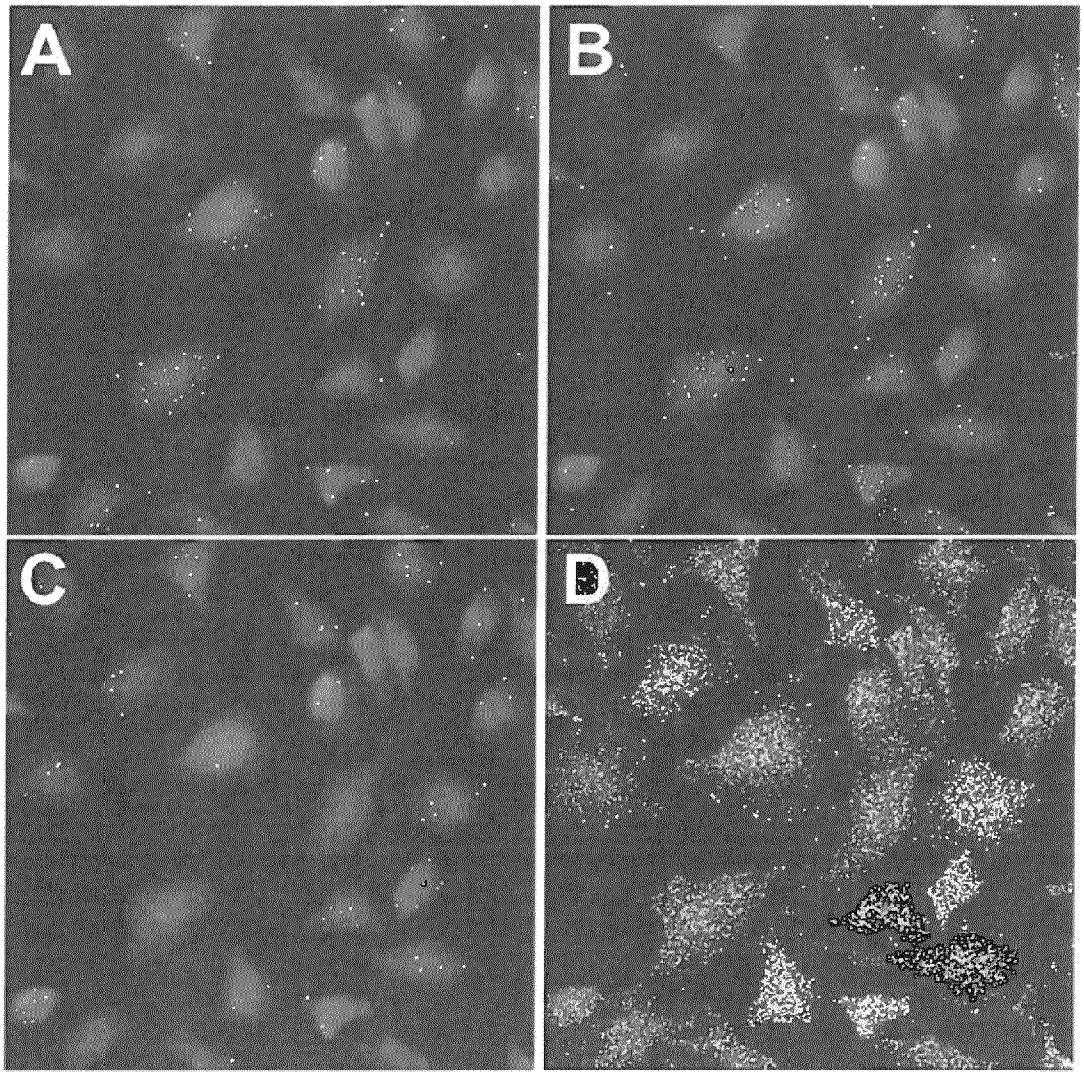


FIG. 12

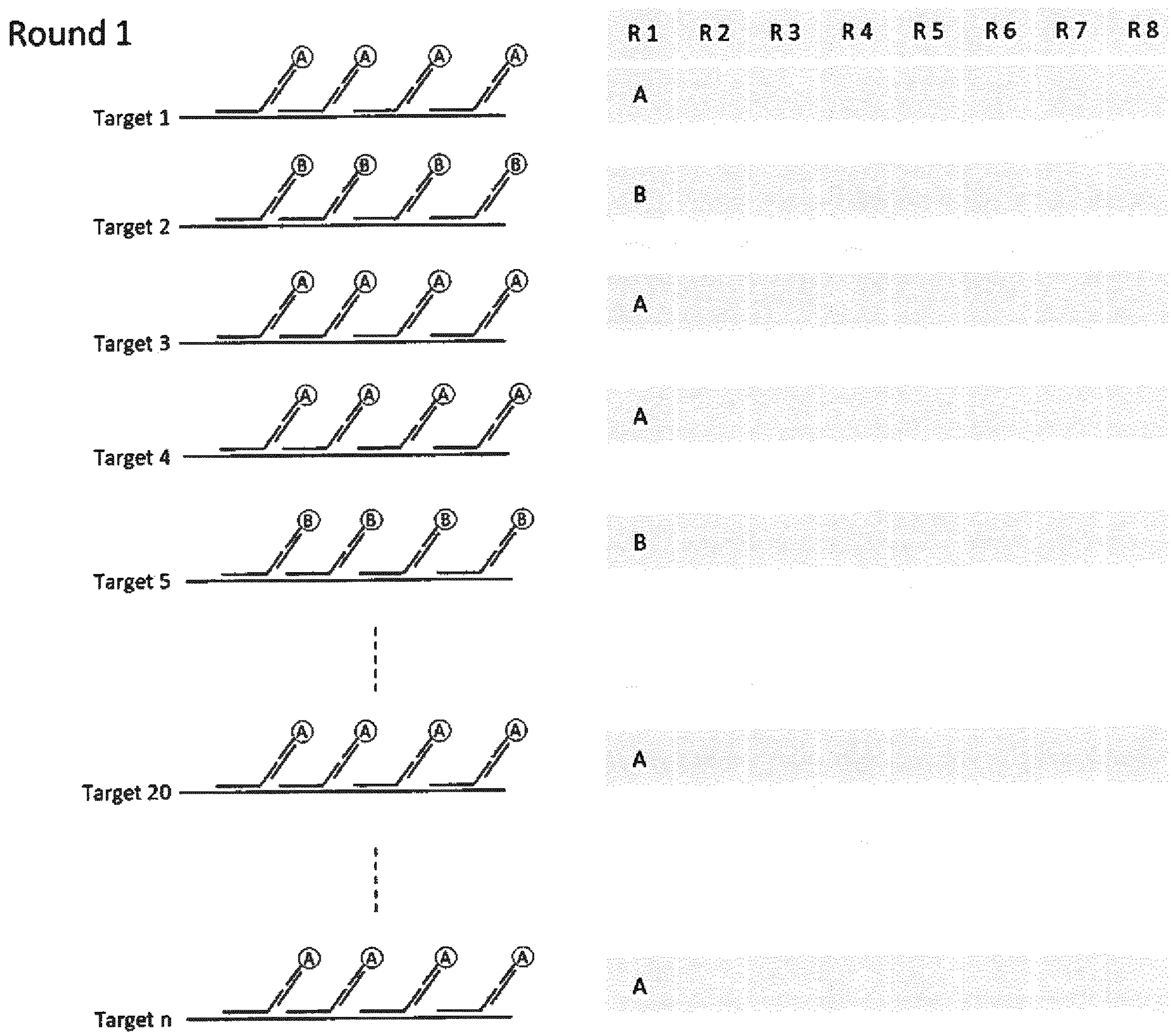
Round 1
Target 1
Target 2
Target 3
Target 4
Target 5
Target 20
Target n
A
B
R1
R2
R3
R4
R5
R6
R7
R8

FIG. 13

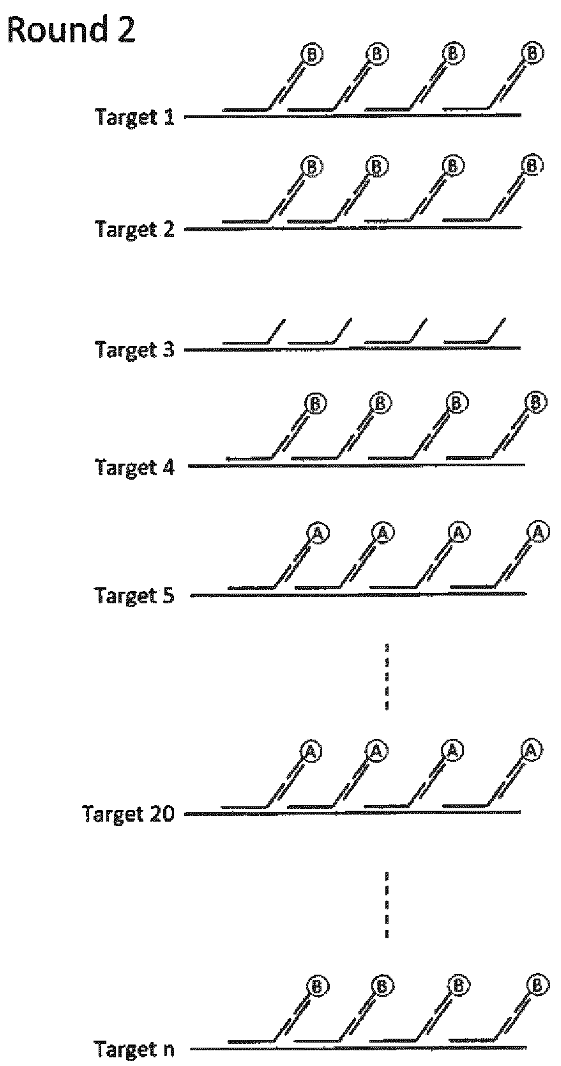


| R1 | R2 | R3 | R4 | R5 | R6 | R7 | R8 |
|---|---|---|---|---|---|---|---|
| A | B | | | | | | |
| B | B | | | | | | |
| A | - | | | | | | |
| A | B | | | | | | |
| B | A | | | | | | |
| A | A | | | | | | |
| A | B | | | | | | |

FIG. 13 continued

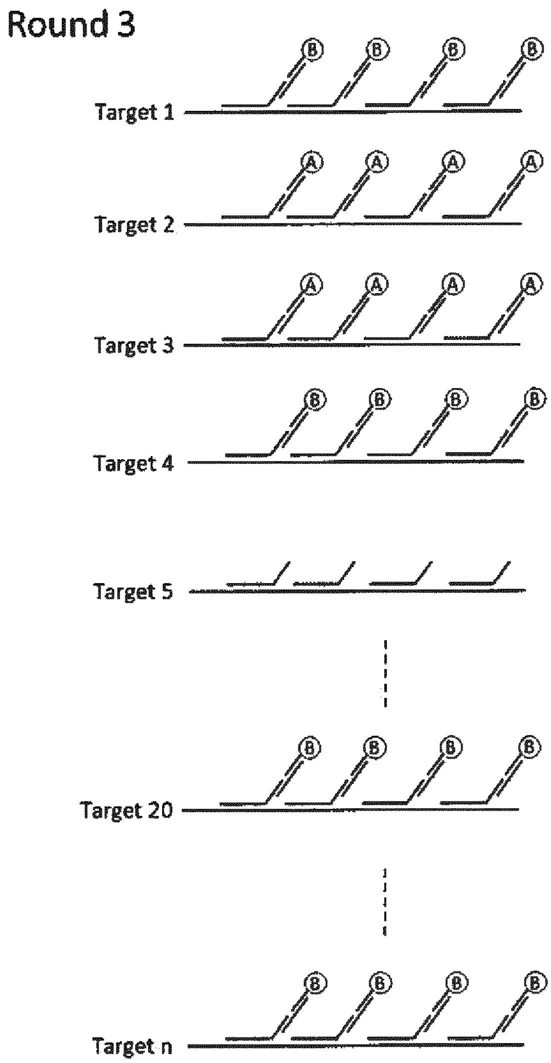


| R1 | R2 | R3 | R4 | R5 | R6 | R7 | R8 |
|---|---|---|---|---|---|---|---|
| A | B | B | | | | | |
| B | B | A | | | | | |
| A | - | A | | | | | |
| A | B | B | | | | | |
| B | A | - | | | | | |
| A | A | B | | | | | |
| A | B | B | | | | | |

FIG. 13 continued

Round 8

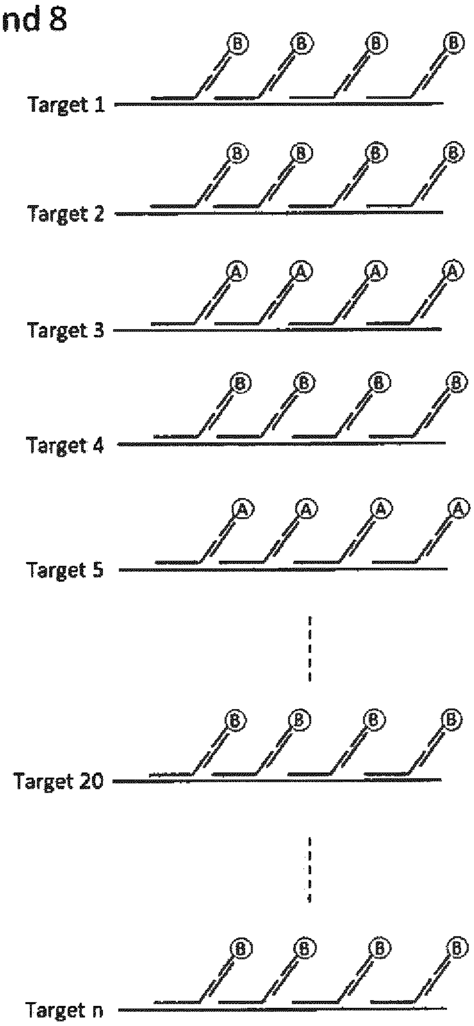


| R1 | R2 | R3 | R4 | R5 | R6 | R7 | R8 |
|---|---|---|---|---|---|---|---|
| A | B | B | A | - | B | A | B |
| B | B | A | A | B | A | - | B |
| A | - | A | B | A | B | B | A |
| A | B | B | - | A | A | B | B |
| B | A | - | A | B | A | A | A |
| A | A | B | - | B | A | B | B |
| A | B | B | B | A | - | A | A |

FIG. 13 continued

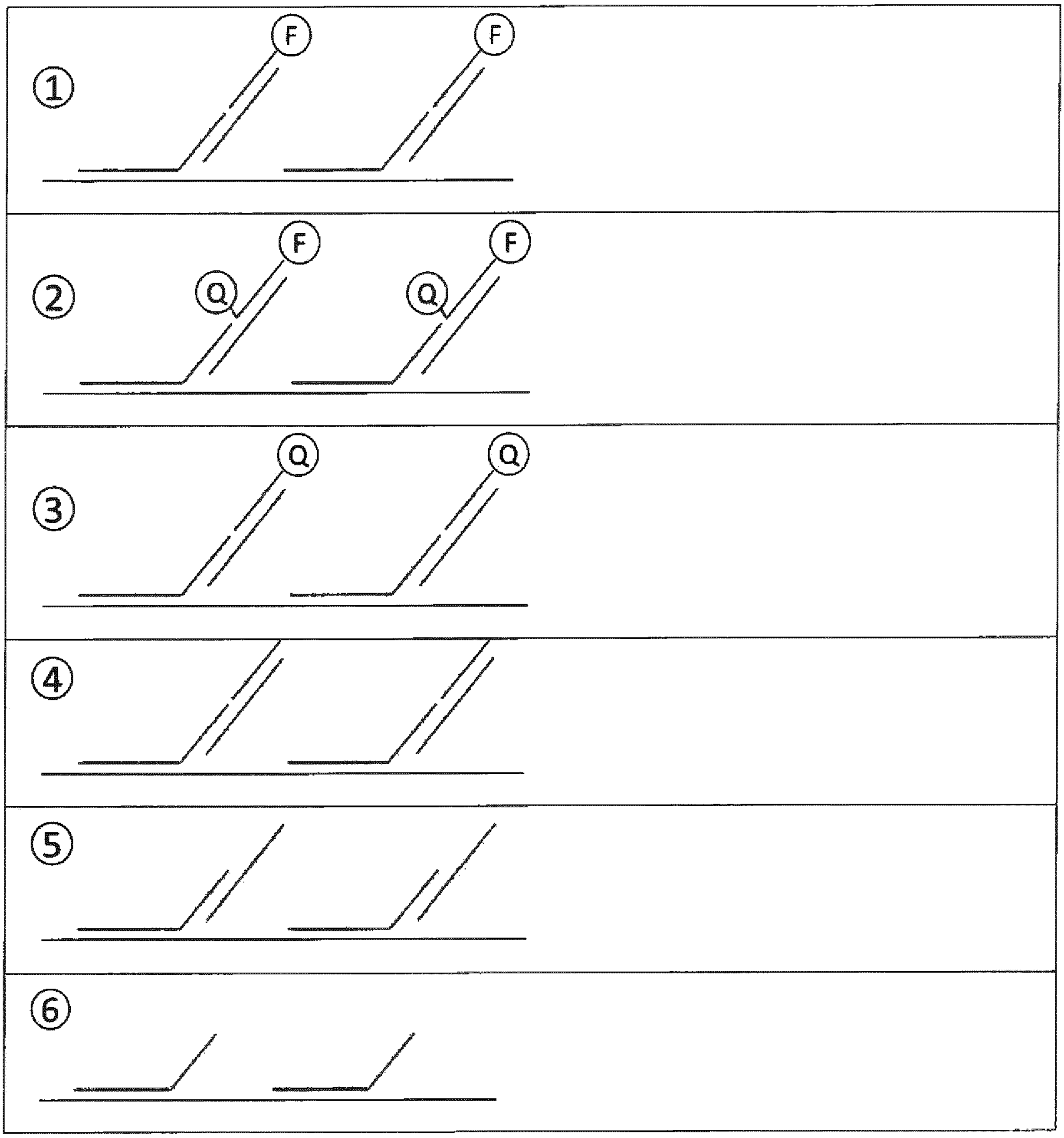


FIG. 14

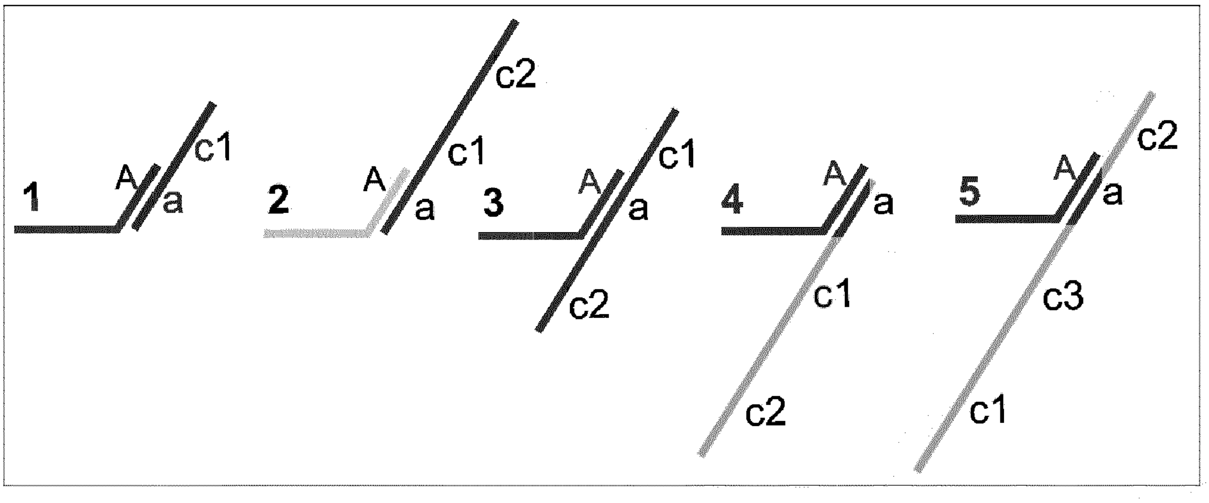


FIG. 15

Step 1:

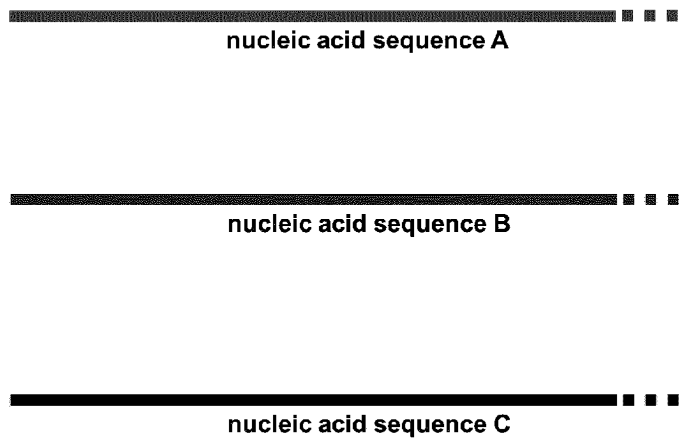


Step 2:

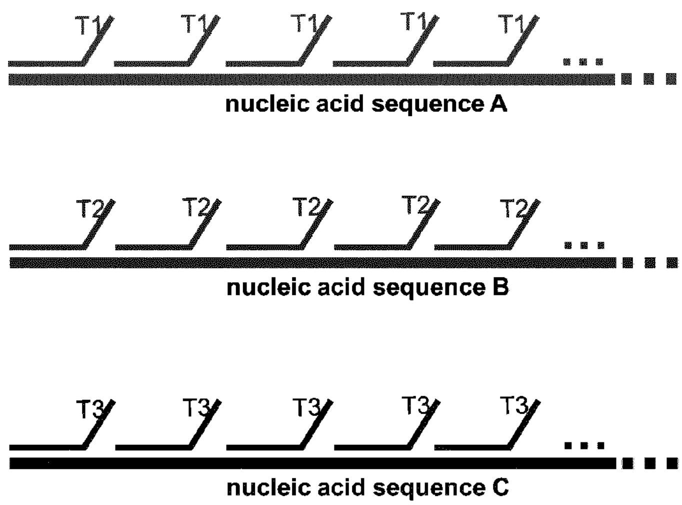


FIG. 16

Step 3:

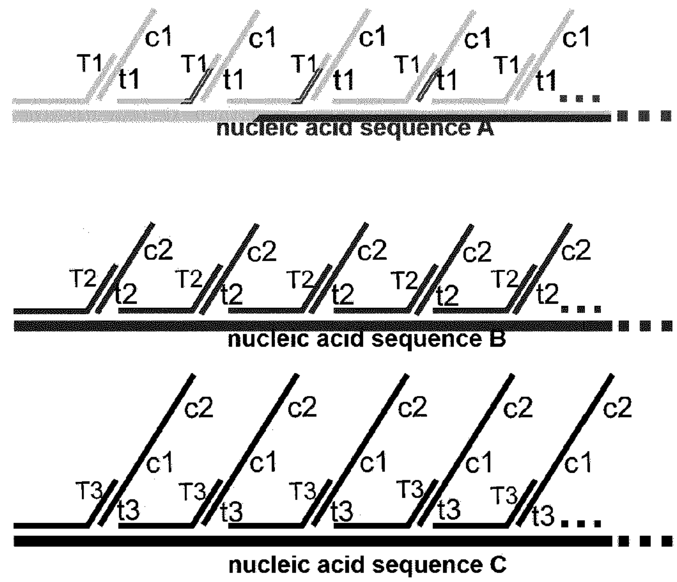


Step 4:

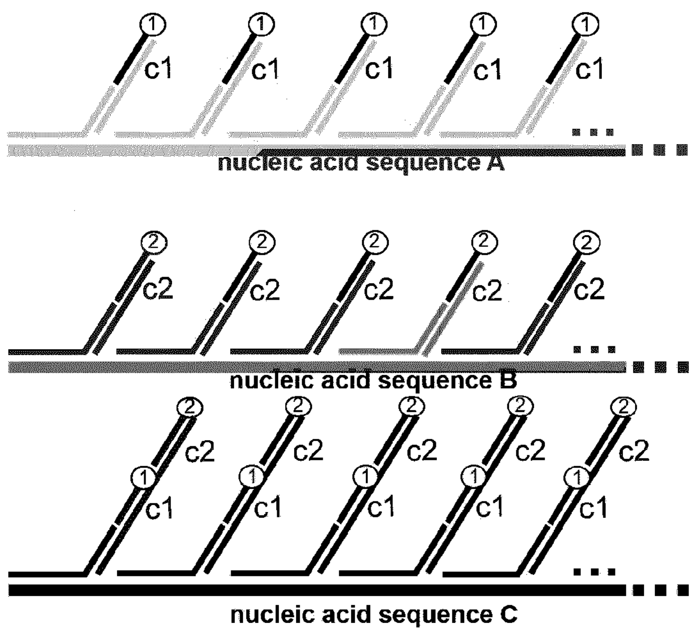


FIG. 16 (continued)

Step 5:

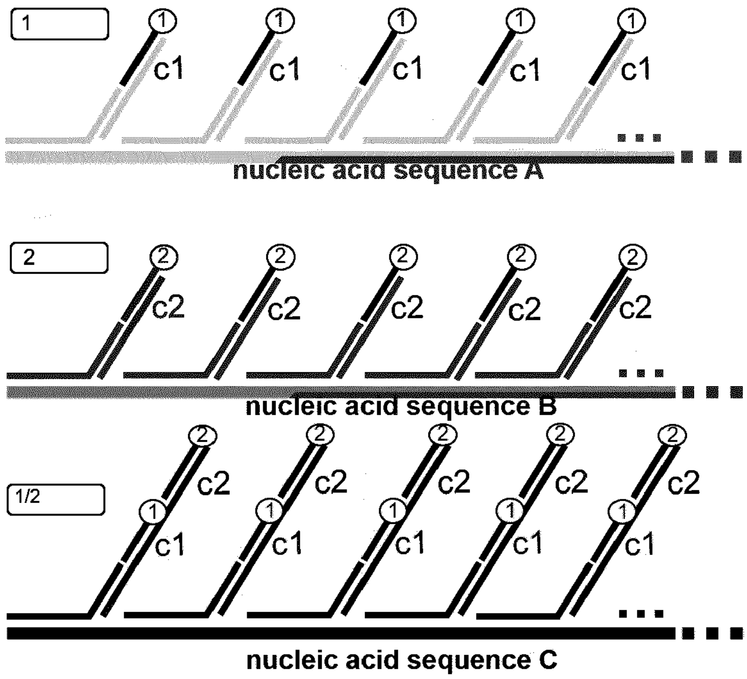


Step 6:

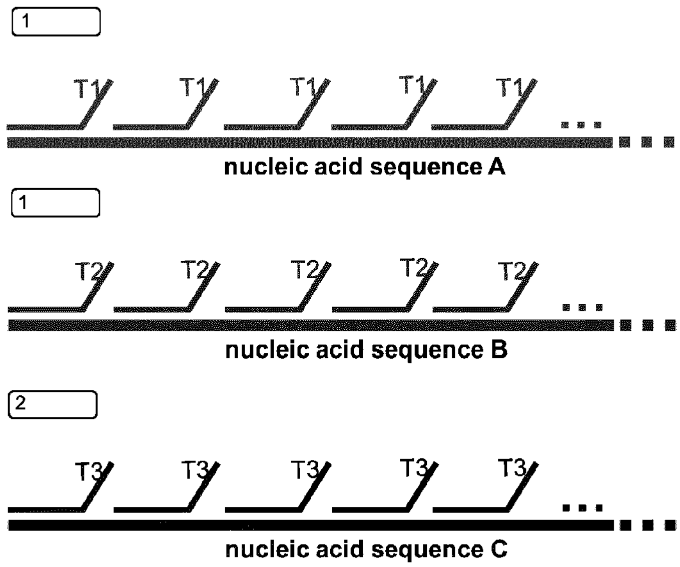


FIG. 16 (continued)

Step 7:

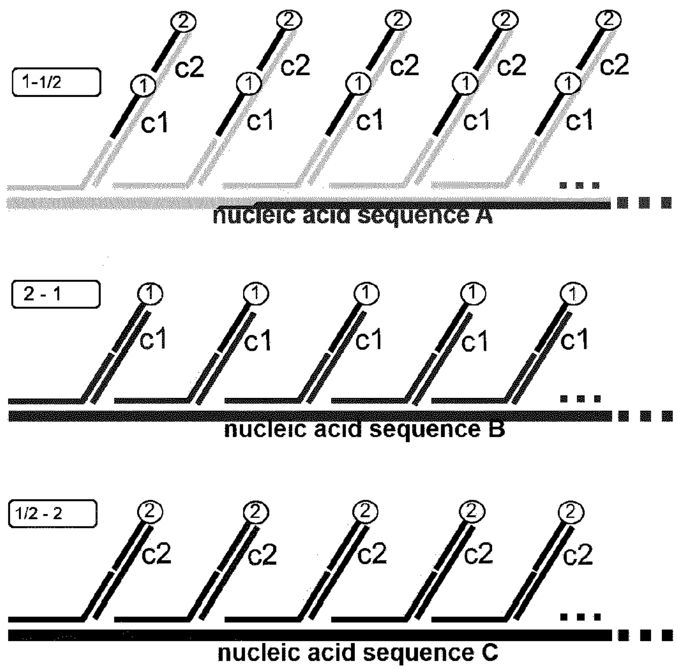


Step 8:

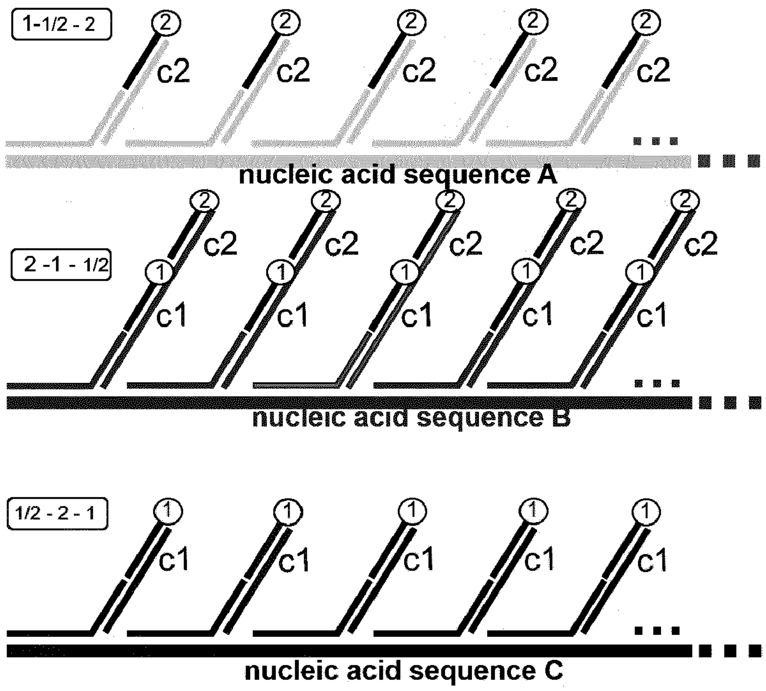


FIG. 16 (continued)

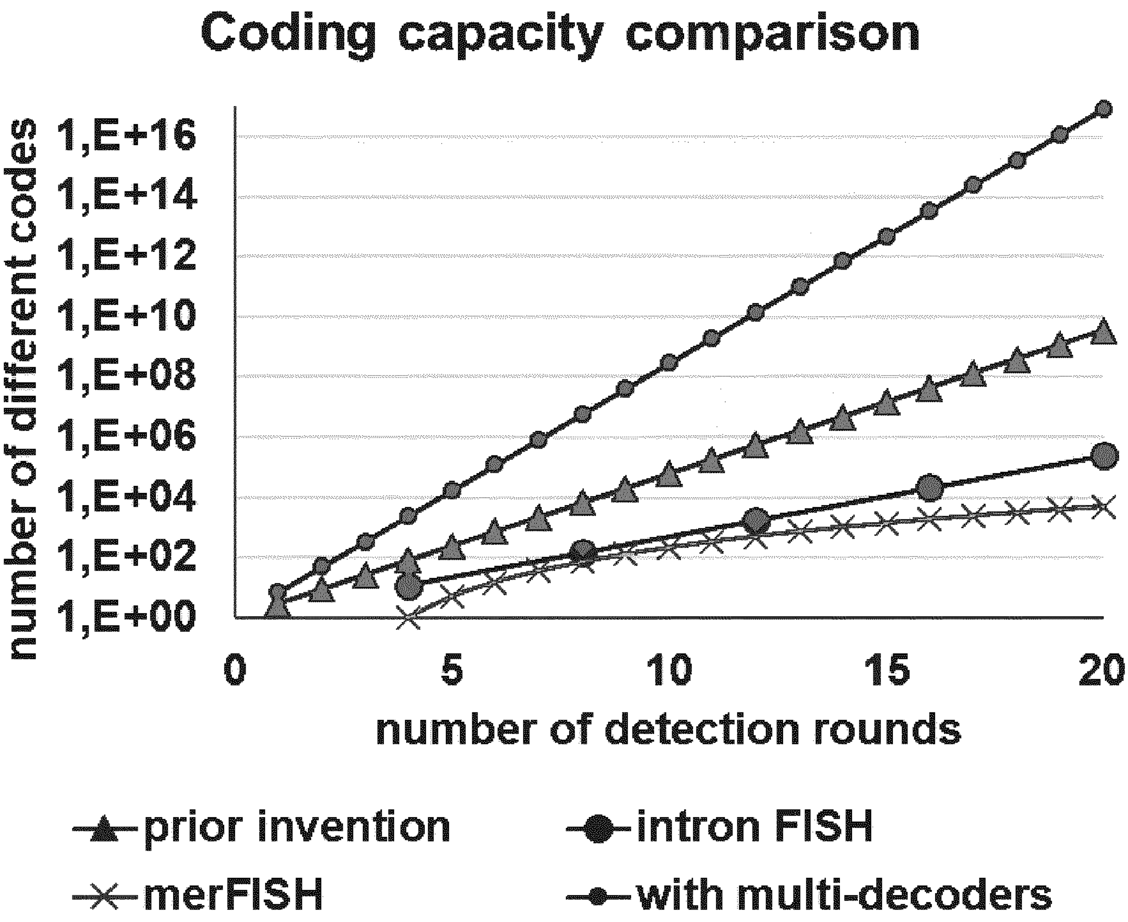
Coding capacity comparison
number of different codes
1,E+16
1,E+14
1,E+12
1,E+10
1,E+08
1,E+06
1,E+04
1,E+02
1,E+00
0
5
10
15
20
number of detection rounds
prior invention
intron FISH
merFISH
with multi-decoders

FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0611828 A
- WO 2017019481 A
- EP 2992115 B1
- WO 2020254519 A1
- WO 1993015215 A1


### Non-patent literature cited in the description


- **LUBECK et al.** Single-cell in situ RNA profiling by sequential hybridization. *Nat. Methods*, 2014, vol. 11 (4), 360-361
- **SHAH et al.** In situ transcription profiling of single cells reveals spatial organization of cells in the mouse hippocampus. *Neuron*, 2016, vol. 92 (2), 342-357
- **CHEN et al.** RNA imaging. Spatially resolved, highly multiplexed RNA profiling in single cells. *Science*, 2015, vol. 348 (6233), aaa6090
- **MOFFITT et al.** High-throughput single-cell gene-expression profiling with multiplexed error-robust fluorescence in situ hybridization. *Proc. Natl. Acad. Sci. U S A.*, 2016, vol. 113 (39), 11046-11051
- **SHAH et al.** Dynamics and spatial genomics of the nascent transcriptome by intron seqFISH. *Cell*, 2018, vol. 117 (2), 363-376
- **PLAYER et al.** Single-copy gene detection using branched DNA (bDNA) in situ hybridization. *J. Histochem. Cytochem.*, 2001, vol. 49 (5), 603-611
- **WANG et al.** RNAscope: a novel in situ RNA analysis platform for formalin-fixed, paraffin-embedded tissues. *J. Mol. Diagn.*, 2012, vol. 14 (1), 22-29
- **CHOI et al.** Programmable in situ amplification for multiplexed imaging of mRNA expression. *Nat. Biotechnol.*, 2010, vol. 28 (11), 1208-1212
- **CHOI et al.** Third-generation in situ hybridization chain reaction: multiplexed, quantitative, sensitive, versatile, robust. *Development*, 2018, vol. 145 (12)
- **MATEO et al.** Visualizing DNA folding and RNA in embryos at single-cell resolution. *Nature*, 2019, vol. 568, 49
- **C.V. HANSON**. *J. Clin. Microbiol.*, 1990, vol. 23, 2030
- Cell culture. **GEORGE VG** ; **HIERHOLZER JC** ; **ADES EW**. Virology methods manual. Academic Press: London, 1996, 3-24